(19) Europäisches Patentamt
European Patent Office
Office européen des brevets

(11) EP 2 029 745 B1

(12) EUROPEAN PATENT SPECIFICATION

(45) Date of publication and mention of the grant of the patent:
**12.10.2011 Bulletin 2011/41**

(21) Application number: **07808640.2**

(22) Date of filing: **07.06.2007**

(51) Int Cl.:
*C12Q 1/68* (2006.01)

(86) International application number:
**PCT/NZ2007/000142**

(87) International publication number:
**WO 2007/142540 (13.12.2007 Gazette 2007/50)**

(54) **DIAGNOSTIC METHODS AND MARKERS**

DIAGNOSEVERFAHREN UND -MARKER

PROCÉDÉS DE DIAGNOSTIC ET MARQUEURS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.06.2006 US 811407 P**

(43) Date of publication of application:
**04.03.2009 Bulletin 2009/10**

(73) Proprietor: **Otago Innovation Limited**
**Dunedin 9016 (NZ)**

(72) Inventors:
- **ASSINDER, Stephen, John**
  **Sydney, New South Wales 2042 (AU)**
- **STANTON, Jo-Ann**
  **Dunedin (NZ)**

(74) Representative: **Lee, Nicholas John et al**
**Kilburn & Strode LLP**
**20 Red Lion Street**
**London WC1R 4PJ (GB)**

(56) References cited:
**WO-A2-01/70979 US-A1- 2004 142 325**

- **DATABASE EMBL [Online] 20 January 2003 (2003-01-20), "RZPD Homo sapiens cDNA clone IMAGp998D225571 = IMAGE:2250981 5' EST." XP002551979 retrieved from EBI accession no. EMBL:BX109457 Database accession no. BX109457**
- **DATABASE EMBL [Online] 26 April 1999 (1999-04-26), "tu13h11.x1 NCI_CGAP_Pr28 Homo sapiens cDNA clone IMAGE:2250981 3', mRNA sequence." XP002551980 retrieved from EBI accession no. EMBL:AI611685 Database accession no. AI611685**
- **DATABASE Netaffx [Online] 2009, "Human Genome U95D Array" XP002551981 retrieved from Affymetrix Database accession no. 81941_AT (Probe Set ID)**
- **DATABASE Geo [Online] 11 March 2002 (2002-03-11), [HG_U95D] Affymetrix Human Genome U95D Array: XP002551982 retrieved from Ncbi Database accession no. GPL94**
- **ERNST T ET AL: "GENEXPRESSIONS-ANALYSE DES PROSTATAKARZINOMS GENE EXPRESSION PROFILING IN PROSTATE CANCER" VERHANDLUNGEN DER DEUTSCHEN GESELLSCHAFT FUER PATHOLOGIE, GUSTAV FISCHER VERLAG, STUTTGART, DE, vol. 86, 1 January 2002 (2002-01-01), pages 165-175, XP009024850 ISSN: 0070-4113**
- **DALMA-WEISZHAUSZ DENNISE D ET AL: "THE AFFYMETRIX GENECHIP PLATFORM: AN OVERVIEW" METHODS IN ENZYMOLOGY; [METHODS IN ENZYMOLOGY], ACADEMIC PRESS INC, SAN DIEGO, CA, US, vol. 410, 1 January 2006 (2006-01-01), pages 3-28, XP009079981 ISSN: 0076-6879**
- **OUDES ASA J ET AL: "Application of affymetrix array and massively parallel signature sequencing for identification of genes involved in prostate cancer progression" BMC CANCER, BIOMED CENTRAL, LONDON, GB, vol. 5, no. 1, 22 July 2005 (2005-07-22), page 86, XP021004851 ISSN: 1471-2407**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YU YAN PING ET AL: "Gene expression alterations in prostate cancer predicting tumor aggression and preceding development of malignancy." JOURNAL OF CLINICAL ONCOLOGY : OFFICIAL JOURNAL OF THE AMERICAN SOCIETY OF CLINICAL ONCOLOGY 15 JUL 2004, vol. 22, no. 14, 15 July 2004 (2004-07-15), pages 2790-2799, XP002551983 ISSN: 0732-183X
- CHAKRABARTI RATNA ET AL: "Profiling of differential expression of messenger RNA in normal, benign, and metastatic prostate cell lines." CANCER GENETICS AND CYTOGENETICS, vol. 139, no. 2, December 2002 (2002-12), pages 115-125, XP002551984 ISSN: 0165-4608
- HARDIMAN G: "MICROARRAY PLATFORMS - COMPARISONS AND CONTRASTS" PHARMACOGENOMICS, ASHLEY PUBLICATIONS, GB, vol. 5, no. 5, 1 January 2004 (2004-01-01), pages 487-502, XP009079995 ISSN: 1462-2416
- DATABASE GENBANK [Online] ROLFS I., XP008092913 Database accession no. (BX109457)
- DATABASE GENBANK [Online] STRAUSBERG R., XP008092912 Database accession no. (BF222603)
- DATABASE GENBANK [Online] STRAUSBERG R., XP008092916 Database accession no. (AI611685)
- DATABASE GENBANK [Online] STRAUSBERG R., XP008092915 Database accession no. (BF446403)
- DATABASE GENBANK [Online] STRAUSBERG R., XP008092914 Database accession no. (AI418055)
- DATABASE GENBANK [Online] STRAUSBERG R., XP008092920 Database accession no. (AA658380)

SEQUENCE LISTING

<110> ASSINDER, Stephen J
STANTON, Jo-Ann L

<120> DIAGNOSTIC METHODS AND MARKERS

<130> 516938 TVG

<150> 60/811,407
<151> 2006-06-09

<160> 35

<170> PatentIn version 3.3

<210> 1
<211> 368
<212> DNA

<213> Pspu1

<400> 1

aagaattcgg cacgaggaat atttaagggt aaaatttttc tacttttaaa gcttaaaaaa 60
atgttttttt actactgtaa aagtaatgca gagaaatgtt cacttaccaa acacatacct 120
ttgtaaaaat caccacttaa agtttgtttc taaagatttt aggacaccaa gatgcaaata 180
atatttttgg ctgttacctg ctctttcact actgctgagt ctgcagtggc aagatagcta 240
cacagtacct cagccctcct gctcagtttt taacatctat tgataatact aattacaaga 300
aaatttaaaa tgtctttttg caaaaaaata ccataagcag tcaaaacaca attaaaaaaa 360
aaaaaaaa 368

<210> 2
<211> 394
<212> DNA
<213> Pspu2

<220>
<221> misc_feature
<222> (143)..(143)
<223> where "N" can be "A" or nothing

<400> 2

gcaaaattag gacattccca gataaacaaa taaagagact tcatcactaa taaacatgcc 60
ctgtatgaaa tactaagagg agtccttggg gcggacatga aaggacacta gacaaatcca 120
catgaagacg taaagattgc cangtgaagt aactacatag gcaaatataa acgatagtat 180
aaatgtattt ttgtttgtga ctcttttttt tgtgctgatt taaaagacag cttcatagaa 240
aaaaattata aatctgtgtt gatgggcaca cagtgcacaa agatgtaata ttaatacaat 300
aacagcacag gaatggagag ggaacagagg aatacaaaat ctaactagac ttataacaag 360
caaatggatt gatttaatta aaaaaaaaaa aaaa 394

<210> 3
<211> 392
<212> DNA
<213> Pspu43

<400> 3

tttattaaca aatataaagt accagacact ccaagtgctt agaaaattaa attaactcat 60
ttaattttac taaaaacctg tgagataagg gactattgtt atgcccattt ttcagatgag 120
gaaagtggga cagagtttac tgagtggtag agatgagatt tgaacccagg caagcaggct 180
ctaggtctgg actcttggtc actctgctct gtccccacac taagccaggt atctgggctt 240
gtgtacatag tcaggtaagt ggaaatcaca ggaattgcta gaggactcca gtagagggat 300
gattttcatg aatgttcttt catctcaaga aagcaaaggc tgctttttgg gtgcaaagga 360
gtttggctag aaggaagatc tggagataga gc 392

<210> 4
<211> 1320
<212> DNA
<213> Pspu8

<220>
<221> misc_feature
<222> (1021)..(1021)
<223> where s can be C or G, w can be A or T, and k can be T or G

<400> 4

cacgaggcga gaaacaccca agaatgatca attaaaaaaa aaaaagaaag aaagaaagaa 60
agaaaagaag cagagttgga ctcagcggga aaataggcgt gctaccacct caggagagtt 120
ccagggaaga accccacccg cactccaatg aggtcacaat ggctggagct ctgaggggcc 180
caggctccct gagccaggag gagaggagaa agtccaagga aagatggctg gcagtcaccc 240
ctacttcaac ctgcctgact ccacacaccc atcgccgccc tccgctccac ccagcctccg 300
ctggcaccag cgctgccagc cctctggtgc caccaatggc ctgctggtgg ccctgctggg 360
tgggggcctg cctgctggct tcgtgggccc cctttctcgt atggcttacc agggttccaa 420
cctgccctcg ctggagctgc tcatctgtcg atgcctcttc cacctcccta ttgccctgct 480
acttaaactg cgtggcgacc cccttctggg acctcctgac atccgaggct gggcctgctt 540
ctgtgccctg ctcaacgtcc tcagcattgg atgtgcctac agtgcagttc aggtggtgcc 600
cgctggcaac gctgccactg ttcgcaaagg ttcttccacc gtatgctccg ctgtcctcac 660
cctactgcct tgagagccag ggtctcggtg gctacgagtg gtgtggactg ttgggcagca 720
tcctaggact aatcatcatt ctgggacctg gactctggac actaccagga ggggaccaca 780
ggtgtctaca ccaccctggg ctatgtgcag gctttcctgg gaggcctggc gctgtccctg 840
gggctaatct ggtctatcgt atctctgcac tttcccatcc tgcctcccaa cagtggcctt 900
cctatctggc ttggatgggg ctgctgggct stgtgccagg cctctttgtg ctgcagaccc 960
ccgtgttgcc cagtgacctc ctgagttgga gttgtgtggg gggcagaggg gatcctcgcc 1020
swtkggtctc ccttcacatg ggtgtgggct atgcgggtca ccaaggcccc acaccctgcc 1080
ctggtgtgcg ctgtcctgca ttccgaggtg gttgtggccc ttatactgca gtattatatg 1140
ctccatgaga ctgtggcact ttctgacatc atgggggcag gggttgtgct gggcagcatt 1200

gccatcatta cagcccggaa cctcagctgt gagaggacag ggaaggtgga ggagtgagat 1260
agaacttggg agcccggggg ttgggaggga cagggataaa taaagacaaa gactgaagac 1320

<210> 5
<211> 1906
<212> DNA
<213> PSA

<400> 5

agccccaagc ttaccacctg cacccggaga gctgtgtcac catgtgggtc ccggttgtct 60
tcctcaccct gtccgtgacg tggattggtg ctgcacccct catcctgtct cggattgtgg 120
gaggctggga gtgcgagaag cattcccaac cctggcaggt gcttgtggcc tctcgtggca 180
gggcagtctg cggcggtgtt ctggtgcacc cccagtgggt cctcacagct gcccactgca 240
tcaggaacaa aagcgtgatc ttgctgggtc ggcacagcct gtttcatcct gaagacacag 300
gccaggtatt tcaggtcagc cacagcttcc cacacccgct ctacgatatg agcctcctga 360
agaatcgatt cctcaggcca ggtgatgact ccagccacga cctcatgctg ctccgcctgt 420
cagagcctgc cgagctcacg gatgctgtga aggtcatgga cctgcccacc caggagccag 480
cactggggac cacctgctac gcctcaggct ggggcagcat tgaaccagag gagttcttga 540
ccccaaagaa acttcagtgt gtggacctcc atgttatttc caatgacgtg tgtgcgcaag 600
ttcaccctca gaaggtgacc aagttcatgc tgtgtgctgg acgctggaca gggggcaaaa 660
gcacctgctc gtgggtcatt ctgatcaccg aactgaccat gccagccctg ccgatggtcc 720
tccatggctc cctagtgccc tggagaggag gtgtctagtc agagagtagt cctggaaggt 780
ggcctctgtg aggagccacg gggacagcat cctgcagatg gtcctggccc ttgtcccacc 840
gacctgtcta caaggactgt cctcgtggac cctcccctct gcacaggagc tggaccctga 900
agtcccttcc ccaccggcca ggactggagc ccctacccct ctgttggaat ccctgcccac 960
cttcttctgg aagtcggctc tggagacatt tctctcttct tccaaagctg ggaactgcta 1020
tctgttatct gcctgtccag gtctgaaaga taggattgcc caggcagaaa ctgggactga 1080
cctatctcac tctctccctg cttttaccct tagggtgatt ctgggggccc acttgtctgt 1140
aatggtgtgc ttcaaggtat cacgtcatgg ggcagtgaac catgtgccct gcccgaaagg 1200
ccttccctgt acaccaaggt ggtgcattac cggaagtgga tcaaggacac catcgtggcc 1260
aacccctgag caccccctatc aacccccctat tgtagtaaac ttggaacctt ggaaatgacc 1320
aggccaagac tcaagcctcc ccagttctac tgacctttgt ccttaggtgt gaggtccagg 1380
gttgctagga aaagaaatca gcagacacag gtgtagacca gagtgtttct taaatggtgt 1440
aattttgtcc tctctgtgtc ctggggaata ctggccatgc ctggagacat atcactcaat 1500
ttctctgagg acacagatag gatggggtgt ctgtgttatt tgtggggtac agagatgaaa 1560
gaggggtggg atccacactg agagagtgga gagtgacatg tgctggacac tgtccatgaa 1620
gcactgagca gaagctggag gcacaacgca ccagacactc acagcaagga tggagctgaa 1680

aacataaccc actctgtcct ggaggcactg ggaagcctag agaaggctgt gagccaagga 1740
gggagggtct tcctttggca tgggatgggg atgaagtaag gagagggact ggaccccctg 1800
gaagctgatt cactatgggg ggaggtgtat tgaagtcctc cagacaaccc tcagatttga 1860
tgatttccta gtagaactca cagaaataaa gagctgttat actgtg 1906

<210> 6
<211> 3922

<212> DNA
<213> PCA3/DD3

<400> 6

acagaagaaa tagcaagtgc cgagaagctg gcatcagaaa aacagagggg agatttgtgt 60
ggctgcagcc gagggagacc aggaagatct gcatggtggg aaggacctga tgatacagag 120
gaattacaac acatatactt agtgtttcaa tgaacaccaa gataaataag tgaagagcta 180
gtccgctgtg agtctcctca gtgacacagg gctggatcac catcgacggc actttctgag 240
tactcagtgc agcaaagaaa gactacagac atctcaatgg caggggtgag aaataagaaa 300
ggctgctgac tttaccatct gaggccacac atctgctgaa atggagataa ttaacatcac 360
tagaaacagc aagatgacaa tataatgtct aagtagtgac atgtttttgc acatttccag 420
cccctttaaa tatccacaca cacaggaagc acaaaaggaa gcacagagat ccctgggaga 480
aatgcccggc cgccatcttg ggtcatcgat gagcctcgcc ctgtgcctgg tcccgcttgt 540
gagggaagga cattagaaaa tgaattgatg tgttccttaa aggatgggca ggaaaacaga 600
tcctgttgtg gatatttatt tgaacgggat tacagatttg aaatgaagtc acaaagtgag 660
cattaccaat gagaggaaaa cagacgagaa aatcttgatg gcttcacaag acatgcaaca 720
aacaaaatgg aatactgtga tgacatgagg cagccaagct ggggaggaga taaccacggg 780
gcagagggtc aggattctgg ccctgctgcc taaactgtgc gttcataacc aaatcatttc 840
atatttctaa ccctcaaaac aaagctgttg taatatctga tctctacggt tccttctggg 900
cccaacattc tccatatatc cagccacact catttttaat atttagttcc cagatctgta 960
ctgtgacctt tctacactgt agaataacat tactcatttg ttcaaagacc cttcgtgttg 1020
ctgcctaata tgtagctgac tgtttttcct aaggagtgtt ctggcccagg ggatctgtga 1080
acaggctggg aagcatctca agatctttcc agggttatac ttactagcac acagcatgat 1140
cattacggag tgaattatct aatcaacatc atcctcagtg tctttgccca tactgaaatt 1200
catttcccac ttttgtgccc attctcaaga cctcaaaatg tcattccatt aatatcacag 1260
gattaacttt tttttttaac ctggaagaat tcaatgttac atgcagctat gggaatttaa 1320
ttacatattt tgttttccag tgcaaagatg actaagtcct ttatccctcc cctttgtttg 1380
atttttttc cagtataaag ttaaaatgct tagccttgta ctgaggctgt atacagcaca 1440
gcctctcccc atccctccag ccttatctgt catcaccatc aacccctccc ataccaccta 1500
aacaaaatct aacttgtaat tccttgaaca tgtcaggaca tacattattc cttctgcctg 1560
agaagctctt ccttgtctct taaatctaga atgatgtaaa gttttgaata agttgactat 1620

cttacttcat gcaaagaagg gacacatatg agattcatca tcacatgaga cagcaaatac 1680
taaaagtgta atttgattat aagagtttag ataaatatat gaaatgcaag agccacagag 1740
ggaatgttta tggggcacgt ttgtaagcct gggatgtgaa gcaaaggcag ggaacctcat 1800
agtatcttat ataatatact tcatttctct atctctatca caatatccaa caagcttttc 1860
acagaattca tgcagtgcaa atccccaaag gtaaccttta tccatttcat ggtgagtgcg 1920
ctttagaatt ttggcaaatc atactggtca cttatctcaa ctttgagatg tgtttgtcct 1980
tgtagttaat tgaaagaaat agggcactct tgtgagccac tttagggttc actcctggca 2040
ataaagaatt tacaaagagc tactcaggac cagttgttaa gagctctgtg tgtgtgtgtg 2100
tgtgtgtgtg agtgtacatg ccaaagtgtg cctctctctc ttgacccatt atttcagact 2160
taaaacaagc atgttttcaa atggcactat gagctgccaa tgatgtatca ccaccatatc 2220
tcattattct ccagtaaatg tgataataat gtcatctgtt aacataaaaa aagtttgact 2280
tcacaaaagc agctggaaat ggacaaccac aatatgcata aatctaactc ctaccatcag 2340
ctacacactg cttgacatat attgttagaa gcacctcgca tttgtgggtt ctcttaagca 2400
aaatacttgc attaggtctc agctggggct gtgcatcagg cggtttgaga aatattcaat 2460
tctcagcaga agccagaatt tgaattccct catcttttag gaatcattta ccaggtttgg 2520
agaggattca gacagctcag gtgctttcac taatgtctct gaacttctgt ccctctttgt 2580
gttcatggat agtccaataa ataatgttat ctttgaactg atgctcatag gagagaatat 2640
aagaactctg agtgatatca acattaggga ttcaaagaaa tattagattt aagctcacac 2700
tggtcaaaag gaaccaagat acaaagaact ctgagctgtc atcgtcccca tctctgtgag 2760
ccacaaccaa cagcaggacc caacgcatgt ctgagatcct taaatcaagg aaaccagtgt 2820
catgagttga attctcctat tatggatgct agcttctggc catctctggc tctcctcttg 2880
acacatatta gcttctagcc tttgcttcca cgacttttat cttttctcca acacatcgct 2940
taccaatcct ctctctgctc tgttgctttg gacttcccca caagaatttc aacgactctc 3000
aagtcttttc ttccatcccc accactaacc tgaatgccta gacccttatt tttattaatt 3060
tccaatagat gctgcctatg ggctatattg ctttagatga acattagata tttaaagctc 3120
aagaggttca aaatccaact cattatcttc tctttctttc acctccctgc tcctctccct 3180
atattactga ttgcactgaa cagcatggtc cccaatgtag ccatgcaaat gagaaaccca 3240
gtggctcctt gtggtacatg catgcaagac tgctgaagcc agaaggatga ctgattacgc 3300
ctcatgggtg gaggggacca ctcctgggcc ttcgtgattg tcaggagcaa gacctgagat 3360
gctccctgcc ttcagtgtcc tctgcatctc ccctttctaa tgaagatcca tagaatttgc 3420
tacatttgag aattccaatt aggaactcac atgttttatc tgccctatca atttttttaaa 3480
cttgctgaaa attaagtttt ttcaaaatct gtccttgtaa attacttttt cttacagtgt 3540
cttggcatac tatatcaact ttgattcttt gttacaactt ttcttactct tttatcacca 3600
aagtggcttt tattctcttt attattatta ttttctttta ctactatatt acgttgttat 3660

tattttgttc tctatagtat caatttattt gatttagttt caatttattt ttattgctga 3720
cttttaaaat aagtgattcg gggggtggga gaacagggga gggagagcat taggacaaat 3780
acctaatgca tgtgggactt aaaacctaga tgatgggttg ataggtgcag caaaccacta 3840
tggcacacgt atacctgtgt aacaaaccta cacattctgc acatgtatcc cagaacgtaa 3900
agtaaaattt aaaaaaaagt ga 3922

<210> 7
<211> 1574
<212> DNA
<213> Transgelin 1

<400> 7

tcaccacggc ggcagccctt taaacccctc acccagccag cgccccatcc tgtctgtccg 60
aacccagaca caagtcttca ctccttcctg cgagccctga ggaagccttg tgagtgcatt 120
ggctggggct tggagggaag ttgggctgga gctggacagg agcagtgggt gcatttcagg 180
caggctctcc tgaggtccca ggcgccagct ccagctccct ggctagggaa acccaccctc 240
tcagtcagca tgggggccca agctccaggc agggtgggct ggatcactag cgtcctggat 300
ctctctcaga ctgggcagcc ccgggctcat tgaaatgccc cggatgactt ggctagtgca 360
gaggaattga tggaaaccac cggggtgaga gggaggctcc ccatctcagc cagccacatc 420
cacaaggtgt gtgtaagggt gcaggcgccg gccggttagg ccaaggctct actgtctgtt 480
gcccctccag gagaacttcc aaggagcttt ccccagacat ggccaacaag ggtccttcct 540
atggcatgag ccgcgaagtg cagtccaaaa tcgagaagaa gtatgacgag gagctggagg 600
agcggctggt ggagtggatc atagtgcagt gtggccctga tgtgggccgc ccagaccgtg 660
ggcgcttggg cttccaggtc tggctgaaga atggcgtgat tctgagcaag ctggtgaaca 720
gcctgtaccc tgatggctcc aagccggtga aggtgcccga gaacccaccc tccatggtct 780
tcaagcagat ggagcaggtg gctcagttcc tgaaggcggc tgaggactat ggggtcatca 840
agactgacat gttccagact gttgacctct ttgaaggcaa agacatggca gcagtgcaga 900
ggaccctgat ggctttgggc agcttggcag tgaccaagaa tgatgggcac taccgtggag 960
atcccaactg gtttatgaag aaagcgcagg agcataagag ggaattcaca gagagccagc 1020
tgcaggaggg aaagcatgtc attggccttc agatgggcag caacagaggg gcctcccagg 1080
ccggcatgac aggctacgga cgacctcggc agatcatcag ttagagcgga gagggctagc 1140
cctgagcccg gccctccccc agctccttgg ctgcagccat cccgcttagc ctgcctcacc 1200
cacacccgtg tggtaccttc agccctggcc aagctttgag gctctgtcac tgagcaatgg 1260
taactgcacc tgggcagctc ctccctgtgc cccagcctc agcccaactt cttacccgaa 1320
agcatcactg ccttggcccc tccctcccgg ctgcccccat cacctctact gtctcctccc 1380
tgggctaagc aggggagaag cgggctgggg gtagcctgga tgtgggccaa gtccactgtc 1440
ctccttggcg gcaaaagccc attgaagaag aaccagccca gcctgccccc tatcttgtcc 1500

tggaatattt ttggggttgg aactcaaaaa aaaaaaaaaa aaatcaatct tttctcaaaa 1560
aaaaaaaaaa aaaa 1574

<210> 8
<211> 1360
<212> DNA
<213> Transgelin 2

<400> 8

```
gcccttgcct tgagtcagtg cgctgctctc cagcccgctt gaacgctccc cgcagccacc     60
gccacccatt ggaatggcca acaggggacc tgcatatggc ctgagccggg aggtgcagca    120
gaagattgag aaacaatatg atgcagatct ggagcagatc ctgatccagt ggatcaccac    180
ccagtgccga aaggatgtgg gccggcccca gcctggacgc gagaacttcc agaactggct    240
caaggatggc acggtgctat gtgagctcat taatgcactg taccccgagg ggcaggcccc    300
agtaaagaag atccaggcct ccaccatggc cttcaagcag atggagcaga tctctcagtt    360
cctgcaagca gctgagcgct atggcattaa caccactgac atcttccaaa ctgtggacct    420
ctgggaagga aagaacatgg cctgtgtgca gcggacgctg atgaatctgg gtgggctggc    480
agtagcccga gatgatgggc tcttctctgg ggatcccaac tggttcccta agaaatccaa    540
ggagaatcct cggaacttct cagataacca gctgcaagag ggcaagaacg tgatcgggtt    600
acagatgggc accaaccgcg gggcgtctca ggcaggcatg actggctacg ggatgccacg    660
ccagatcctc tgatcccacc ccaggccttg cccctgccct cccacgaatg gttaatatat    720
atgtagatat atattttagc agtgacattc ccagagagcc ccagagctct caagctcctt    780
tctgtcaggg tgggggttc agcctgtcct gtcacctctg aggtgcctgc tggcatcctc    840
tcccccatgc ttactaatac attcccttcc ccatagccat caaaactgga ccaactggcc    900
tcttcctttc ccctgggacc aaaatttagg ggcctcagtc cctcaccgcc atgccctggc    960
ctattctgtc tctccttctt cccccctggcc tgttctgtct ctgagctctg tgtcctccgt   1020
tcattccatg gctgggagtc actgatgctg cctctgcctt ctgatgctgg actggccttg   1080
cttctacaag tatgcttctc ccacagctgt ggctgcagga acttaattta tagggaggag   1140
cctgtggcag ctgctgcccc agccacagct gcactgactg tgctcaccac acatctgggg   1200
cagccttccc tggcaggggc cctcgtggct tctcattttc cattcccttc actgtggcta   1260
aggggtgggg tgaggggatg gagagggagg gctgcctacc atggtctggg gcttgaggaa   1320
gatgagtttg ttgatttaaa taaagaattt gtcattttg                           1360
```

<210> 9
<211> 130
<212> PRT
<213> Forward Reading Frame 1

<220>
<221> MISC_FEATURE
<222> (14)..(14)
<223> where Xaa represents a stop codon

<220>
<221> MISC_FEATURE
<222> (50)..(50)
<223> Where Xaa represents a stop codon

<220>
<221> MISC_FEATURE
<222> (52)..(52)

<223> where Xaa represents a stop codon

<220>
<221> MISC_FEATURE
<222> (86)..(86)
<223> Where Xaa represents a stop codon

<220>
<221> MISC_FEATURE
<222> (98)..(98)
<223> where xaa represents a stop codon

<400> 9

```
Phe Ile Asn Lys Tyr Lys Val Pro Asp Thr Pro Ser Ala Xaa Lys Ile
1               5                   10                  15

Lys Leu Thr His Leu Ile Leu His Lys Thr Cys Glu Ile Arg Asp Tyr
            20                  25                  30

Cys Tyr Ala His Phe Ser Asp Glu Glu Ser Gly Thr Glu Phe Thr Glu
        35                  40                  45

Trp Xaa Arg Xaa Asp Leu Asn Pro Gly Lys Gln Ala Leu Gly Leu Asp
    50                  55                  60

Ser Trp Ser Leu Cys Cys Val Pro Thr Leu Ser Gln Val Ser Gly Leu
65                  70                  75                  80

Val Tyr Ile Val Arg Xaa Val Glu Cys Thr Gly Ile Ala Arg Gly Leu
                85                  90                  95

Gln Xaa Arg Asp Asp Phe His Glu Cys Ser Phe Ile Ser Arg Lys Gln
            100                 105                 110

Arg Leu Leu Phe Gly Cys Lys Gly Val Trp Leu Glu Gly Arg Ser Gly
        115                 120                 125

Asp Arg
    130
```

<210> 10
<211> 130
<212> PRT
<213> Forward Reading Frame 2

<220>
<221> MISC_FEATURE
<222> (18)..(18)
<223> where Xaa represents a stop codon

<220>
<221> MISC_FEATURE
<222> (21)..(21)
<223> where Xaa represents a stop codon

<220>
<221> MISC_FEATURE
<222> (24)..(24)
<223> where Xaa represents a stop codon

<220>
<221> MISC_FEATURE
<222> (29)..(29)
<223> where Xaa represents a stop codon

<220>
<221> MISC_FEATURE
<222> (54)..(54)
<223> Where Xaa represents a stop codon

<220>
<221> MISC_FEATURE
<222> (61)..(61)
<223> where xaa represents a stop codon

<220>
<221> MISC_FEATURE
<222> (74)..(74)
<223> where Xaa represents a stop codon

<220>
<221> MISC_FEATURE
<222> (83)..(83)
<223> where Xaa represents a stop codon

<220>
<221> MISC_FEATURE
<222> (123)..(123)
<223> where Xaa represents a stop codon

<400> 10

```
Leu Leu Thr Asn Ile Lys Tyr Gln Thr Leu Gln Val Leu Arg Lys Leu
1               5                   10                  15

Asn Xaa Leu Ile Xaa Phe Tyr Xaa Lys Pro Val Arg Xaa Gly Thr Ile
            20                  25                  30

Val Met Pro Ile Phe Gln Met Arg Lys Val Gly Gln Ser Leu Leu Ser
        35                  40                  45

Gly Arg Asp Glu Ile Xaa Thr Gln Ala Ser Arg Leu Xaa Val Trp Thr
    50                  55                  60

Leu Gly His Ser Ala Leu Ser Pro His Xaa Ala Arg Tyr Leu Gly Leu
65                  70                  75                  80

Cys Thr Xaa Ser Gly Lys Trp Lys Ser Gln Glu Leu Leu Glu Asp Ser
                85                  90                  95
```

Ser Arg Gly Met Ile Phe Met Asn Val Leu Ser Ser Gln Glu Ser Lys
100 105 110

Gly Cys Phe Leu Gly Ala Lys Glu Phe Gly Xaa Lys Glu Asp Leu Glu
115 120 125

Ile Glu
130

<210> 11
<211> 130
<212> PRT
<213> Forward Reading Frame 3

<220>
<221> MISC_FEATURE
<222> (2)..(2)
<223> where Xaa represents a stop codon

<220>
<221> MISC_FEATURE
<222> (5)..(5)
<223> where xaa represents a stop codon

<220>
<221> MISC_FEATURE
<222> (16)..(16)
<223> where xaa represents a stop codon

<220>
<221> MISC_FEATURE
<222> (27)..(27)
<223> where Xaa represents a stop codon

<220>
<221> MISC_FEATURE
<222> (39)..(39)
<223> where Xaa represents a stop codon

<220>
<221> MISC_FEATURE
<222> (47)..(47)
<223> where Xaa represents a stop codon

<220>
<221> MISC_FEATURE
<222> (93)..(93)
<223> where Xaa represents a stop codon

<220>
<221> MISC_FEATURE
<222> (100)..(100)
<223> where Xaa represents a stop codon

<220>
<221> MISC_FEATURE

<222> (103)..(103)
<223> where Xaa represents a stop codon

<220>
<221> MISC_FEATURE
<222> (129)..(129)
<223> where Xaa represents a stop codon

<400> 11

```
Tyr Xaa Gln Ile Xaa Ser Thr Arg His Ser Lys Cys Leu Glu Asn Xaa
1               5                   10                  15

Ile Asn Ser Phe Asn Phe Thr Lys Asn Leu Xaa Asp Lys Gly Leu Leu
            20                  25                  30

Leu Cys Pro Phe Phe Arg Xaa Gly Lys Trp Asp Arg Val Tyr Xaa Val
        35                  40                  45

Val Glu Met Arg Phe Glu Pro Arg Gln Ala Gly Ser Arg Ser Gly Leu
    50                  55                  60

Leu Val Thr Leu Leu Cys Pro His Thr Lys Pro Gly Ile Trp Ala Cys
65                  70                  75                  80

Val His Ser Gln Val Ser Gly Asn His Arg Asn Cys Xaa Arg Thr Pro
                85                  90                  95

Val Glu Gly Xaa Phe Ser Xaa Met Phe Phe His Leu Lys Lys Ala Lys
            100                 105                 110

Ala Ala Phe Trp Val Gln Arg Ser Leu Ala Arg Arg Lys Ile Trp Arg
        115                 120                 125

Xaa Ser
    130
```

<210> 12
<211> 130
<212> PRT
<213> Reverse Reading Frame 1

<220>
<221> MISC_FEATURE
<222> (29)..(29)
<223> where Xaa represents a stop codon

<220>
<221> MISC_FEATURE
<222> (98)..(98)
<223> where Xaa represents a stop codon

<220>
<221> MISC_FEATURE

<222> (100)..(100)
<223> where Xaa represents a stop codon

<220>
<221> MISC_FEATURE
<222> (112)..(112)
<223> where Xaa represents a stop codon

<220>
<221> MISC_FEATURE
<222> (118)..(118)
<223> where Xaa represents a stop codon

<400> 12

```
Ala Leu Ser Pro Asp Leu Pro Ser Ser Gln Thr Pro Leu His Pro Lys
1               5                   10                  15

Ser Ser Leu Cys Phe Leu Glu Met Lys Glu His Ser Xaa Lys Ser Ser
            20                  25                  30

Leu Tyr Trp Ser Pro Leu Ala Ile Pro Val Ile Ser Thr Tyr Leu Thr
        35                  40                  45

Met Tyr Thr Ser Pro Asp Thr Trp Leu Ser Val Gly Thr Glu Gln Ser
    50                  55                  60

Asp Gln Glu Ser Arg Pro Arg Ala Cys Leu Pro Gly Phe Lys Ser His
65                  70                  75                  80

Leu Tyr His Ser Val Asn Ser Val Pro Leu Ser Ser Ser Glu Lys Trp
                85                  90                  95

Ala Xaa Gln Xaa Ser Leu Ile Ser Gln Val Phe Ser Lys Ile Lys Xaa
            100                 105                 110

Val Asn Leu Ile Phe Xaa Ala Leu Gly Val Ser Gly Thr Leu Tyr Leu
        115                 120                 125

Leu Ile
    130
```

<210> 13
<211> 130
<212> PRT
<213> Reverse Reading Frame 2

<220>
<221> MISC_FEATURE
<222> (24)..(24)
<223> where Xaa represents a stop codon

<220>
<221> MISC_FEATURE

<222> (38)..(38)
<223> where Xaa represents a stop codon

<220>
<221> MISC_FEATURE
<222> (42)..(42)
<223> where Xaa represents a stop codon

<220>
<221> MISC_FEATURE
<222> (47)..(47)
<223> where Xaa represents a stop codon

<220>
<221> MISC_FEATURE
<222> (85)..(85)
<223> where Xaa represents a stop codon

<220>
<221> MISC_FEATURE
<222> (115)..(115)
<223> where Xaa represents a stop codon

<220>
<221> MISC_FEATURE
<222> (129)..(129)
<223> where Xaa represents a stop codon

<220>
<221> MISC_FEATURE
<222> (130)..(130)
<223> where Xaa represents a stop codon

<400> 13

Leu Tyr Leu Gln Ile Phe Leu Leu Ala Lys Leu Leu Cys Thr Gln Lys
1               5                   10                  15

Ala Ala Phe Ala Phe Leu Arg Xaa Lys Asn Ile His Glu Asn His Pro
            20                  25                  30

Ser Thr Gly Val Leu Xaa Gln Phe Leu Xaa Phe Pro Leu Thr Xaa Leu
        35                  40                  45

Cys Thr Gln Ala Gln Ile Pro Gly Leu Val Trp Gly Gln Ser Arg Val
    50                  55                  60

Thr Lys Ser Pro Asp Leu Glu Pro Ala Cys Leu Gly Ser Asn Leu Ile
65                  70                  75                  80

Ser Thr Thr Gln Xaa Thr Leu Ser His Phe Pro His Leu Lys Asn Gly
                85                  90                  95

His Asn Asn Ser Pro Leu Ser His Arg Phe Leu Val Lys Leu Asn Glu
            100                 105                 110

Leu Ile Xaa Phe Ser Lys His Leu Glu Cys Leu Val Leu Tyr Ile Cys
        115                 120                 125

Xaa Xaa
    130

<210> 14
<211> 130
<212> PRT
<213> Reverse Reading Frame 3

<220>
<221> MISC_FEATURE
<222> (8)..(8)
<223> where Xaa represents a stop codon

<220>
<221> MISC_FEATURE
<222> (22)..(22)
<223> where xaa represents a stop codon

<220>
<221> MISC_FEATURE
<222> (57)..(57)
<223> where Xaa represents a stop codon

<220>
<221> MISC_FEATURE
<222> (64)..(64)
<223> where Xaa represents a stop codon

<220>
<221> MISC_FEATURE
<222> (70)..(70)

<223> where xaa represents a stop codon

<220>
<221> MISC_FEATURE
<222> (93)..(93)
<223> where xaa represents a stop codon

<220>
<221> MISC_FEATURE
<222> (107)..(107)
<223> where Xaa represents a stop codon

<220>
<221> MISC_FEATURE
<222> (108)..(108)
<223> where Xaa represents a stop codon

<220>
<221> MIST_FEATURE
<222> (110)..(110)
<223> where Xaa represents a stop codon

<220>
<221> MISC_FEATURE
<222> (113)..(113)
<223> where Xaa represents a stop codon

<400> 14

```
Ser Ile Ser Arg Ser Ser Phe Xaa Pro Asn Ser Phe Ala Pro Lys Lys
1               5                   10                  15

Gln Pro Leu Leu Ser Xaa Asp Glu Arg Thr Phe Met Lys Ile Ile Pro
            20                  25                  30

Leu Leu Glu Ser Ser Ser Asn Ser Cys Asp Phe His Leu Pro Asp Tyr
        35                  40                  45

Val His Lys Pro Arg Tyr Leu Ala Xaa Cys Gly Asp Arg Ala Glu Xaa
    50                  55                  60

Pro Arg Val Gln Thr Xaa Ser Leu Leu Ala Trp Val Gln Ile Ser Ser
65                  70                  75                  80

Leu Pro Leu Ser Lys Leu Cys Pro Thr Phe Leu Ile Xaa Lys Met Gly
                85                  90                  95
```

Ile Thr Ile Val Pro Tyr Leu Thr Gly Phe Xaa Xaa Asn Xaa Met Asn
100 105 110

Xaa Phe Asn Phe Leu Ser Thr Trp Ser Val Trp Tyr Phe Ile Phe Val
115 120 125

Asn Lys
130

<210> 15
<211> 29
<212> DNA
<213> Pspu1 Forward Primer

<400> 15
attgtgtttt gactgcttat ggtattttt 29

<210> 16
<211> 28
<212> DNA
<213> Pspu1 Reverse Primer

<400> 16
cagagaaatg ttcacttacc aaacacat 28

<210> 17
<211> 30
<212> DNA
<213> Pspu2 Forward Primer

<400> 17
aatcaatcca tttgcttgtt ataagtctag 30

<210> 18
<211> 29
<212> DNA
<213> Pspu2 Reverse Primer

<400> 18
aaattaggac attcccagat aaacaaata 29

<210> 19
<211> 21
<212> DNA
<213> Beta-actin Forward Primer

<400> 19
aaccgtgaga agatgaccca g 21

<210> 20
<211> 21
<212> DNA
<213> Beta-actin Reverse Primer

<400> 20
gtgaggatct tcatgaggta g 21

<210> 21
<211> 19
<212> DNA
<213> Pspu8 Forward Primer

<400> 21
ccctttctcg tatggctta 19

<210> 22
<211> 20
<212> DNA
<213> Pspu28 Reverse Primer

<400> 22
gctgtgagag gacagggaag 20

<210> 23
<211> 27
<212> DNA
<213> Pspu43 Forward Primer

<400> 23
aacaaatata aagtaccaga cactcca 27

<210> 24
<211> 23
<212> DNA
<213> Pspu43 Reverse Primer

<400> 24
atctccagat cttccttcta gcc 23

<210> 25
<211> 238
<212> PRT
<213> PSA Amino Acid Sequence

<400> 25

Met Trp Val Pro Val Val Phe Leu Thr Leu Ser Val Thr Trp Ile Gly
1               5                   10                  15

Ala Ala Pro Leu Ile Leu Ser Arg Ile Val Gly Gly Trp Glu Cys Glu
            20                  25                  30

Lys His Ser Gln Pro Trp Gln Val Leu Val Ala Ser Arg Gly Arg Ala
        35                  40                  45

Val Cys Gly Gly Val Leu Val His Pro Gln Trp Val Leu Thr Ala Ala
    50                  55                  60

His Cys Ile Arg Asn Lys Ser Val Ile Leu Leu Gly Arg His Ser Leu
65                  70                  75                  80

Phe His Pro Glu Asp Thr Gly Gln Val Phe Gln Val Ser His Ser Phe
                85                  90                  95

Pro His Pro Leu Tyr Asp Met Ser Leu Leu Lys Asn Arg Phe Leu Arg
            100                 105                 110

Pro Gly Asp Asp Ser Ser His Asp Leu Met Leu Leu Arg Leu Ser Glu
        115                 120                 125

Pro Ala Glu Leu Thr Asp Ala Val Lys Val Met Asp Leu Pro Thr Gln
    130                 135                 140

Glu Pro Ala Leu Gly Thr Thr Cys Tyr Ala Ser Gly Trp Gly Ser Ile
145                 150                 155                 160

Glu Pro Glu Glu Phe Leu Thr Pro Lys Lys Leu Gln Cys Val Asp Leu
                165                 170                 175

His Val Ile Ser Asn Asp Val Cys Ala Gln Val His Pro Gln Lys Val
            180                 185                 190

Thr Lys Phe Met Leu Cys Ala Gly Arg Trp Thr Gly Gly Lys Ser Thr
        195                 200                 205

Cys Ser Trp Val Ile Leu Ile Thr Glu Leu Thr Met Pro Ala Leu Pro
    210                 215                 220

Met Val Leu His Gly Ser Leu Val Pro Trp Arg Gly Gly Val
225                 230                 235

<210> 26
<211> 201
<212> PRT
<213> Transgelin 1 Amino Acid Sequence

<400> 26

Met Ala Asn Lys Gly Pro Ser Tyr Gly Met Ser Arg Glu Val Gln Ser
1               5                   10                  15

Lys Ile Glu Lys Lys Tyr Asp Glu Glu Leu Glu Glu Arg Leu Val Glu
            20                  25                  30

Trp Ile Ile Val Gln Cys Gly Pro Asp Val Gly Arg Pro Asp Arg Gly
        35                  40                  45

Arg Leu Gly Phe Gln Val Trp Leu Lys Asn Gly Val Ile Leu Ser Lys
    50                  55                  60

Leu Val Asn Ser Leu Tyr Pro Asp Gly Ser Lys Pro Val Lys Val Pro
65                  70                  75                  80

Glu Asn Pro Pro Ser Met Val Phe Lys Gln Met Glu Gln Val Ala Gln
                85                  90                  95

Phe Leu Lys Ala Ala Glu Asp Tyr Gly Val Ile Lys Thr Asp Met Phe
            100                 105                 110

Gln Thr Val Asp Leu Phe Glu Gly Lys Asp Met Ala Ala Val Gln Arg
        115                 120                 125

Thr Leu Met Ala Leu Gly Ser Leu Ala Val Thr Lys Asn Asp Gly His
    130                 135                 140

Tyr Arg Gly Asp Pro Asn Trp Phe Met Lys Lys Ala Gln Glu His Lys
145                 150                 155                 160

Arg Glu Phe Thr Glu Ser Gln Leu Gln Glu Gly Lys His Val Ile Gly
                165                 170                 175

Leu Gln Met Gly Ser Asn Arg Gly Ala Ser Gln Ala Gly Met Thr Gly
            180                 185                 190

Tyr Gly Arg Pro Arg Gln Ile Ile Ser
        195                 200

<210> 27
<211> 199
<212> PRT
<213> Transgelin 2 Amino Acid sequence

<400> 27

```
Met Ala Asn Arg Gly Pro Ala Tyr Gly Leu Ser Arg Glu Val Gln Gln
1               5                   10                  15

Lys Ile Glu Lys Gln Tyr Asp Ala Asp Leu Glu Gln Ile Leu Ile Gln
            20                  25                  30

Trp Ile Thr Thr Gln Cys Arg Lys Asp Val Gly Arg Pro Gln Pro Gly
        35                  40                  45

Arg Glu Asn Phe Gln Asn Trp Leu Lys Asp Gly Thr Val Leu Cys Glu
    50                  55                  60

Leu Ile Asn Ala Leu Tyr Pro Glu Gly Gln Ala Pro Val Lys Lys Ile
65                  70                  75                  80

Gln Ala Ser Thr Met Ala Phe Lys Gln Met Glu Gln Ile Ser Gln Phe
                85                  90                  95

Leu Gln Ala Ala Glu Arg Tyr Gly Ile Asn Thr Thr Asp Ile Phe Gln
            100                 105                 110

Thr Val Asp Leu Trp Glu Gly Lys Asn Met Ala Cys Val Gln Arg Thr
        115                 120                 125

Leu Met Asn Leu Gly Gly Leu Ala Val Ala Arg Asp Asp Gly Leu Phe
    130                 135                 140

Ser Gly Asp Pro Asn Trp Phe Pro Lys Lys Ser Lys Glu Asn Pro Arg
145                 150                 155                 160

Asn Phe Ser Asp Asn Gln Leu Gln Glu Gly Lys Asn Val Ile Gly Leu
                165                 170                 175

Gln Met Gly Thr Asn Arg Gly Ala Ser Gln Ala Gly Met Thr Gly Tyr
            180                 185                 190

Gly Met Pro Arg Gln Ile Leu
        195
```

<210> 28
<211> 329
<212> DNA
<213> EST 1

<400> 28

```
ctttcttttt ttttgctcta tctccagatc ttccttctag ccaaactcct ttgcacccaa    60
aaagcagcct ttgctttctt gagatgaaag aacattcatg aaaatcatcc ctctactgga   120
gtcctctagc aattcctgtg atttccactt acctgactat gtacacaagc ccagatacct   180
ggcttagtgt ggggacagag cagagtgacc aagagtccag acctagagcc tgcttgcctg   240
ggttcaaatc tcatctctac cactcagtaa actctgtccc actttcctca tctgaaaaat   300
gggcataaca atagtccctt atctacagg                                     329
```

<210> 29
<211> 312
<212> DNA
<213> EST 2

<400> 29

```
tttttttttt ttttgctcta tctccagatc ttccttctag ccaaactcct ttgcacccaa    60
aaagcagcct ttgctttctt gagatgaaag aacattcatg aaaatcatcc ctctactgga   120
gtcctctagc aattcctgtg atttccactt acctgactat gtacacaagc ccagatacct   180
ggcttagtgt ggggacagag cagagtgacc aagagtccag acctagagcc tgcttgcctg   240
ggttcaaatc tcatctctac cactcagtaa actctgtccc actttcctca tctgaaaaat   300
gggcataaca at                                                       312
```

<210> 30
<211> 329
<212> DNA
<213> EST 3

<400> 30

```
tttttttttt tttttgctct atctccagat cttccttcta gccaaactcc tttgcaccca    60
aaaagcagcc tttgctttct tgagatgaaa gaacattcat gaaaatcatc cctctactgg   120
agtcctctag caattcctgt gatttccact tacctgacta tgtacacaag cccagatacc   180
tggcttagtg tggggacaga gcaaagtgac caagagtcca aacctagagc ctgcttgcct   240
gggttcaaat ctcatctcta ccactcagta aactctgtcc cactttcctc atctgaaaaa   300
tgggcataac aatagtccct tatctcaca                                     329
```

<210> 31
<211> 373
<212> DNA
<213> EST 4

<400> 31

ctttcttttt ttttgctcta tctccagatc ttccttctag ccaaactcct ttgcacccaa 60
aaagcagcct ttgctttctt gagatgaaag aacattcatg aaaatcatcc ctctactgga 120
gtcctctagc aattcctgtg atttccactt acctgactat gtacacaagc ccagatacct 180
ggcttagtgt ggggacagag cagagtgacc aagagtccag acctagagcc tgcttgcctg 240
ggttcaaatc tcatctctac cactcagtaa actctgtccc actttcctca tctgaaaaat 300
gggcataaca atagtccctt atctcacagg tttttagtaa aattaaatga gttaatttaa 360
ttttctaagc act 373

<210> 32
<211> 421
<212> DNA
<213> EST 5

<400> 32

tttattaaca aatataaagt accagacact ccaagtgctt agaaaattaa attaactcat 60
ttaattttac taaaaacctg tgagataagg gactattgtt atgcccattt ttcagatgag 120
gaaagtggga cagagtttac tgagtggtag agatgagatt tgaacccagg caagcaggct 180
ctaggtctgg actcttggtc actctgctct gtccccacac taagccaggt atctgggctt 240
gtgtacatag tcaggtaagt ggaaatcaca ggaattgcta gaggactcca gtagagggat 300
gattttcatg aatgttcttt catctcaaga aagcaaaggc tgctttttgg gtgcaaagga 360
gtttggctag aaggaagatc tggagataga gcaaaaaaaa agaaagaaaa aaaaaaaaaa 420
a 421

<210> 33
<211> 296
<212> DNA
<213> EST 6

<400> 33

tttttttttt gctctatctc cagatcttcc ttctagccaa actcctttgc acccaaaaag 60
cagcctttgc tttcttgaga tgaaagaaca ttcatggaaa tcatccctct actggagtcc 120
tctagcaatt cctgtgattt ccacttacct gactatgtac acaagcccag atacctggct 180
tagtgtgggg acagagcaga gtgaccaaga gtccagacct agagcctgct tgcctgggtt 240
caaatctcat ctctaccact cagtaaactc tgtcccactt tcctcatctg gtcgac 296

<210> 34
<211> 51
<212> PRT
<213> PCA 3A Amino Acid sequence

<400> 34

```
Met Phe Leu His Ile Ser Ser Pro Phe Lys Tyr Pro His Thr Gln Glu
1               5                   10                  15

Ala Gln Lys Glu Ala Gln Arg Ser Leu Gly Glu Met Pro Gly Arg His
            20                  25                  30

Leu Gly Ser Ser Met Ser Leu Ala Leu Cys Leu Val Pro Leu Val Arg
        35                  40                  45

Gln Gly His
    50
```

<210> 35
<211> 51
<212> PRT
<213> PCA 3B Amino Acid sequence

<400> 35

```
Met Phe Leu His Ile Ser Ser Pro Phe Lys Tyr Pro His Thr Gln Glu
1               5                   10                  15

Ala Gln Lys Glu Ala Gln Arg Ser Leu Gly Glu Met Pro Gly Arg His
            20                  25                  30

Leu Gly Ser Ser Met Ser Leu Ala Leu Cys Leu Val Pro Leu Val Arg
        35                  40                  45

Glu Gly His
    50
```

## Description

### Field of the Invention

This invention relates to *in vitro* methods of detecting, diagnosing, monitoring and treating prostate cancer (PRC), prostatic intraepithelial neoplasia (PIN) or a predisposition to same; and to markers useful in such methods.

### Background of the Invention

Prostate cancer is the most commonly diagnosed cancer in European and North American men. In those regions prostate cancer is second only to lung cancer as a cause of death in men (Frankel *et al.* 2003). The disease is also on the increase in other parts of the world such as Japan, and may reflect an adoption of Western diets in Eastern Counties.

Prostate cancer is a disease of the aging male. Forty percent of men aged 60 years have localised prostate tumours, and more than 75 percent of men aged 85 years and older have prostate cancer. The cancer is a latent disease often present without other signs of disease, and can take up to 10 years from diagnosis to death. The disease's usual progression is from a well defined mass within the prostate to a breakdown and invasion of the lateral margins of the prostate, followed by metastasis to regional lymph nodes, and/or metastasis to bone marrow.

Published PCT application WO 01/070979 describes the use of ESTs for the identification of ovarian cancer.

Published US patent application US 2004/142325 describes methods for annotating biomolecular sequences, such as sequences associated with pathology.

EMBL database entries having EBI accession numbers EMBL:BX109457 and EMBL:AI611685 describe ESTs identified in the human genome.

Chakrabarti et al (2002) Cancer Genetics and Cytogenetics 139:115-125 describe the profiling of differential expression of mRNA in normal, benign and metastic prostate cell lines.

Yu et al (2004) J. Clin. Oncology 22:2790-2799 describe gene expression alterations in prostate cancer.

Ernst et al (2002) Verh. Dtsch. Ges. Path. 86:165-175 describe gene expression profiling in prostate cancer.

Oudes et al (2005) BMC Cancer 5:86-97 describe methods for identification of genes involved in prostate cancer progression.

Prostatic intraepithelial neoplasia (PIN) is a specific type of lesion that is believed to be a precursor to prostate cancer (McNeal and Bostwick, 1986). If diagnosed early, patients are currently treated by androgen ablation therapy. Ablation therapy has undesirable side effects such as loss of libido and potency. As the disease develops it becomes androgen independent. At that stage surgery (radical prostatectomy) is the main option employed. The patient's life may be saved but common outcomes of surgery are incontinence, erectile dysfunction and urinary leakage.

It remains unclear why prostate cancer develops and what determines its progression. Moreover, tests for prostate cancer are limited primarily to physical examinations, needle biopsy and bone scan. Currently, a raised level of circulating prostate specific antigen (PSA) is most commonly used to predict the presence of prostate cancer. This is the only common non-invasive screen for prostate cancer.

However, the PSA test is not diagnostic. A raised level of PSA can be caused by other non-related factors such as benign prostate hyperplasia (BPH) and prostatitis. It is not specific to the disease state and is unable to indicate risk of death (Frankel *et al.,* 2003). Clinical decisions cannot be informed by the PSA screen alone. The PSA test is unable to distinguish between malignant and nonmalignant forms or predict how a lesion may progress. Furthermore, not all prostate cancers give rise to an increase in serum PSA concentrations. Indeed 85% of men with raised PSA levels who undergo radical treatment (prostatectomy) do so without prospective benefit (Frankel *et al.,* 2003).

Accordingly, there is a need for more reliable methods of diagnosing prostate cancer or its precursor, and for monitoring the disease over time (active monitoring), particularly at an early stage so that treatment options remain open. There is also a need for markers useful in determining patient status.

It is therefore an object of the invention to provide a marker useful in determining the prostate cancer status of a patient, or which at least provides the public with a useful choice.

It is a further object of the present invention to provide methods for diagnosing and/or prognosing prostate cancer or prostatic intraepithelial neoplasia or a predisposition thereto.

### Summary of the Invention

In a first aspect, the invention provides an *in vitro* method of (a) diagnosing prostatic intraepithelial neoplasia (PIN) or prostate cancer (PRC) or a predisposition to PIN or PRC in a patient, (b) monitoring a course of treatment for PIN or PRC in a patient, or (c) for prognosing
PIN or PRC in a patient, comprising

obtaining a PIN or PRC expression profile from a sample from the patient, the expression profile comprising a pattern of marker expression including a nucleic acid molecule, and
comparing the expression profile to an expression profile of a control sample, wherein the nucleic acid molecule comprises:

(i) the sequence of SEQ ID NO:3,
(ii) a sequence which hybridises under stringent conditions to SEQ ID NO:3,
(iii) a sequence which has 70%, 75%, 80%, 90%, 95%, or 99% sequence identity to SEQ ID NO:3, or
(iv) an at least 20 nucleotide fragment of (i) or a complement thereof, which fragment or complement hybridizes under stringent conditions to:

(a) a nucleic acid molecule of (i), (ii) or (iii) or a complement thereof;
(b) a reverse complement of (a).

The nucleic acid molecule may be at least 30, at least 40, at least 50 nucleotides, at least 60 nucleotides, at least 70 nucleotides, at least 80 nucleotides, at least 90 nucleotides, or preferably is at least 100 nucleotides.
Preferably, the expression profile further comprises one or more nucleic acid sequences which bind one or more of transgelin 1 (SEQ ID NO:7), (transgelin 2 (SEQ ID NO:8), PCA3 (SEQ ID NO:6), and PSA (SEQ ID NO:5).
The invention also provides an in vitro assay for diagnosing prostatic intraepithelial neoplasia (PIN) or prostate cancer (PRC) in a patient, the assay comprising detecting the presence of a nucleic acid molecule as defined in the first aspect of the invention, preferably PSPU43 (SEQ ID NO:3) in a sample, the detection comprising:

(a) contacting the sample with a nucleotide probe which hydridises to the nucleic acid sequence of the invention, as defined in the first aspect, preferably PSPU43 (SEQ ID NO:3), under stringent hybridisation conditions; and
(b) detecting the presence of a hybridisation complex in the sample.

Preferably, the probe is a labelled probe, commonly a fluorescently labelled probe. In one embodiment the probe is a complement of SEQ ID NO:3.
Preferably, the diagnosing comprises testing for, prognosing or monitoring response for treatment of PIN or PRC in a patient.
Preferably, the nucleic acid molecule is SEQ ID NO: 3 (PSPU43).
In the *in vitro* diagnostic, determining or monitoring methods of the invention, the level of expression of a nucleic acid molecule of the invention as defined in the first aspect, preferably PSPU 43 (SEQ ID NO:3), in a patient sample, is determined by a method comprising (i) direct or indirect measurement of the nucleic acid molecule, or (ii)

(a) amplifying the DNA sequence of the nucleic acid molecule or a complement thereof; or
(b) amplifying the cDNA sequence of the nucleic acid molecule or a complement thereof; and
(c) measuring the level of one or more of DNA, cDNA or RNA in said sample.

Preferably, the nucleic acid molecule is employed in an *in situ* hybridisation or RT-PCR analysis.
Preferably, the DNA or cDNA is amplified using PCR.
Preferably, the level of DNA, cDNA, or RNA in the sample is measured using electrophoresis.
In a further aspect, the invention relates to an *in vitro* method of diagnosing prostatic intraepithelial neoplasia (PIN), prostate cancer (PRC) status or a predisposition to developing PIN or PRC in a patient, the method comprising determining the expression level of a nucleic acid molecule of the invention, as defined in the first aspect, preferably PSPU 43 (SEQ ID NO:3) in a patient sample, wherein an alteration in expression level compared to a control level of said nucleic acid molecule indicates that the patient has PIN, PRC, or is at risk of developing PIN or PRC.
Preferably, the alteration is an increase in expression level.
Most usually, the alteration in expression level is at least 10% above the normal control level. The control level is conveniently the expression level of PSPU 43 measured in nonnal prostate.
The sample may comprise normal prostate cells, or PIN or PRC cells, and preferably epithelial cells from normal prostate, PIN or prostate cancer tumour. Preferably, the sample is a urine, lymph, blood, plasma, semen, prostate massage fluid, or prostate tissue sample.
In another aspect, the invention relates to an *in vitro* method of monitoring response to treatment of PIN or PRC in a subject, the method comprising determining the expression level of a nucleic acid molecule of the invention, as defined in the first aspect, preferably PSPU 43 (SEQ ID NO:3) in a patient sample, and comparing the level of said nucleic acid molecule to a control level, wherein a statistically significant change in the determined level from the control level is indicative of a response to the treatment

Preferably, these *in vitro* determining, testing and monitoring methods as defined above further comprise determining the level of one or more additional markers of PIN or PRC and comparing the levels to marker levels from a control, wherein a significant deviation in the levels from a control level, together with a statistically significant increase in the level of a nucleic acid molecule of the invention, as defined in the first aspect, preferably PSPU 43 (SEQ ID NO: 3) is indicative of PRC or PIN, or can be used to monitor PIN or PRC status.

The additional markers may be one or more markers selected from transgelin 1 (SEQ ID NO:7), transgelin 2 (SEQ ID NO:8), PCA 3 (SEQ ID NO:6) and PSA (SEQ ID NO:5).

Preferably, in the *in vitro* diagnostic, testing or monitoring method of the invention, transgelin 2 (SEQ ID NO: 8) is used as a reference marker.

The invention also relates to the use of a nucleic acid molecule as defined in the first aspect in an *in vitro* method of diagnosing PIN or PRC in a patient, preferably the nucleic acid molecule is SEQ ID NO:3 (PSPU43). Preferably, the diagnosing comprises testing for, prognosing or monitoring response for treatment of PIN or PRC in a patient.

DESCRIPTION OF THE DRAWINGS

The invention will now be described with reference to the figures in accompanying drawings in which:

**Figure 1:** Shows the consensus sequence for *Pspu43.* The consensus sequence is generated from contigs of EST's comprising UniGene cluster Hs.161160;

**Figure 2:** Shows the dissociation curves for SYBR Green qPCR assays using primer sets for *Pspu1, Pspu2, Pspu8, Pspu43,* T1 and T2. The cDNA template used was generated from the PC3 cell line;

**Figure 3:** Shows the qPCR efficiency of each primer and probe/primer combination used to test for marker expression in prostate tissue. Standard curves used a universal reference cDNA as template;

**Figure 4:** Shows the average raw CT values from cDNA templates generated from matched tissue pairs. Each error bar indicates +/- 1 standard deviation calculated from duplicate qPCR reactions;

**Figure 5:** Shows the relative amount of cDNA per sample for each marker corrected for genomic DNA contamination. Relative quantity was determined from the average CT and the line of best fit calculated for the standard curve run for each marker. A similar calculation was performed for qPCR on RNA only templates (no RT reaction) and the relative quantity values for this reaction subtrated from the values determined for cDNA templates; and

**Figure 6:** Shows the translation in six reading frames of nucleotide sequence *Pspu43.* The single letter amino acid code has been used. - represents a stop codon.

<u>Definitions</u>

The term "comprising" as used in this specification and claims means "consisting at least in part of", that is to say when interpreting statements in this specification and claims which include the term, the features, prefaced by that term in each statement, all need to be present but other features can also be present. Related terms such as "comprise" and "comprised" are to be interpreted in a similar manner.

It is intended that reference to a range of numbers disclosed herein (for example 1 to 10) also incorporates reference to all related numbers within that range (for example, 1, 1.1, 2, 3, 3.9, 4, 5, 6, 6.5, 7, 8, 9 and 10) and also any range of rational numbers within that range (for example 2 to 8, 1.5 to 5.5 and 3.1 to 4.7) and, therefore, all sub-ranges of all ranges expressly disclosed herein are expressly disclosed. These are only examples of what is specifically intended and all possible combinations of numerical values between the lowest value and the highest value enumerated are to be considered to be expressly stated in this application in a similar manner.

The term "marker" as used herein refers to a segment of DNA with an identifiable physical location on a chromosome. A marker may be a gene or other identifiable nucleic acid sequence, such as an open reading frame, a portion of an intron or an intergenic genomic DNA segment

A "control level" of a marker as used herein refers to a level of expression detected in a sample from a normal healthy individual, or a level determined based on a population of individuals not known to be suffering from PRC or PIN. The control level may be a single expression pattern derived from a single reference population or may be a plurality of expression patterns. For example, the control level can be a database of expression patterns from previously tested cells. Another example may be a ratiometric measure between a reference marker (e.g. transgelin 2) and a marker used in a method of the invention. Alternatively, the control level may be one or more readings or the mean of such readings taken from the same patient at an earlier time.

The term "polynucleotide(s)," as used herein, means a single or double-stranded deoxyribonucleotide or ribonucleotide polymer of any length, and include as non-limiting examples, coding and non-coding sequences of a gene, sense and antisense sequences, exons, introns, genomic DNA, cDNA, pre-mRNA, mRNA, rRNA, siRNA, miRNA, tRNA, ribozymes, recombinant polynucleotides, isolated and purified naturally occurring DNA or RNA sequences, synthetic RNA and DNA sequences, nucleic acid probes, primers, fragments Reference to a nucleic acid molecule is to be similarly understood.

"Antisense" as used herein generally means DNA or RNA or a combination of same that is complementary to at least a portion of an mRNA molecule encoding a polypeptide and capable of interfering with a post-transcriptional event such as mRNA translation.

A "fragment" of a polynucleotide sequence provided herein is a subsequence of contiguous nucleotides that is capable of specific hybridization to a target of interest, e.g., a sequence that is at least 10 nucleotides in length. The fragments used in a method of the invention comprise at least 20 nucleotides, preferably at least 30 nucleotides, more preferably at least 40 nucleotides, more preferably at least 50 nucleotides and most preferably at least 60 nucleotides of contiguous nucleotides of a polynucleotide of the invention. A fragment of a polynucleotide sequence can be used in antisense, gene silencing, triple helix or ribozyme technology, or as a primer, a probe, included in a microarray, or used in polynucleotide-based selection methods of the invention.

The term "patient" as used herein is preferably a mammalian patient and includes humans, and non-human mammals such as cats, dogs, horses, cows, sheep, deer, mice, possum and primates (including gorillas, rhesus monkeys and chimpanzees) and other domestic farm or zoo animals. Preferably, the mammal is human.

The term "treat", "treating" or "treatment" and "preventing" refer to therapeutic and phrophylactic measures which stop, reverse or lessen prostate cancer or PIN. The patient shows observable or measurable (statistically significant) reduction in one or more of: number of cancer or PIN cells; tumour size; symptoms associated with the cancer or PIN; inhibition of: tumour size; tumour growth; metastasis; improvement in quality of life.

A "patient sample" as used herein means a biological sample derived from a patient to be screened. The biological sample may be any suitable sample known in the art in which the expression of the selected markers can be detected. Included are individual cells and cell populations obtained from bodily tissues or fluids. Examples of suitable body fluids to be tested are plasma, prostate massage fluid, blood, semen, lymph and urine.

Preferably, the sample to be tested comprises epithelial cells derived from tissue that is known or suspected to exhibit PIN or PRC, most usually prostate tissue. Samples from healthy individuals may also be tested. A normal healthy individual is one with no clinical symptoms of PIN or PRC or benign prostate hypoplasia (BPH) and preferably under 30 years of age. Alternately, normal healthy cells from normal regions of a prostate biopsy may be used as controls in the methods.

The term "primer" refers to a short polynucleotide, usually having a free 3'OH group, that is hybridized to a template and used for priming polymerization of a polynucleotide complementary to the target.

The term "probe" refers to a short polynucleotide that is used to detect a polynucleotide sequence, that is complementary to the probe, in a hybridization-based assay. The probe may consist of a "fragment" of a polynucleotide as defined herein.

The term "polypeptide", as used herein, encompasses amino acid chains of any length, but preferably at least 5 amino acids, preferably at least 10, preferably at least 15, preferably at least 20, preferably at least 25, preferably at least 30, preferably at least 40, preferably at least 50, preferably at least 60, preferably at least 70, preferably at least 80, preferably at least 90, preferably at least 100, preferably at least 110, preferably at least 120, preferably at least 125 amino acids, and including full-length proteins, in which amino acid residues are linked by covalent peptide bonds. Polypeptides may be purified natural products, or may be produced partially or wholly using recombinant or synthetic techniques. The term may refer to a polypeptide, an aggregate of a polypeptide such as a dimer or other multimer, a fusion polypeptide, a polypeptide fragment, a polypeptide variant, or derivative thereof.

A "fragment" of a polypeptide is a subsequence of the polypeptide that perfonns a function that is required for the biological activity and/or provides three dimensional structure of the polypeptide. The term may refer to a polypeptide, an aggregate of a polypeptide such as a dimer or other multimer, a fusion polypeptide, a polypeptide fragment, a polypeptide variant, or derivative thereof.

The term "isolated" as applied to the polynucleotide or polypeptide sequences disclosed herein is used to refer to sequences that are removed from their natural cellular environment. An isolated molecule may be obtained by any method or combination of methods including biochemical, recombinant, and synthetic techniques. The polynucleotide or polypeptide sequences may be prepared by at least one purification step.

The term "recombinant" refers to a polynucleotide sequence that is removed from sequences that surround it in its natural context and/or is recombined with sequences that are not present in its natural context.

A "recombinant" polypeptide sequence is produced by translation from a "recombinant" polynucleotide sequence.

As used herein, the term "variant" refers to polynucleotide or polypeptide sequences different from the specifically identified sequences, wherein one or more nucleotides or amino acid residues is deleted, substituted, or added. Variants may be naturally occurring allelic variants, or non-naturally occurring variants. Variants may be from the same or from other species and may encompass homologues, paralogues and orthologues. In certain embodiments, variants of the polypeptides and polynucleotides possess biological activities that are the same or similar to those of the polypeptides or polynucleotides. The term "variant" with reference to polynucleotides and polypeptides encompasses all forms of polynucleotides and polypeptides as defined herein.

Variant polynucleotide sequences preferably exhibit at least 70%, more preferably at least 71 %, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequence of the present invention. Identity is found over a comparison window of at least 20 nucleotide positions, preferably at least 50 nucleotide positions, more preferably at least 100 nucleotide positions, and most preferably over the entire length of a polynucleotide used in a method of the invention.

Polynucleotide sequence identity may be calculated over the entire length of the overlap between a candidate and subject polynucleotide sequences using global sequence alignment programs (e.g. Needleman, S. B. and Wunsch, C. D. (1970) J. Mol. Biol. 48, 443-453). A full implementation of the Needleman-Wunsch global alignment algorithm is found in the needle program in the EMBOSS package (Rice,P. Longden,I. and Bleasby,A. EMBOSS: The European Molecular Biology Open Software Suite, Trends in Genetics June 2000, vol 16, No 6. pp.276-277) which can be obtained from hap:llwww.hgmp.mrc.ac.uk/Software/EMBOSS/. The European Bioinformatics Institute server also provides the facility to perform EMBOSS-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

Polynucleotide variants also encompass those which exhibit a similarity to one or more of the specifically identified sequences that is likely to preserve the functional equivalence of those sequences and which could not reasonably be expected to have occurred by random chance. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than $1 \times 10^{-5}$, more preferably less than $1 \times 10^{-6}$, more preferably less than $1 \times 10^{-9}$, more preferably less than $1 \times 10^{-12}$, more preferably less than $1 \times 10^{-15}$, more preferably less than $1 \times 10^{-18}$ and most preferably less than $1 \times 10^{-21}$ when compared with any one of the specifically identified sequences.

Use of BLASTN is preferred for use in the determination of sequence identity for polynucleotide variants suitable for use in methods according to the present invention.

The identity of polynucleotide sequences may be examined using the following UNIX command line parameters:

bl2seq -i nucleotideseq1 -j nucleotideseq2 -F F -p blastn

The parameter -F F turns off filtering of low complexity sections. The parameter -p selects the appropriate algorithm for the pair of sequences. The bl2seq program reports sequence identity as both the number and percentage of identical nucleotides in a line "Identities = ".

Polynucleotide sequence identity and similarity can also be determined in the following manner. The subject polynucleotide sequence is compared to a candidate polynucleotide sequence using sequence alignment algorithms and sequence similarity search tools such as in Genbank, EMBL, Swiss-PROT and other databases. Nucleic Acids Res 29:1-10 and 11-16, 2001 provides examples of online resources. BLASTN (from the BLAST suite of programs, version 2.2.13 March 2007 in bl2seq (Tatiana A. et al, FEMS Microbiol Lett. 174:247-250 (1999), Altschul et al., Nuc.Acis Res 25:3389-3402, (1997)), which is publicly available from NCBI (ftp://ftp.ncbi.nih.gov/blast/) or from NCB1 at Bethesda, Maryland, USA. The default parameters of bl2seq are utilized except that filtering of low complexity parts should be turned off.

Alternatively, variant polynucleotides hybridize to the specified polynucleotide sequence, or a complement thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30° C (for example, 10° C) below the melting temperature (Tm) of the native duplex (see generally, Sambrook et al., Eds, 1987, Molecular Cloning, A Laboratory Manual, 2nd Ed. Cold Spring Harbor Press; Ausubel et al., 1987, Current Protocols in Molecular Biology, Greene Publishing, incorporated herein by reference). Tm for polynucleotide molecules greater than about 100 bases can be calculated by the formula Tm = 81. 5+0.41%(G+ C-log (Na+) (Sambrook et al., Eds, 1987, Molecular Cloning, A Laboratory Manual, 2nd Ed. Cold Spring Harbor Press; Bolton and McCarthy, 1962, PNAS 84:1390). Typical stringent conditions for a polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in 1X SSC, 0.1% SDS at 65° C and two washes of 30 minutes each in 0.2X SSC, 0.1% SDS at 65°C.

In one embodiment stringent conditions use 50% formamide, 5 x SSC, 50 mM sodium phosphate (pH 6.8), 0.1% sodium pyrophosphate, 5 x Denhardt's solution, sonicated salmon sperm DNA (50 μg/ml), 0.1% SDS, and 10% dextran sulphate at 42°C, with washes at 42°C in 0.2 x SSC and 50% formamide at 55°C, followed by a wash comprising of 0.1 x SSC containing EDTA at 55°C.

With respect to polynucleotide molecules having a length less than 100 bases, exemplary stringent hybridization conditions are 5 to 10° C below Tm. On average, the Tm of a polynucleotide molecule of length less than 100 bp is reduced by approximately (500/oligonucleotide length)° C.

With respect to the DNA mimics known as peptide nucleic acids (PNAs) (Nielsen et al., Science. 1991 Dec 6; 254(5037):1497-500) Tm values are higher than those for DNA-DNA or DNA-RNA hybrids, and can be calculated using the formula described in Giesen et al., Nucleic Acids Res. 1998 Nov 1;26(21):5004-6. Exemplary stringent hybridization conditions for a DNA-PNA hybrid having a length less than 100 bases are 5 to 10° C below the Tm.

Variant polynucleotides also encompasses polynucleotides that differ from the sequences of the invention but that, as a consequence of the degeneracy of the genetic code, encode a polypeptide having similar activity to a polypeptide encoded by a polynucleotide of the present invention. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al., 1990, Science 247, 1306).

Variant polynucleotides due to silent variations and conservative substitutions in the encoded polypeptide sequence may be determined using the bl2seq program via the tblastx algorithm as described above.

The term "antisense-oligonucleotides" as used herein encompasses both nucleotides that are entirely complementary to the target sequence and those having a mismatch of one or more nucleotides, so long as the antisense-oligonucleotides can specifically hybridize to the target sequence. For example, the antisense-oligonucleotides disclosed herein include polynucleotides that have an identity of at least 70% or higher, preferably at least 75% or higher, at least 76%, at least 77%, at least 78%, at least 79%, at least 80% or higher, more preferably at least 81%, at least 82%, at least 83%, at least 84%, at least 85%, at least 86%, at least 87%, at least 88%, at least 89%, at least 90% or higher, even more preferably 91%, 92%, 93%, 94%, 95%, 96%, 97%, or 99% over a span of at least 15, at least 20, at least 30, at least 40, at least 50, preferably 75, preferably 100, more preferably 200 contiguous nucleotides, or the full length of a nucleic acid sequence disclosed herein preferably of PSPU 43 (SEQ ID NO:3). Algorithms known in the art as discussed above can be used to determine the identity. Furthermore, derivatives or modified products of the antisense-oligonucleotides can also be used as antisense-oligonucleotides.

The term "variant" with reference to polypeptides encompasses naturally occurring, recombinantly and synthetically produced polypeptides. Variant polypeptide sequences preferably exhibit at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, more preferably at least 61%, more preferably at least 62%, more preferably at least 63%, more preferably at least 64%, more preferably at least 65%, more preferably at least 66%, more preferably at least 67%, more preferably at least 68%, more preferably at least 69%, more preferably at least 70%, more preferably at least 71 %, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequence of the present invention. Identity is found over a comparison window of at least 20 amino acid positions, preferably at least 50 amino acid positions, more preferably at least 100 amino acid positions, and most preferably over the entire length of a polypeptide. Polypeptide variants also encompass those which exhibit a similarity to one or more of the specifically identified sequences that is likely to preserve the functional equivalence of those sequences and which could not reasonably be expected to have occurred by random chance.

Polypeptide sequence identity and similarity can be determined in the following manner. The subject polypeptide sequence is compared to a candidate polypeptide sequence using BLASTP (from the BLAST suite of programs, version 2.2.13 May 2007) in bl2seq, which is publicly available from NCBI (ftp://flp.ncbi.nih.gov/blast/). The default parameters of bl2seq are utilized except that filtering of low complexity regions should be turned off.

The similarity of polypeptide sequences may be examined using the following UNIX command line parameters:

bl2seq –i peptideseq1 –j peptideseq2 -F F –p blastp

Variant polypeptide sequences preferably exhibit an E value of less than 1 x 10 $^{-5}$, more preferably less than 1 x 10 $^{-6}$, more preferably less than 1 x 10 $^{-9}$, more preferably less than 1 x 10 $^{-12}$, more preferably less than 1 x 10 $^{-15}$, more preferably less than 1 x 10 $^{-18}$ and most preferably less than 1 x 10 $^{-21}$ when compared with any one of the specifically identified sequences.

The parameter -F F turns off filtering of low complexity sections. The parameter -p selects the appropriate algorithm for the pair of sequences. This program fmds regions of similarity between the sequences and for each such region reports an "E value" which is the expected number of times one could expect to see such a match by chance in a database of a fixed reference size containing random sequences. For small E values, much less than one, this is approximately the probability of such a random match.

Polypeptide sequence identity may also be calculated over the entire length of the overlap between a candidate and subject polypeptide sequences using global sequence alignment programs. EMBOSS-needle (available at http: /www.ebi.ac.uk/emboss/align/) and GAP (Huang, X. (1994) On Global Sequence Alignment. Computer Applications in the Biosciences 10, 227-235.) as discussed above are also suitable global sequence alignment programs for calculating polypeptide sequence identity.

Use of BLASTP as described above is preferred for use in the determination of polypeptide variants.

Conservative substitutions of one or several amino acids of a described polypeptide sequence without significantly altering its biological activity are also described . Conservative substitutions typically include the substitution of one amino acid for another with similar characteristics, e.g., substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Other conservative substitutions can be taken from Table 1 below.

TABLE 1

| Original Residue | Exemplary Substitutions |
|---|---|
| Ala (A) | val; leu; ile; gly |
| Arg (R) | lys; gln; asn |
| Asn (N) | gln; his; lys; arg |
| Asp (D) | glu |
| Cys (C) | ser |
| Gln (Q) | asn; his |
| Glu (E) | asp |
| Gly (G) | pro; ala |
| His (H) | asn; gln; lys; arg |

(continued)

| Original Residue | Exemplary Substitutions |
|---|---|
| Ile (I) | leu; val; met; ala; phe; norleucine |
| Leu (L) | norleucine; ile; val; met; ala; phe |
| Lys (K) | arg; gln; asn |
| Met (M) | leu; phe; ile |
| Phe (F) | leu; val; ile; ala; tyr |
| Pro (P) | ala; gly |
| Ser (S) | thr |
| Thr (T) | ser |
| Trp (W) | tyr; phe |
| Tyr (Y) | trp; phe; thr; ser |
| Val (V) | ile; leu; met; phe; ala; norleucine |

Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg:
(5) residues that influence chain orientation: gly, pro; and
(6) aromatic: trp, tyr, phe.

Non-conservative substitutions will entail exchanging a member of one of these classes for a member of another class.

Other variants include peptides with modifications which influence peptide stability. Such analogs may contain, for example, one or more non-peptide bonds (which replace the peptide bonds) in the peptide sequence. Also described are analogs that include residues other than naturally occurring L-amino acids, e.g. D-amino acids or non-naturally occurring synthetic amino acids, e.g. beta or gamma amino acids and cyclic analogs.

Substitutions, deletions, additions or insertions may be made by mutagenesis methods known in the art. A skilled artisan will be aware of methods for making phenotypically silent amino acid substitutions (see, e.g., Bowie *et al.,* 1990, Science 247, 1306).

Also described are those polypeptides which have been modified during or after synthesis for example by biotinylation, benzylation, glycosylation, phosphorylation, amidation, by derivatization using blocking/protecting groups and the like. Such modifications may increase stability or activity of the polypeptide.

The term "genetic construct" refers to a polynucleotide molecule, usually double-stranded DNA, which may have inserted into it another polynucleotide molecule (the insert polynucleotide molecule) such as, but not limited to, a cDNA molecule. A genetic construct may contain the necessary elements that permit transcribing the insert polynucleotide molecule, and, optionally, translating the transcript into a polypeptide. The insert polynucleotide molecule may be derived from the host cell, or may be derived from a different cell or organism and/or may be a recombinant polynucleotide. Once inside the host cell the genetic construct may become integrated in the host chromosomal DNA. The genetic construct may be linked to a vector.

The term "vector" refers to a polynucleotide molecule, usually double stranded DNA, which is used to transport the genetic construct into a host cell. The vector may be capable of replication in at least one additional host system, such as *E. coli.*

The term "expression construct" refers to a genetic construct that includes the necessary elements that permit transcribing the insert polynucleotide molecule, and, optionally, translating the transcript into a polypeptide. An expression construct typically comprises in a 5' to 3' direction:

a) a promoter functional in the host cell into which the construct will be transformed,
b) the polynucleotide to be expressed, and
c) a terminator functional in the host cell into which the construct will be transformed.

The term "coding region" or "open reading frame" (ORF) refers to the sense strand of a genomic DNA sequence or a cDNA sequence that is capable of producing a transcription product and/or a polypeptide under the control of appropriate regulatory sequences. The coding sequence is identified by the presence of a 5' translation start codon and a 3' translation stop codon. When inserted into a genetic construct, a "coding sequence" is capable of being expressed when it is operably linked to promoter and terminator sequences and/or other regulatory elements.

"Operably-linked" means that the sequence to be expressed is placed under the control of regulatory elements that include promoters, transcription control sequences, translation control sequences, origins of replication, tissue-specific regulatory elements, temporal regulatory elements, enhancers, polyadenylation signals, repressors and terminators.

The term "noncoding region" refers to untranslated sequences that are upstream of the translational start site and downstream of the translational stop site. These sequences are also referred to respectively as the 5' UTR and the 3' UTR. These regions include elements required for transcription initiation and termination and for regulation of translation efficiency.

Terminators are sequences, which terminate transcription, and are found in the 3' untranslated ends of genes downstream of the translated sequence. Terminators are important determinants of mRNA stability and in some cases have been found to have spatial regulatory functions.

The term "promoter" refers to nontranscribed cis-regulatory elements upstream of the coding region that regulate gene transcription. Promoters comprise cis-initiator elements which specify the transcription initiation site and conserved boxes such as the TATA box, and motifs that are bound by transcription factors.

The terms "to alter expression of" and "altered expression" of a polynucleotide or polypeptide are intended to encompass the situation where genomic DNA corresponding to a polynucleotide is modified thus leading to altered expression of a polynucleotide or polypeptide. Modification of the genomic DNA may be through genetic transformation or other methods known in the art for inducing mutations. The "altered expression" can be related to an increase or decrease in the amount of messenger RNA and/or polypeptide produced and may also result in altered activity of a polypeptide due to alterations in the sequence of a polynucleotide and polypeptide produced.

Detailed Description of the Invention

The applicants have identified a novel marker for prostate cancer or PIN using a new bioinformatics approach to mine sequenced prostate cDNA libraries. Data-panning is a technique which determines the degree of specificity transcripts have to a given tissue. This method utilises the UniGene database (www.ncbi.nlm.nih.gov/UniGene). ESTs within a UniGene cluster are assessed for library of origin and a tally of those from the specified tissue is kept. This tally is expressed as a percentage of the total number of EST's in the UniGene cluster. The higher the percentage the fewer instances of that transcript being detected in tissues other than those specified. This approach has advantages over other technologies such as cDNA microarrays (Carlisle *et al.,* 2000), due to unbiased gene selection, and greater discriminatory power in identifying differences between disease states. Previous attempts to profile gene expression in prostate cancer have employed methods that are limited in the number of expressed sequence tags analysed (Huang *et al.,* 1999) or biased in gene selection (Carlisle *et al.,* 2000).

From this analysis the applicants have identified a new marker whose expression is believed to alter with the progression of prostate cancer or PIN. This marker may also be a promising new target for the development of drugs to treat prostate cancer or PIN.

The marker is listed in Table 1 below, and the full sequence given in the sequence listing. The expression level of the markers is altered in prostate cancer patients. For convenience the marker is referred to herein as prostate specific unigene (PSPU) marker. The marker may be a DNA or RNA sequence, gene or chromosomal fragment. Any corresponding polypeptides encoded by genes are referred to as PSPU polypeptides or PSPU proteins.

**Marker Implicated in Prostate Cancer or Pin**

| Name | Unigene # | SEQ ID NO: | % Enrichment | Tissues |
|---|---|---|---|---|
| PSPU 43 | 161160 | 3 | 83 | Prostate, Other |

The nucleic acid molecules suitable for use in the methods of the invention or otherwise described here can be isolated from tissue using a variety of techniques known to those of ordinary skill in the art. By way of example, such polynucleotides can be isolated through use of the polymerase chain reaction (PCR) described in Mullis et al., Eds. 1994 The Polymerase Chain Reaction, Birkhauser. The nucleic acid molecules can be amplified using primers, as defined herein, derived from the polynucleotide sequences described herein.
Further methods for isolating polynucleotides include use of all, or portions of, the polynucleotide disclosed herein, particularly a polynucleotide having the sequence set forth in SEQ ID NO:3 as hybridization probes. The technique of hybridizing labeled polynucleotide probes to polynucleotides immobilized on solid supports such as nitrocellulose filters or nylon membranes, can be used to screen genomic or cDNA libraries. Similarly, probes may be coupled to beads and hybridized to the target sequence. Isolation can be effected using known art protocols such as magnetic separation. Exemplary stringent hybridization and wash conditions are as given above.

Polynucleotide fragments may be produced by techniques well-known in the art such as restriction endonuclease digestion and oligonucleotide synthesis.

Accordingly in a first aspect, the invention provides an *in vitro* method of (a) diagnosing prostatic intraepithelial neoplasia (PIN) or prostate cancer (PRC) or a predisposition to PIN or PRC in a patient, (b) monitoring a course of treatment for PIN or PRC in a patient, or (c) for prognosing PIN or PRC in a patient, comprising
obtaining a PIN or PRC expression profile from a sample from the patient, the expression profile comprising a pattern of marker expression including a nucleic acid molecule, and
comparing the expression profile to an expression profile of a control sample,
wherein the nucleic acid molecule comprises:

(i) the sequence of SEQ ID NO:3,
(ii) a sequence which hybridises under stringent conditions to SEQ ID NO:3,
(iii) a sequence which has 70%, 75%, 80%, 90%, 95%, or 99% sequence identity to SEQ ID NO:3, or
(iv) an at least 20 nucleotide fragment of (i) or a complement thereof, which fragment or complement hybridizes under stringent conditions to:

(a) a nucleic acid molecule of (i), (ii) or (iii) or a complement thereof;
(b) a reverse complement of (a).

Stringent conditions are discussed above.

The nucleic acid molecule may be at least 30 nucleotides, at least 40 nucleotides, at least 50 nucleotides, at least 60 nucleotides, at least 70 nucleotides, at least 80 nucleotides, at least 90 nucleotides, or preferably is at least 100 nucleotides.

A partial polynucleotide sequence may be used as a probe, in methods well-known in the art to identify the corresponding full length polynucleotide sequence in a sample. Such methods include PCR-based methods, 5'RACE (Methods Enzymol. 218: 340-56 (1993); Sambrook *et al.,* Supra) and hybridization-based method, computer/database-based methods. Detectable labels such as radioisotopes, fluorescent, chemiluminescent and bioluminescent labels may be used to facilitate detection. Inverse PCR also permits acquisition of unknown sequences, flanking the polynucleotide sequences disclosed herein, starting with primers based on a known region (Triglia et al., Nucleic Acids Res 16, 8186, (1998)) The method uses several restriction enzymes to generate a suitable fragment in the known region of a gene. The fragment is then circularized by intramolecular ligation and used as a PCR template. Divergent primers are designed from the known region. In order to physically assemble full-length clones, standard molecular biology approaches can be utilized (Sambrook *et al.,* Supra). Primers and primer pairs which allow amplification of polynucleotides as described herein are also disclosed.

Variants (including orthologues) may be identified by the methods described. Variant polynucleotides may be identified using PCR-based methods (Mullis et al., Eds. 1994 The Polymerase Chain Reaction, Birkhauser). Typically, the polynucleotide sequence of a primer, useful to amplify variants of polynucleotide molecules by PCR, may be based on a sequence encoding a conserved region of the corresponding amino acid sequence.

Further methods for identifying variant polynucleotides include use of all, or portions of, the specified polynucleotides as hybridization probes to screen genomic or cDNA libraries as described above. Typically probes based on a sequence encoding a conserved region of the corresponding amino acid sequence may be used. Hybridisation conditions may also be less stringent than those used when screening for sequences identical to the probe.

The variant sequences, including both polynucleotide and polypeptide variants, may also be identified by the computer-based methods discussed above.

Multiple sequence alignments of a group of related sequences can be carried out with CLUSTALW (Thompson, et al., Nucleic Acids Research, 22:4673-4680 (1994), http://www-igbmc.u-strasbg.fr/BioInfo/ClustalW/Top.html) or T-COFFEE (Cedric Notredame et al., J. Mol. Biol. 302: 205-217 (2000))) or PILEUP, which uses progressive, pairwise alignments. (Feng et al., J. Mol. Evol. 25, 351 (1987)).

Pattern recognition software applications are available for finding motifs or signature sequences. For example, MEME (Multiple Em for Motif Elicitation) finds motifs and signature sequences in a set of sequences, and MAST (Motif Alignment and Search Tool) uses these motifs to identify similar or the same motifs in query sequences. The MAST results are provided as a series of alignments with appropriate statistical data and a visual overview of the motifs found. MEME and MAST were developed at the University of California, San Diego.

PROSITE (Bairoch et al., Nucleic Acids Res. 22, 3583 (1994); Hofmann et al., Nucleic Acids Res. 27, 215 (1999)) is a method of identifying the functions of uncharacterized proteins translated from genomic or cDNA sequences. The PROSITE database (www.expasy.org/prosite) contains biologically significant patterns and profiles and is designed so that it can be used with appropriate computational tools to assign a new sequence to a known family of proteins or to determine which known domain(s) are present in the sequence (Falquet et al., Nucleic Acids Res. 30, 235 (2002)). Prosearch is a tool that can search SWISS-PROT and EMBL databases with a given sequence pattern or signature.

Proteins can be classified according to their sequence relatedness to other proteins in the same genome (paralogues) or a different genome (orthologues). Orthologous genes are genes that evolved by speciation from a common ancestral gene and normally retain the same function as they evolve. Paralogous genes are genes that are duplicated within a genome and genes may acquire new specificities or modified functions which may be related to the original one. Phylogenetic analysis methods are reviewed in Tatusov et al., Science 278, 631-637, 1997).

In addition to the computer/database methods described above, polypeptide variants may be identified by physical methods, for example by screening expression libraries using antibodies raised against polypeptides of the invention (Sambrook et al., Molecular Cloning: A Laboratory Manual, 2nd Ed. Cold Spring Harbor Press, 1987) by recombinant DNA techniques also described by Sambrook *et al.* or by identifying polypeptides from natural sources with the aid of such antibodies.

Polypeptides, including variant polypeptides, may be prepared using peptide synthesis methods well known in the art such as direct peptide synthesis using solid phase techniques (e.g, Merrifield, 1963, in J. Am Chem. Soc. 85, 2149; Stewart et al., 1969, in Solid-Phase Peptide Synthesis, WH Freeman Co, San Francisco California; Matteucci et al. J. Am. Chem. Soc. 103:3185-3191, 1981) or automated synthesis, for example using a Synthesiser from Applied Biosystems (California, USA). Mutated forms of the polypeptides may also be produced using synthetic methods such as site-specific mutagensis of the DNA encoding the amino acid sequence as described by Adelmen et al; DNA 2, 183 (1983).

The polypeptides and variant polypeptides may also be isolated or purified from natural sources using a variety of techniques that are well known in the art (e.g. Deutscher, 1990, Ed, Methods in Enzymology, Vol. 182, Guide to Protein Purification,). Technologies include HPLC, ion-exchange chromatography, and immunochromatography but are not limited thereto.

Alternatively the polypeptides and variant polypeptides may be expressed recombinantly in suitable host cells and separated from the cells as discussed below. The polypeptides and variants have utility in generating antibodies, and generating ligands amongst other uses.

Specific polypeptides include polypeptides having the amino acid sequences of SEQ ID NO:9, SEQ ID NO: 10, SEQ ID NO:11, SEQ ID NO:12, SEQ ID NO:13 and SEQ ID NO:14 all as set forth in the accompanying sequence listing.

The genetic constructs may comprise one or more of the disclosed polynucleotide sequences and/or polynucleotides and may be useful for transforming, for example, bacterial, fungal, insect, mammalian or plant organisms. Genetic constructs may include expression constructs as herein defined. Included are vectors (such as pBR322, pUC18, pU19, Mp18, Mp19, ColEl, PCR1 and pKRC), phages (such as lambda gt10), and M13 plasmids (such as pBR322, pACYC184, pT127, RP4, p1J101, SV40 and BPV), cosmids, YACS, BACs shuttle vectors such as pSA3, PAT28 transposons (such as described in US 5,792,294) and the like.

The constructs may conveniently include a selection gene or selectable marker. Typically an antibiotic resistance marker such as ampicillin, methotrexate, or tetracycline is used.

Promoters useful in the constructs include β. lactamase, alkaline phosphatase, tryptophan, and tac promoter systems which are all well known in the art. Yeast promoters include 3-phosphoglycerate kinase, enolase, hexokinase, pyruvate decarboxylase, glucokinase, and glyceraldehydrate-3-phosphanate dehydrogenase but are not limited thereto.

Enhancers may also be employed to act on the promoters to enhance transcription. Suitable enhancers for use herein include SV40 enhancer, cytomeglovirus early promoter enhancer, globin, albumin, insulin and the like.

Methods for producing and using genetic constructs and vectors are well known in the art and are described generally in Sambrook *et al.,* (supra), and Ausubel et al., Current Protocols in Molecular Biology, Greene Publishing, 1987. Methods for transforming selected host cells with the vectors are also known, for example, the calcium chloride treatment described by Cohen, SN; PNAS 69, 2110, 1972.

Host cells comprising the genetic constructs and vectors described may be derived from prokaryotic or eukaryotic sources, for example yeast, bacteria, fungi, insect (eg baculovirus), animal, mammalian or plant organisms. Prokaryotes most commonly employed as host cells are strains of *E. coli.* Other prokaryotic hosts include *Pseudomonas, Bacillus, Serratia, Klebsiella, Streptomyces, Listeria, Saccharomyces, Salmonella and Mycobacteria* but are not limited thereto.

Eukaryotic cells for expression of recombinant protein include but are not limited to Vero cells, HeLa, CHO (Chinese Hamster ovary cells), 293, BHK cells, MDCK cells, and COS cells as well as prostate cancer cell lines such as PrEC, LNCaP, Du 145 and RWPE-2. The cells are available from ATCC, Virginia, USA.

Prokaryotic promoters compatible with expression of nucleic acid molecules of the invention include known art constitutive promoters (such as the int promoter of bacteriophage lamda and the bla promoter of the beta-lactamase gene sequence of pBR322) and regulatable promoters (such as lacZ, recA and gal). A ribosome binding site upstream of the PSPU 43 coding sequence is also required for expression.

Host cells comprising genetic constructs, such as expression constructs, are useful in methods for recombinant production of polypeptides. Such methods are well known in the art (see for example Sambrook *et al.* supra). The methods commonly involve the culture of host cells in an appropriate medium in conditions suitable for or conducive to, expression and selection of a polypeptide. Cells with a selectable marker may additionally be grown on medium appropriate for selection of host cells expressing a polypeptide of the invention. Transformed host cells expressing a polypeptide are selected and cultured under conditions suitable for expression of the polypeptide. The expressed recombinant polypeptide, may be separated and purified from the culture medium using methods well known in the art including ammonium sulfate precipitation, ion exchange chromatography, gel filtration, affinity chromatography, electrophoresis and the like (e.g. Deutscher, Ed, 1990, Methods in Enzymology, Vol 182, Guide to Protein Purification). Host cells may also be useful in methods for production of a product generated by an expressed polypeptide

Host cells or animals that are predisposed to
prostate cancer are useful for testing compounds which may be used to treat prostate cancer, or to identify compounds that may be implicated in causing the cancer. Animal models are particularly useful for testing purposes. Non-human patients may be suitable animals to use. Preferably the animal is a rodent or rabbit. Rats, and particularly mice are preferred for use.

Animal models may incorporate a gene coding for a polypeptide disclosed herein or an antisense or siRNA sequence thereto that does not occur naturally in the animal, (exogenous), or does not occur at the location in which the gene is introduced, or does not occur in the same configuration as the introduced gene. Also encompassed by the animal models are animals in which endogenous genes corresponding to a nucleic acid molecule disclosed herein are altered, disrupted or eliminated.

Alterations in the germ line of the animals may be achieved using any known art methods. For example genes may be incorporated into the genome of an animal through microinjection of zygotes (Brinster et al., PNAS (USA) 82: 4438-4442 (1985); through viral integration using retrovirus infection of blastomeres or blastocoels (Jaenuch, R; PNAS (USA) 73:1260-1264 (1976), Johner, D et al., Nature 298:623-628 (1982); or by transformation of embryonic stem cells (Lovel-Badge, R. H., Tetracarcinomas and Embryonic Stem Cells: A Practical Approach, Robertson, E. J. et al., DRL Press, Oxford, 153-182 (1987). See also Houdebine, Transgenic Animals - Generation and Use (Harwood Academic, 1997).

In another aspect, the present invention provides : *in vitro* methods of diagnosing and/or prognosing prostate cancer, PIN or a predisposition to developing same in a patient.

In one embodiment the method is carried out by determining the expression level of a nucleic acid molecule of the invention, such as PSPU 43 (SEQ ID NO:3), in a patient sample. An alteration in the expression level of the molecule compared to a control level of the molecule indicates that the subject has PIN, PRC, or is at risk of developing same. Alterations in expression levels of the molecules include identifying the presence or absence of the molecule from the patient sample.

Described herein is a method of testing for prostatic
intraepithelial neoplasia (PIN), prostate cancer (PRC) status in a patient, the method comprising determining the expression level of PSPU 43 (SEQ ID NO:3) or other nucleic acid molecule of the invention in a patient sample, wherein an increase in expression level compared to a control level of said molecule indicates that the patient has PIN, PRC status or is at risk of developing PIN or PRC.

The expression level of a molecule of the invention can be considered to be altered, including increased, if the expression level differs from the control level by a statistically significant amount. Usually by more than 5%, more than 10%, more than 20% more than 30%, more than 40%, preferably by more than 50% or more compared to the control level. Statistically significant may alternatively be calculated as P< 0.05. In a further alternative, deviation can be determined by recourse to assay reference limits or reference intervals. These can be calculated from intuitive assessment or non-parametric methods. Overall these methods calculate the 0.025, and 0.975 fractiles as 0.025 * (n+1) and 0.975 (n+1). Such methods are well known in the art. See for example the Immunoassay Handbook, 3rd edition, ed. David Wild. Elsevier Ltd, 2005; and Solber H. Approved Recommendation (1987) Collected reference values. Determination of reference limits. Journal of Clinical Chemishy and Clinical Biochemistry 1987, 25:645-656.

Presence of a marker absent in a control, or absence of a marker present in a control are also contemplated as changes in expression levels.

The presence of the markers and their level of expression in the sample may be determined according to methods known in the art such as Southern Blotting, Northern Blotting, FISH or quantative PCR to quantitate the transcription of mRNA [(Thomas, Pro. NAH, Acad. Sci. USA 77; 5201-5205 1980), (Jain KK, Med Device Technol. 2004 May; 15(4):14-7)], dot blotting, (DNA analysis) or *in situ* hybridization using an appropriately labelled probe, based on the marker sequences provided herein.

Accordingly, the invention also provides an *in vitro* assay for detecting the presence of a nucleic acid molecule of the invention, preferably PSPU 43 (SEQ ID NO:3) in a sample, the method comprising:

(a) contacting the sample with a polynucleotide probe which hydridises to the nucleic acid sequence under stringent hybridisation conditions, and
(b) detecting the presence of a hybridisation complex in the sample.

Preferably the hybridisation probe is a labelled probe. Examples of labels include fluorescent, chemiluninescent, radioenzyme and biotin-avidin labels. Labelling and visualisation of labelled probes is carried out according to known art methods such as those above.

For convenience the nucleic acid probe may be immobilized on a solid support including resins (such as polyacrylamides), carbohydrates (such as sepharose), plastics (such as polycarbonate), and latex beads.

As discussed above the nucleic acid molecule probe may be an RNA or DNA molecule. Preferred probes include

| | | |
|---|---|---|
| *Pspu43* | Forward<br>Reverse | 5'-AACAAATATAAAGTACCAGACACTCCA -3' (SEQ ID NO:15)<br>5'-ATCTCCAGATCTFCCTTCTAGCC -3' (SEQ ID NO:16) |

The expression level of the nucleic acid marker may be determined using known art techniques such as RT-PCR and electrophoresis techniques including SDS-PAGE. Using these techniques the DNA or cDNA sequence of a nucleic acid molecule of the invention, and PSPU 43 (SEQ ID NO:3) in a patient sample is amplified, and the level of DNA or cDNA or RNA measured.

In an alternate method the DNA, cDNA or RNA level may be measured directly in the sample without amplification.

A currently preferred method is Northern blot hybridization analysis. Probes for use in Northern blot hybridization analysis may be prepared based on the marker sequences identified herein. A probe preferably includes at least 20, at least 30, at least 40, at least 50, preferably 75, preferably 100, or more preferably 200 or more contiguous nucleotides of a reference sequence.

Alternatively, the expression level may be measured using reverse transcription based PCR (RT-PCR) assays using primers specific for the nucleic acid sequences. If desired, comparison of the expression level of the marker in the sample can be made with reference to a control nucleic acid molecule the expression of which is independent of the parameter or condition being measured. A control nucleic acid molecule refers to a molecule in which expression does not differ between the PIN/PRC state and the healthy state. Expression levels of the control molecule can be used to normalise expression levels in the compared populations. An example of such a control molecule is transgelin 2. The markers will change expression levels with disease.

Alternatively, for peptide markers, antibodies may be employed that can recognize specific duplexes, including DNA duplexes, RNA duplexes, and DNA-RNA hybrid duplexes or DNA-protein duplexes. The antibodies in turn may be labelled and the assay may be carried out where the duplex is bound to a surface, so that when the duplex is formed on the surface the presence of the antibody bound to the duplex can be detected. Immunoassays commonly available in the art for this purpose include radioinnmunoassay, (RIA), enzyme immunosorbant assays (ELISA) and the like (Lutz et al., Exp. Cell. Res. 175: 109-124 (1988).

Marker expression may alternatively be measured by immunological methods such as immunohistochemical staining of cells or tissue sections and assay of cell culture or body fluids to quantitate directly the expression level. Antibodies useful for immunohistochemical staining and/or for assay of sample fluids are preferably either monoclonal or polyclonal and are discussed in greater detail below. Conveniently the antibodies may be prepared against a polypeptide or against a synthetic peptide based on the DNA sequences disclosed herein, or against exogenous sequence fused to DNA of a nucleic acid molecule of the invention, (particularly PSPU 43) and encoding a specific antibody epitope.

Prostate health monitoring from blood cells using the biomarkers of the invention may be carried out using these techniques, for example as set out in "Cytogenetic evidence that circulating epithelial cells in patients with carcinoma are malignant" by Felun et al Clinical Cancer Research, 8:2073-2084 (2002).

Urine sampling is also feasible. The urethra passes through the prostate leading to prostate cells being passed into urine. A prostate test for the nucleic acid PCA3 marker in urine has been developed by Bostwick Laboratories (http://bostwicklaboratories.com/patientservices/uPM3.html). Similar tests may be employed for PSPU 43.

Alteration in the expression of one or more of the PSPU markers in a patient sample compared to the normal control level indicates that the patient suffers from or is at risk of developing PIN or PRC. Whether the alteration is an increase or decrease may depend on the stage of the disease. Generally, PSPU 43 has been shown to be over-expressed in prostate cancer patients. However, under-expression for example in advanced stages of prostate cancer is also feasible.

Other markers can also be used in association with PSPU markers of the invention. Useful markers include known markers of prostate cancer such as PCA3, PSA. Transgelin 1 which has been shown to be under-expressed in prostate cancer patients may also be used. It may also be useful to include a benchmark or reference marker which does not change with disease state. Transgelin 2 may be useful for this purpose. Correlating the level of the PSPU marker with other markers can increase the predictive diagnostic of monitoring value of the PSPU marker of the invention. Use of PSPU 43 with known prostate cancer markers can increase the predictive or diagnostic value of patient outcome.

Analysis of a number of peptide markers can be carried out simultaneously or separately using a single test sample. Simultaneous or two site format assays are preferred. Microassay or biochip analysis are particularly useful. The assays or chips can have a number of discreet addressable locations comprising an antibody to one or more markers including a PSPU marker of the invention. US2005/0064511 provides a description of chips and teclmiques.

In another embodiment, the present invention therefore provides an *in vitro* method of monitoring response to treatment of PIN or PRC in a subject, the method comprising determining the expression level of a nucleic acid molecule of the invention, preferably PSPU 43 (SEQ ID NO:3) in a patient sample, and comparing the level of said molecule to a control level, wherein a statistically significant change in the determined level from the control level is indicative of a response to the treatment.

A statistically significant increase in the PSPU molecule, particularly PSPU 43 is indicative of PIN or PRC or the results can be correlated with changes to non-PSPU 43 markers such as including those discussed above. Changes in these marker levels from a control, coupled with an increase in the PSPU molecule compared to a control may be more indicative of PRC or PIN.

Where a subject is to be monitored, a number of biological samples may be taken over time. Serial sampling allows changes in marker levels, particular PSPU 43 to be measured over time. Sampling can provide information about onset of cancer, the severity of the cancer, which therapeutic regimes may be appropriate, response to therapeutic regimes employed, and long term prognosis. Analysis may be carried out at points of care such as in doctors offices, on clinical presentation, during hospital stays, in outpatients, or during routine health screening.

*The in vitro* methods of the invention may also be performed in conjunction with analysis of one or more risk factors such as, but not limited to age, family history and ethnic background.

The methods herein can be used as a guide to therapy. For example, what therapies to initiate and when.

A kit comprising one or more detection reagents which specifically bind to a PSPU nucleic acid marker molecule as disclosed herein is described. The kit may include PSPU43 (SEQ ID NO:3). The detection reagents may be oligonucleotide sequences complementary to a portion of the PSPU marker, could be designed to nucleic acid or peptide sequences known to flank the PSPU marker. The reagents may be bound to a solid matrix as discussed above or packaged with reagents for binding them to the matrix. The solid matrix or substrate may be in the form of beads, plates, tubes, dip sticks, strips or biochips. Biochips or plates with addressable locating and discreet microtitre plates are particularly useful.

Detection reagents include wash reagents and reagents capable of detecting bound antibodies (such as labelled secondary antibodies), or reagents capable of reacting with the labelled antibody.

The kit may also conveniently include a control reagent (positive and/or negative) and/or a means for detecting the nucleic acid or antibody. Instructions for use may also be included with the kit. Most usually, the kits will be formatted for assays known in the art, and more usually for PCR, Northern hybridization or Southern ELISA assays, as are known in the art.

Kits may also be formatted from using the nucleic acid molecules of the invention for use in screening procedures such as FISH that detect chromosomal rearrangements associated with disease and disease progression. The kit may additionally include detection reagents for the nucleic acid, and controls.

The kits may also include one or more additional markers for prostate cancer or controls including transgelin 1, transgelin 2, PCA 3 and PSA. All of the markers may be included in the kit.

The kit may be comprised of one or more containers and may also include collection equipment, for example, bottles, bags (such as intravenous fluids bags), vials, syringes, and test tubes. At least one container holds a composition which is effective for diagnosing, monitoring, or heating PIN or PRC. The active agent in the composition is usually a compound, polypeptide or an antibody of the invention. In a preferred embodiment, an instruction or label on, or associated with, the container indicates that the composition is used for diagnosing, monitoring or treating PIN or PRC. Other components may include needles, diluents and buffers. Usefully, the kit may include at least one container comprising a pharmaceutically-acceptable buffer, such as phosphate-buffered saline, Ringer's solution and dextrose solution.

Antibodies used in the assays and kits may be monoclonal or polyclonal and may be prepared in any mammal. They may be raised against an exogenous sequence fused to a nucleic acid sequence as disclosed herein.

Antibody binding studies may be carried out using any known assay method, such as competitive binding assays, non-competitive assays, direct and indirect sandwich assays, fluoroimmunoassays, immunoradiometric assays, luminescence assays, chemiluminesence assays, enzyme linked immunofluorescent assays (ELIFA) and immunoprecipitation assays. Zola, Monoclonal Antibodies: A Manual of Techniques, pp.147-158 (CRC Press, Inc., 1987); Harlow and Lome (1998) Antibodies, A Laboratory Manual, Cold Spring Harbour Publications, New York; US 5,221,685; US 5,310,687; US 5,480,792; US 5,525,524; US 5,679,526; US 5,824,799; US 5,851,776; US 5,885,527; US 5,922,615; US 5,939,2,7;2; US 5,647,124; US 5,985,579; US 6,019,944; US 6,113,855; US 6,143,576; US 5,955,371; US 5,631,171 and US 2005/0064511.

For example, one type of sandwich assay is an ELISA assay, in which case the detectable moiety is an enzyme. ELISA is particularly useful for predicting, detecting or monitoring PIN or PRC.

Alternate analytical techniques useful herein include mass spectrometry analysis such as surface-enhanced laser desorption and ionization (SELDI), electrospray ionization (ESI) and matrix assisted laser-desorption ionization (MALDI).

For immunohistochemistry, the tissue sample may be fresh or frozen or may be embedded in paraffin and fixed with a preservative such as formalin, for example.

In one kit a PSPU detection reagent is immobilized on a solid matrix such as a porous strip to form at least one PSPU detection site. The measurement or detection region of the porous strip may include a plurality of detection sites, such detection sites containing a PSPU detection reagent. The sites may be arranged in a bar, cross or dot or other arrangement. A test strip may also contain sites for negative and/or positive controls. The control sites may alternatively be on a different strip. The different detection sites may contain different amounts of immobilized nucleic acids eg, a higher amount in the first detection site and lower amounts in subsequent sites. Upon the addition of a test biological sample the number of sites displaying a detectable signal provides a quantitative indication of the amount of PSPU present in the sample.

An assay comprising one or more nucleic acid
sequences which bind to one or more of the PSPU nucleic acid sequences of PSPU 43 is described large range of sense and antisense probes and primers can be designed from the nucleic acid sequences for the PSPUs herein. The expression level of the PSPU sequence is identified using known art techniques discussed above. The array can be a solid substrate e.g., a "chip" as described in US Patent No. 5,744,305 or a nitrocellulose membrane.

A method for screening for a compound that
alters the expression of a nucleic acid molecule as disclosed herein, particularly PSPU 43 (SEQ ID NO:3) is described. In broad terms, a test cell that expresses the molecule is contacted with a test compound and a compound selected that alters the expression level of the marker compared to that in the absence of the compound. Such compounds include molecules that agonize or antagonize the nucleic acid molecule expression.

Test compounds can be obtained from a wide range of known
compounds, unknown compounds obtained from natural sources such as plant, extracts and microorganisms, or using any of the numerous approaches in combinatorial library methods known in the art. See for example Lam Anticancer Drug Des. 12: 1 145 (1997) and De Witt et al. PNAS 90:6909 (1993).

The assays can be performed in a variety of formats.

Peptides, non-peptide compounds, synthetic micromolecular compounds and natural compounds can be used in the screening methods of the present invention

Computer modelling of agonists and antagonists to nucleic acid molecules of the invention is also possible using well known programmes such as AUTODOCK (Dunbrack et al., 1997, Folding and Design 2:R27-42) CHARMm and QUANTA programs (Polygen Corporation, Massachusetts, USA),

A PIN or PRC reference expression profile is described. This comprises a pattern of marker expression including a nucleic acid molecule of the invention, preferably PSPU 43. Usefully, the expression profile includes one or more additional markers selected from PCA3, transgetin 1, transgelin 2, and PSA. In one embodiment the markers are PCA3 and PSA. In another embodiment all the additional markers are included. Using the expression techniques discussed above the profile can be generated and used as a point of comparison for new patient samples in the diagnosis of PIN or PRC or a predisposition to same. The profiles can also be used to monitor a course of treatment for PIN or PRC, and as a prognosis tool for a patient identified as having PIN or PRC.

A method of treating or preventing
PIN or PRC in a patient wherein a PSPU molecule of the invention is over-expressed is described. The method comprises altering the expression of the PSPU marker . Inhibition may be effected by administration of one or more compounds obtained by the screening methods above. Alternatively, expression may be inhibited by known art methods such as administration of nucleic acid that inhibits or antagonises the expression of the marker. Antisense oligonucleotides, siRNA, intracellular antibodies and, ribozymes which disrupt expression of the marker can all be used for inhibiting expression.

Antisense-oligonucleotides corresponding to a PSPU molecule herein, preferably PSPU 43 can be used to reduce the expression level of the PSPU molecule in situations where that is required. The antisense-oligonucleotides of the present invention may act by binding to PSPU nucleic acid molecules of the invention, or DNAs or mRNAs corresponding thereto, thereby inhibiting the transcription of the markers, promoting the degradation of the mRNAs.

The nucleic acids that inhibit one or more gene products of overexpressed genes also include small interfering RNAs (siRNA) comprising a combination of a sense strand nucleic acid and an antisense strand nucleic acid of the nucleotide sequence encoding the PSPU marker. The term "siRNA" refers to a double stranded RNA molecule which prevents translation of a target mRNA. Standard techniques of introducing siRNA into the cell can be used in the treatment or prevention of PIN or PRC, including those in which DNA is a template from which RNA is transcribed, The siRNA may be constructed such that a single transcript has both the sense and complementary antisense sequences from the target gene, e.g., a hairpin.

The method is used to suppress gene expression of a cell with up-regulated expression of a PSPU molecule of the invention.

The length of the oligonucleotide is at least 10 nucleotides and may be as long as the naturally occurring transcript. Preferably, the oligonucleotide is less than 100, less than 75, less than 50 or less than 25 nucleotides in length. Preferably, the oligonucleotide is 19-25 nucleotides in length.

The nucleotide sequence of siRNAs may be designed using a siRNA design computer program available from the Ambion website (http://www.ambion.com/tehlib/misc/siRNA finder.html) and as described in Yuan et al., Nucleic Acids Research 2004 vol 32, W130-W134. Nucleotide sequences for the siRNA are selected by the computer program based on the following protocol:

Selection of siRNA Target Sites:

1. Beginning with the AUG start codon of transcript, scan downstream for AA dinucleotide sequences. Record the occurrence of each AA and the 3' adjacent 19 nucleotides as potential siRNA target sites. Harborth *et al.* (2003) recommend against designing siRNA against the 5' and 3' untranslated regions (UTRs) and regions near the start codon (within 75 bases) as these may be richer in regulatory protein binding sites. Complexes of endonuclease and siRNAs designed against these regions may interfere with the binding of UTR-binding proteins and/or translation initiation complexes.
2. Compare the potential target sites to the human genome database and eliminate from consideration any target sequences with significant homology to other coding sequences. The homology search can be performed using BLAST, as described above, and which can be found on the NCBI server at: www.ncbi.nlm.nih.gov/BLAST/
3. Select qualifying target sequences for synthesis. On the Ambion website, several preferred target sequences along the length of the gene can be selected for evaluation.

The siRNAs may inhibit the expression of the PSPU molecule and therefore be useful for suppressing the biological activity. Therefore, a composition comprising the siRNA may be useful in treating or preventing PIN or PRC in which over-expression of a PSPU molecule is implicated.

The nucleic acids that inhibit one or more gene products of overexpressed genes also include ribozymes against the over-expressed markers. Ribozymes are generally RNA molecules which possess the ability to cleave other single stranded RNA in a manner analogous to DNA restriction endonucleases.

Methods for designing and constructing ribozymes are known in the art (see for example Koizumi et al. FEBS Lett. 228: 225 (1998); Kikuchi et al., NAR 19: 6751 (1992)) and ribozymes inhibiting the expression of an over-expressed PIN or PRC protein can be constructed based on the sequence information of the nucleotide sequence encoding the PIN or PRC protein according to conventional methods for producing ribozymes. Therefore, a composition comprising the ribozyme may be useful in treating or preventing PIN or PRC.

Alternatively, the function of one or more gene products of any over-expressed genes may be inhibited by administering a compound that binds to, or otherwise inhibits the function of the gene products. For example, an antibody which binds to an over-expressed marker product or products may be useful in PIN/PRC treatment as well as in diagnostic and prognostic assays.

The use of antibodies, or a fragment of the antibody, is also described.

As used herein, the term "antibody" refers to an immunoglobulin molecule having a specific structure that interacts (binds) specifically with a molecule comprising the antigen used for synthesizing the antibody or with an antigen closely related to it. Usually, the antibody will have a binding affinity (dissociation constant (Kd) value), for the PSPU antigen of no more than $10^{-7}$M, preferably less than about $10^{-8}$M, preferably less than about $10^{-9}$M. Binding affinity may be assessed using surface plasma resonance.

An antibody may be in any form, such as monoclonal or polyclonal antibodies, and includes antiserum obtained by immunizing an animal such as a mouse, rat or rabbit, all classes of polyclonal, monoclonal, human antibodies and humanized and intracellular antibodies produced by genetic recombination.

Furthermore, the antibody used in the method of treating or preventing PIN or PRC may be a fragment of an antibody or a modified antibody . The fragment will usually comprise the antigen binding region or a complementarity determining region of same, or both. The antibody fragment may be Fab, F(ab')2, and Fc or Fv or single chain Fv (scFv), in which Fv fragments from H and L chains are ligated by an appropriate linker (Huston et al. Proc. Natl. Acad. Sci. USA 85: 5879-83 (1988)).

Methods for preparing antibodies are well known in the art (see for example Antibodies: A Laboratory Manual, CSH press, eds, Harlow and Lane (1988)). Most commonly used antibodies are produced by immunizing a suitable host mammal as discussed above. An antibody may be modified by conjugation with a variety of molecules, such as polyethylene glycol (PEG). The modified antibody can be obtained by chemically modifying an antibody. These modification methods are conventional in the field. Alternatively, an antibody may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) derived from human antibody, and the constant region. Such antibodies can be prepared using known art methods.

In brief, methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL TDM adjuvant (moriophosphoryl Lipid A, synthetic trehalose dicorynomycolate). The immunization protocol may be selected by one skilled in the art without undue experimentation.

Intracellular antibodies are generally single chain antibodies herein they will comprise single chain antibodies. They may be used in gene therapy by incorporating the sequence encoding the antibody into a recombinant vector and administering to cells . Methods for producing these antibodies are known in the art. (see for example Tanaka et al., Nucleic Acids Research 31 (5):e23 (2003).

Monoclonal antibodies may be prepared using hybridoma methods which are also well known in the art. See for example Kohler and Milstein, Nature, 256:495 (1975). The hybridoma cells may be cultured in a suitable culture medium, alternatively, the hybridoma cells may be grown *in vivo* as ascites in a mammal. Preferred immortalized cell lines are murine myeloma lines, such as MPC-11 an MOPC-21 which can be obtained, for example, from the American Type Culture Collection, Virginia, USA. Immunoassays may be used to screen for immortalized cell lines which secrete the antibody of interest. Polypeptides encoded for by the PSPU markers herein or variants or fragments thereof may be used in screening.

Accordingly, also contemplated herein are hybridomas which are immortalized cell lines capable of secreting a specific monoclonal antibody.

Well known means for establishing binding specificity of monoclonal antibodies produced by the hybridoma cells include immunoprecipitation, radio-linked immunoassay (RIA), enzyme-linked immunoabsorbent assay (ELISA) and Western blot. (Lutz et al., Exp. Cell. Res. 175:109-124 (1988)). Antivirus from immunised animals may similarly be screened for the presence of polyclonal antibodies.

To facilitate detection, antibodies and fragments herein may be labelled with detectable markers that allow for direct measurement of antibody binding such as fluorescent, bioluminescent, and chemiluminescent compounds, as well as radioisotopes, magnetic beads, and affinity labels (e.g biotin and avidin). Examples of labels which permit indirect measurement of binding include enzymes where the substrate may provide for a coloured fluorescent product, suitable enzymes include horseradish peroxidase, alkaline phosphatase, malate dehydrogenase and the like. Fluorochromes (e.g Texas Red, fluorescein, phycobiliproteins, and phycoerythrin) can be used with a fluorescence activated cell sorter. Labelling techniques are well known in the art.

The monoclonal antibodies secreted by the cells may be isolated or purified from the culture medium or ascites fluid by conventional immunoglobulin purification procedures such as, for example, protein A-Sepharose, hydroxyapatite chromatography, gel electrophoresis, dialysis, or affinity chromatography.

The monoclonal antibodies or fragments may also be produced by recombinant DNA means (see for example U.S. Patent No. 4,816,567). DNA modifications such as substituting the coding sequence for human heavy and light chain constant domains in place of the homologous murine sequences (U.S. Patent No. 4,816,567; supra) are also possible. Production of chimeric bivalent antibodies are also contemplated herein.

The antibodies may be monovalent antibodies. Methods for preparing monovalent antibodies are well known in the art.

The antibodies may further comprise humanized antibodies or human antibodies. Such humanized antibodies are preferred for therapeutic use. Humanized antibodies include human immunoglobulins in which residues from a complementary determining region (CDR) of the recipient are replaced by residues from a CDR of a non-human species. The production of humanized antibodies from non-human sources such as rabbit, rat and mouse are well known. (Verhoeyen et al, Science, 239:1534-1536 (1988); Jones et al., Nature, 321:522-525 (1986); Riechmann et al., Nature 332:323-329 (1988);

Human antibodies can also be produced using various techniques known in the art, including phage display technologies (Hoogenboom and Winter, J. Mol. Biol. 227:381 (1991)); and transgenic methods, see, for example Nature Biotechnology 14, 826 (1996); and Vaughan et al, Nature Biotechnology 16:535-539 (1998).

Bispecific antibodies are monoclonal, preferably human or humanized, antibodies that have binding specificities for at least two different antigens. Contemplated herein are bispecific antibodies wherein one of the binding specificities is for the PSPU marker, the other one is for any other antigen, and preferably for a cell-surface protein or receptor or receptor subunit.

Methods for making bispecific antibodies are known in the art. See for example Milstein and Cuello, Nature, 305:537-539 (1983) and Suresh et al., Methods in Enzymology, 121:210 (1986), Brennan et al., Science 229:81 (1985).

Bispecific antibodies may bind to two different epitopes on a given 4, cl polypeptide herein. Alternatively, they may bind to molecule(s) involved in cellular defence in the cells. For example, leukocyte T-cell receptor molecules, and Fc receptors for IgG. In a further alternative, the bispecific antibodies may include an epitope which binds a cytotoxic agent such as ricin A chain, saporin, or methotrexate or a radionuclide chelator, such as EOTUBE, or DOTA.

Antibodies with greater than two specificities eg trispecific antibodies are also contemplated herein.

Heteroconjugate antibodies composed of two covalently joined antibodies are also contemplated herein. These antibodies have suggested utility in targeting immune system cells to unwanted cells (US Patent No. 4,676,980). The antibodies may be generated *in vitro* using crosslinking techniques known in the art.

The effectiveness of the antibody may be enhanced. For example, by introducing cysteine residue(s) into the Fe region, thereby allowing interchain disulfide bond formation in this region to generate a homodimeric antibody. Homodimeric antibodies may be generated using cross-linkers known in the art such as described in Wolff et al., Cancer Research, 53: 2560-2565 (1993).

Antiidiotypic antibodies can also be used in the therapies discussed herein, to induce an immune response to cells expressing a PSPU protein. Production of these antibodies is also well known (see for example Wagner et al., Hybridoma 16:33-40 (1997)).

Antibodies may be immobilized on a solid support: suitable supports include those discussed above for the nucleic acid sequences. Binding of antibodies to a solid support can be achieved using known art techniques. See for example Handbook of Experimental Immunology, 4th Edition, Blackwell Scientific Publications, Oxford (1986). The bound antibody is useful in the assays discussed herein.

A method of treating or preventing PIN or PRC in a subject by administering a compound that alters the expression of PSPU nucleic acid molecules is also described.

In one embodiment of this method, the therapeutic compounds may include polypeptide products of under-expressed markers, or a biologically active fragment thereof, a nucleic acid encoding an under-expressed gene downstream off expression control elements permitting expression of the gene in the PIN or PRC cells, compounds that increase the expression level of the marker endogenously existing in the PIN or PRC cells. These compounds can be obtained using the screening methods herein. To deliver a missing gene or protein to a cell a retrovirus system can be used. Such systems are known in the art, See for example US 5,082,670 and "Retroviral Vectors" in DNA cloning: A Practical Approach, Voltune 3, DRL Press, Washington (1987). As discussed above vectors can be incorporated into a cell by techniques such as microinjection, tuansfection, transduction and electroporation amongst others (Sambrook et al., supra). Gene therapy can be used to inhibit inappropriate over expression, or to enhance expression of a PSPU 43 molecule .

Compositions for treating or preventing PIN or PRC comprising pharmaceutically effective amounts of:

> a compound identified by a method of the invention are described. The compositions may include two or more of such compounds, antibodies and polypeptides or combinations thereof. Also included in the composition is a pharmaceutically acceptable carrier excipient or diluent. Also provided are pharmaceutical compositions comprising an effective amount of at least one PSPU antisense sequence, siRNA, ribozyme with a pharmaceutically acceptable carrier, excipient or diluent.

Therapeutic compositions containing a compound, antisense sequence, siRNA, ribozymes, polypeptide or antibody may be prepared by mixing an effective amount of the active molecule with optional pharmaceutically acceptable carriers, excipients or stabilizers (Remington's Pharmaceutical Sciences 16th edition, Osol, A. Ed. [1980]).

An effective amount as used herein refers to any of the actives including a polypeptide, antibody, small molecule, siRNA, antisense sequence, ribozyme, agonist or antagonist disclosed herein in an amount sufficient to carry out a stated purpose. A skilled worker can determine the amount empirically using routine methods. Similarly, a "pharmaceutically effective amount" or "therapeutically effective amount" refers to an amount of active disclosed herein which is effective to prevent or treat PIN or PRC (see definition of "treat") above.

The composition may be formulated for oral administration (eg capsules, tablets, lozenges, powders, syrups, and the like), for parenteral administration (eg intravenous solutions, subcutaneous, intramuscular or suppository formulations), for topical administration (eg creams, gels), for inhalation (eg intranasal, intrapulmonary) or such other forms of administration as are known in the art.

Acceptable carriers, excipients, or stabilizers are well known in the art. They must be nontoxic to recipients at the dosages and concentrations employed, and include buffers (eg phosphoric and citratic acid), water, oils, particularly olive, sesame, coconut and mineral and vegetable oils; carbohydrates including lactose, glucose, mannose, or dextrins; chelating agents such as EDTA; sugars such as sucrose, mannitol, trehalose or sorbitol; salt-forming counter-ions such as sodium; metal complexes (e.g., Zn-protein complexes); non-ionic surfactants such as TWEEN™, or polyethylene glycol (PEG)).

For tablets, diluents such as carbonates (eg sodium and calcium) phosphates (such as calcium phosphate) or lactose are commonly used with antioxidants, granulating and disintegrating agents (eg corn starch), binding agents such as starch, and lubricating agents such as stearic acid and magnesium stearate. Tablets may be coated to facilitate ingestion, stability or disintegration.

Injectable compositions are usually prepared with wetting agents (such as polyoxyethylene stearate, lecithin, and polyoxyethylene sorbitol monooleate) and suspending agents (such as methylcellulose, sodium alginate, and gum tragacanth) as well as diluents.

The compositions may also include additives such as colourants, antioxidants (such as ascorbic acid), sweeteners, thickening agents, (eg paraffin, beeswax), flavouring agents and preservatives (such as alkyl parabens, phenol, resorcinol and benzalkonium chloride) as appropriate.

Any conventional technologies may be employed to produce tablets, topical and intravenous formulations, syrups, oil-in-water emulsifiers, inhalants and the like (Remington's *supra*).

Liposomes can also be used to deliver the actives into cells. Where antibody fragments are used, the smallest inhibitory fragment which specifically binds to the binding domain of the target protein is preferred. Peptides can be chemically synthesized produced recombinantly as discussed above, or as otherwise known in the art. See for example PNAS USA 90,7889-7893 (1993).

The therapeutic compositions may also contain one or more additional active agents. Other actives selected should not have significant adverse effects on the main active agent discussed above. Examples of additional active agents are cytotoxic agents, cytokines, chemotherapeutic agents such as Taxol® and cisplatin, and or growth inhibitory agents. The actives are present in combination in therapeutically effective amounts. The actives may be formulated as part of the therapeutic composition, or separately for simultaneous or sequential use with the therapeutic composition.

The active agent may also be formulated as in microcapsules or aqueous suspensions for example, with suspending agents such as sodium alginates, methylcelluloses (eg methylcellulose, carboxymethyl cellulose, hydroxlpopylmethyl cellulose) or in macroemulsions. Such techniques are disclosed in Remington's Pharmaceutical Sciences 16th edition, Osol, A. Ed. (1980).

The compositions to be used for in vivo administration must be sterile. This is readily accomplished by filtration through sterile filtration membranes or other known art techniques.

Sustained-release preparations may be prepared. Suitable examples of sustained-release preparations microcapsules discussed above. Examples of sustained-release matrices include polyesters, and hydrogels.

In immunoadjuvant therapy, administration of the proteins, antibodies or compounds of the instant invention may be used in conjunction with chemotherapy, chemical androgen ablation therapy, or radiation therapy or the separate, simultaneous or sequential administration of other anticancer agents. Preparation and dosing schedules for agents may be used according to manufacturers' instructions or as determined by the skilled practitioner. Preparation and dosing schedules for chemotherapeutic agents is given in for example Chemotherapy Service Ed., M.C. Perry, Williams & Wilkins, Baltimore, MD (1992). For the treatment or reduction in the severity of PIN or PRC or its symptoms, the appropriate dosage of an active of the invention will depend on the patients age, type and severity of disease to be treated, whether the agent is administered for preventive or therapeutic purposes, previous or other concurrent therapies, the route of administration, the patient's clinical history and response to the active, according to the well known principles of medicine. The compound may be administered to the patient once only, continuously or repeatedly. For example daily, weekly, monthly, multiple times in a day, and administration may be regular, intermittent or at spaced intervals.

Depending on the type and severity of the disease, about 1 $\mu$g/kg to 15 mg/kg (e.g., 0.005 to 20 mg/kg, preferably 0.1 to 1mg/kg) of an active of the invention including a compound, composition, nucleic acid molecule, polypeptide or antibody of the invention is an initial candidate dosage for administration to the patient, in single or divided doses or by continuous infusion. A typical daily dosage might range from about 1 pg/kg to 100 mg/kg, more usually 1 mg to 75 mg/kg, or more, depending on the factors highlighted above. Treatment may be effected until the disease or its symptoms have abated or a decision is made to terminate. The treatment regime can be monitored by using assays herein discussed or other conventional monitoring techniques.

In this specification where reference has been made to patent specifications, other external documents, or other sources of information, this is generally for the purpose of providing a context for discussing the features of the invention.

The invention will now be illustrated in a non-limiting way by reference to the following Examples:

## Example 1

### Identification of Chromosome 8, Prostate-enriched sequences: *Pspu1, Pspu2, Pspu8 and Pspu43*

INTRODUCTION

We have developed a system of automated datamining which we refer to as Data-Panning. The starting point for this system is the capture of transcripts (mRNAs) from tissue samples and their conversion to stable products (cDNAs) in the form of cDNA libraries. The extensive sequencing of cDNA libraries has resulted in deposition of large numbers of Expressed Sequence Tags (ESTs) in GenBank. These expressed sequences are the source of the ESTs in the UniGene databases (Schuler *et al.* 1996). Currently, there are about 4.1 I million human ESTs/mRNAs in the human UniGene database.

UniGene partitions the ESTs imported from GenBank into a non-redundant set of gene-oriented clusters, with each UniGene cluster nominally containing sequences that represent transcripts from a single gene (Schuler *et al.* 1996). A key feature of UniGene is the assignment of a dbEST library ID to ESTs. Since the dbEST library ID identifies the tissue from which the dbEST library was constructed, this ID is a computationally unambiguous marker of the tissue source of the EST in a UniGene cluster.

dbEST libraries are derived from a wide range of organs and tissues. If these libraries were representative of the body as a whole, the aggregate of the individual library transcriptomes would reflect a whole-body transcriptome. At the same time, individual libraries would reflect regional differences in transcriptomes attributable to organs and tissues. UniGene clusters containing a high proportion of ESTs from a single tissue would be identifiable against the overall UniGene background.

We have used this computational method to identify enriched gene expression profiles in prostate tissues. No prior knowledge of the function or likely distribution of these genes is required. Four transcripts located on Chromosome 8 were identified using our approach and are described in this work. We named these transcripts Prostate specific unigene 1 (*Pspu1*), Prostate specific, unigene 2 (*Pspu2*), Prostate specific unigene 8 (*Pspu8*) and Prostate specific unigene 43 (*Pspu43*). The EST details are as follows:

Table 1: List of EST sequences making up each Chromosome 8 candidate

| Marker | GenBank Accession Numbers | Full EST sequence |
|---|---|---|
| *Pspu1* | AA635472 AI659328 AI659339 BX112742 AW014583 | |
| *Pspu2* | CB050448 BX113278 AI420913 AI927409 AW293795 | |
| *Pspu8* | BX283231 CF139278 BM043676 BU543602 | |
| *Pspu43* | AI611685 | ctttctttttttttgctctatctccagatcttccttcta gccaaactcctttgcacccaaaaagcagcctttgctttc ttgagatgaaagaacattcatgaaaatcatccctctact ggagtcctctagcaattcctgtgatttccacttacctga ctatgtacacaagcccagatacctggcttagtgtggga cagagcagagtgaccaagagtccagacctagagcctgct tgcctgggttcaaatctcatctctaccactcagtaaact ctgtcccactttcctcatctgaaaatgggcataacaat agtcccttatctacagg (SEQ ID NO:28) |

(continued)

| Marker | GenBank Accession Numbers | Full EST sequence |
|---|---|---|
| | AI418055 | tttttttttttttgctctatctccagatcttccttcta gccaaactcctttgcacccaaaaagcagcctttgctttc ttgagatgaaagaacattcatgaaaatcatccctctact ggagtcctctagcaattcctgtgatttccacttacctga ctatgtacacaagcccagatacctggcttagtgtgggga cagagcagagtgaccaagagtccagacctagagcctgct tgcctgggttcaaatctcatctctaccactcagtaaact ctgtcccactttcctcatctgaaaaatgggcataacaat (SEQ ID NO:29) |
| | BF446403 | ttttttttttttttgctctatctccagatcttccttct agccaaactcctttgcacccaaaaagcagcctttgcttt cttgagatgaaagaacattcatgaaaatcatccctctac tggagtcctctagcaattcctgtgatttccacttacctg actatgtacacaagcccagatacctggcttagtgtgggg acagagcaaagtgaccaagagtccaaacctagagcctgc ttgcctgggttcaaatctcatctctaccactcagtaaac tctgtcccactttcctcatctgaaaaatgggcataacaa tagtcccttatctcaca (SEQ ID NO:30) |
| | BF222603 | ctttcttttttttgctctatctccagatcttccttcta gccaaactcctttgcacccaaaaagcagcctttgctttc ttgagatgaaagaacattcatgaaaatcatccctctact ggagtcctctagcaattcctgtgatttccacttacctga ctatgtacacaagcccagatacctggcttagtgtgggga cagagcagagtgaccaagagtccagacctagagcctgct tgcctgggttcaaatctcatctctaccactcagtaaact ctgtcccactttcctcatctgaaaaatgggcataacaat agtcccttatctcacaggtttttagtaaaattaaatgag ttaatttaattttctaagcact (SEQ ID NO:31) |
| | BX109457 | tttattaacaaatataaagtaccagacactccaagtgct tagaaaattaaattaactcatttaattttactaaaaacc tgtgagataagggactattgttatgcccatttttcagat gaggaaagtgggacagagtttactgagtggtagagatga gatttgaacccaggcaagcaggctctaggtctggactct tggtcactctgctctgtccccacactaagccaggtatct gggcttgtgtacatagtcaggtaagtggaaatcacagga attgctagaggactccagtagagggatgattttcatgaa tgttctttcatctcaagaaagcaaaggctgctttttggg tgcaaaggagtttggctagaaggaagatctggagataga gcaaaaaaaagaaagaaaaaaaaaaaaaa (SEQ ID NO:32) |

(continued)

| Marker | GenBank Accession Numbers | Full EST sequence |
|---|---|---|
| | AA658380 | tttttttttgctctatctccagatcttccttctagcca aactcctttgcacccaaaaagcagcctttgctttcttga gatgaaagaacattcatggaaatcatccctctactggag tcctctagcaattcctgtgatttccacttacctgactat gtacacaagcccagatacctggcttagtgtggggacaga gcagagtgaccaagagtccagacctagagcctgcttgcc tgggttcaaatctcatctctaccactcagtaaactctgt cccactttcctcatctggtcgac (SEQ ID NO:33) |

SYSTEMS AND METHODS

The data recorded in each UniGene cluster provides a method for generating enriched gene expression profiles for any tissue or cell type for which a cDNA library has been sequenced and allocated a dbEST library ID. Each set of ESTs clustered by the UniGene algorithm is allocated a UniGene number. This number heads the cluster entry in the UniGene database along with any known information about the gene from which the cluster arose, any STS markers for the gene, possible protein similarities, chromosome locations, etc. A field within the UniGene record called 'scount' indicates how many sequences form the UniGene cluster. The final section of each UniGene record lists all the sequences that form the cluster. The format of this list includes the accession number for each sequence and a dbEST library ID number if the EST was sequenced as part of a cDNA library. The dbEST ID number acts as a marker for the biological source of a given sequence.

Generation of a gene expression profile from this information relies on the large number of randomly sequenced cDNA libraries and the dbEST library numbering system. If a gene is expressed solely by one tissue then only libraries constructed from that tissue have representative sequence from that gene. By determining the dbEST library IDs of each UniGene cluster, tissue specific gene expression is shown where all ESTs are derived from libraries constructed only from a single tissue.

Most genes are not completely specific to one tissue but show a distribution over a range of tissues. In a randomly sequenced cDNA library, genes expressed in high abundance will be sequenced more frequently than those expressed at low levels. This will be reflected in UniGene. Sequences will be tagged with dbEST library IDs from the tissues in which the gene is highly expressed more often than from tissues where it is expressed at low levels. This means that the number of times a sequence is tagged with a dbEST ID number from a tissue of interest within a UniGene cluster could indicate the level of gene expression in that tissue. This can be expressed as a percentage of the total number of ESTs that contributed to the UniGene cluster. We have used this approach to obtain biomarkers specific to prostate.

ALGORITHM

UniGene data files (Hs.data.gz) were downloaded from ftp://ftp.ncbi.nlm.nih.gov/repository/UniGene. These files were edited using three Perl scripts that utilize the Bioperl toolkit (Stajich *et al.* 2002). Specifically, these scripts call the Bio::Cluster::Unigene and Bio::ClusterIO modules. The first script, "lib_extract" automatically reviewed the number of EST sequence lines in each UniGene cluster and binned those UniGenes where the number of contributing sequences was above a specified threshold level.

This threshold level was set by defining the variable "$threshold" equal to 4 and comparing it with the scount of each UniGene cluster. Scount is the total number of sequences that contributed to the cluster. Any record was discarded if the number of sequences contributing to the cluster was the same or less than the threshold. Those with more than the threshold number of sequence lines were parsed into the in-house database for subsequent data panning (see below).

Scount was parsed from the raw Hs.data files and included those lines with no identifying dbEST library ID number. Since the subsequent computations were based on dbEST library IDs, some clusters were binned that appeared to have less than the stipulated threshold number of ESTs. Where this occurred, the lower number reflects the number of ESTs in the UniGene cluster with dbEST library IDs.

UniGene files downloaded from NIH are large (400Mb for human). Subsequent computation is greatly facilitated by creating a series of edited in-house databases that retain solely a UniGene cluster number and dbEST ID for each of the contributing ESTs. This procedure, using the "lib_extract" script, reduced the human data files to 40Mb.

Library catalogues (Hs.lib.info.gz) with some descriptors are available on the UniGene website. Further details on library construction are available from the UniGene Library Browser (http://ncbi.nlm.nih.gov/UniGene/). All UniGene libraries have dbEST library IDs.

Data panning was undertaken using the "lib_percentage" script. This takes a set of UniGene libraries specified by the investigator and then interrogates the edited in-house databases. These in-house databases no longer contain UniGene clusters with fewer than the threshold number of sequences. The script determines the number of EST sequence lines within each UniGene cluster that are derived from libraries of the specified set. These are expressed as a percentage of the total number of EST lines in the UniGene cluster.

IMPLEMENTATION

The Human dbEST library list was downloaded from the website http://ftp.ncbi.nih.gov/repository/dbEST. The list was opened in the program BBedit, prostate libraries identified and an edited list produced using the GREP function. 290 libraries were identified as being constructed from prostate tissues. The dbEST libraries used for this analysis to identify human prostate specific sequences were: 689, 787, 792, 876, 910, 924, 925, 926, 928, 934, 935, 940, 994, 1016, 1017, 1053, 1054, 1055, 1333, 1410, 1498, 1654, 1655, 1668, 1670, 1671, 1672, 1673, 4267, 4268, 4711, 4712, 4713, 4714, 4715, 4716, 4717, 4718, 4719, 4720, 6013, 6014, 6015, 6016, 6017, 6018, 6019, 6020, 6021, 6022, 6023, 6024, 6025, 6026, 6027, 6028, 6029, 6030, 6031, 6032, 6033, 6034, 6035, 6036, 6037, 6038, 6039, 6040, 6041, 6042, 6043, 6044, 6045, 6046, 6047, 6048, 6049, 6050, 6051, 6052, 6053, 6054, 6055, 6056, 6057, 6058, 6059, 6060, 6061, 6062, 6063, 6064, 6065, 6066, 6067, 6068, 6069, 6070, 6071, 6072, 6073, 6074, 6075, 6076, 6077, 6078, 6079, 6080, 6081, 6082, 6083, 6084, 6085, 6086, 6087, 6088, 6089, 6090, 6091, 6092, 6093, 6094, 6095, 6096, 6097, 6098, 6099, 6100, 6101, 6102, 6103, 6104, 6105, 6106, 6107, 6308, 6309, 6310, 6311, 6312, 6313, 6314, 6315, 6316, 6317, 6318, 6319, 6320, 6321, 6322, 6323, 6324, 6325, 6326, 6327, 6328, 6329, 6330, 6331, 6332, 6333, 6334, 6335, 6336, 6337, 6338, 6339, 6340, 6341, 6342, 6343, 6344, 6345, 6346, 6347, 6348, 6349, 6350, 6351, 6352, 6353, 6354, 6355, 6356, 6357, 6358, 6359, 6360, 6361, 6362, 6363, 6364, 6365, 6366, 6367, 6368, 6369, 6370, 6371, 6372, 6373, 6374, 6375, 6376, 6377, 6378, 6379, 6380, 6381, 6382, 6383, 6384, 6385, 6386, 6387, 6388, 6389, 6390, 6391, 6392, 6393, 6394, 6395, 6396, 6397, 6398, 6399, 6400, 6401, 6402, 6601, 6602, 6603, 6763, 6831, 7180, 7181, 8480, 8481, 8482, 8483, 8484, 8485, 8486, 8487, 8488, 8489, 8490, 8491, 8492, 8493, 8494, 8495, 8496, 8497, 8498, 8499, 8500, 8501, 8502, 8503, 8504, 8505, 8506, 8507, 8508, 8509, 8510, 8511, 8512, 8513, 8514, 8515, 8585, 8834, 9134, 9135, 9136, 9137, 9138, 10161, 10549, 11034, 11037, 14129, 14130, 14131. These libraries were all constructed from either normal or diseased prostate material. Computations were undertaken on human UniGene build available 4 March 2004. The results were imported into Microsoft Excel for sorting.

RESULTS AND DISCUSSION

Several studies have suggested that loss of gene sequences from the short arm of chromosome 8 (8p) is an early molecular event (Cher *et al.,* 1994; Macoska *et al.,* 1994; 1995; 2000; Haggman *et al.,* 1997) in nearly all prostate cancers and, significantly, in prostatic intraepithelial neoplasia (PIN) which is the most likely precursor of prostate cancer (Bostwick, 1996). Three transcripts identified using the data panning algorithm at the 100%, 75% and 80% enrichment level (Table 2 below) are located on 8p. Two, *in silico,* showed a pattern consistent with loss of expression between normal and diseased tissues. Another transcript with an 83% enrichment for prostate expression was located on 8q. We named these transcripts Prostate specific unigene 1 (*Pspu1*), Prostate specific unigene 2 (*Pspu2*), Prostate specific unigene 8 (*Pspu8*) and Prostate specific unigene 43 *(Pspu43).* These transcripts have not previously been described. *Pspu1* is located at 8p12, *Pspu2* is found at 8p21, *Pspu8* resides at 8p22-23 and *Pspu43* is positioned at 8q23.

Table 2: Enrichment results for Pspu1, Pspu2, Pspu8 and Pspu43

| UniGene Number | *Pspu* ID number | EST from prostate libraries | EST from other libraries | Total number of EST in UniGene Cluster | Percentage (%) |
|---|---|---|---|---|---|
| 197095 | *Pspu1* | 4 | 1 | 5 | 80 |
| 444680 | *Pspu2* | 3 | 1 | 4 | 75 |
| 458397 | *Pspu8* | 4 | 0 | 4 | 100 |
| 161160 | *Pspu43* | 5 | 1 | 6 | 83 |

An *in silico* gene expression profile for *Pspu1* and *Pspu2* was determined using the meta-analysis system described by Stanton and Green (2001). Briefly, sequenced prostate cDNA libraries were downloaded from the NCBI (http://ncbi.nlm.nih.gov). Each library was placed into a category determined by the tissue from which it was made. This meant that all libraries made from PIN tissues were grouped together while all cDNA libraries from normal tissues formed another group. ESTs in each library were clustered based on UniGene. This gave a list of transcribed units falling within each category. By tallying the number of ESTs for a given UniGene in each category an *in silico* gene expression profile is generated indicative of the level of specific transcript expressed by each tissue type. This data was used to generate Table 3 below. *Pspu8* and *Pspu43* were not included as the libraries that gave rise to them were excluded from the meta-analysis due to the nature of their construction.

Table 3: Example of expression profiles of genes in normal prostatic epithelium and progressive stages of prostate cancer. A normalized abundance score is given for normal prostate with levels in diseased tissues given as a percentage of normal expression. Levels of significance are given in parentheses as determined by Chi squared test of goodness of fit for 2 classes, ns = no significant difference.

| Description | Normal prostate | Prostatic intraepithelial neoplasia | Invasive carcinoma | Metastatic lesion |
|---|---|---|---|---|
| Prostate specific antigen | 1580 | 188% (*0.001*) | 103 % (ns) | 50 % (*0.001*) |
| Prostatic acid phosphatase | 790 | 169 % (*0.001*) | 83 % (ns) | *33 % (0.001)* |
| Prostate specific unigene 1 | 20 | 0 %(*0.001*) | *0 % (0.001)* | 0 % (*0.001*) |
| Prostate specific unigene 2 | 10 | 0 %(*0.001*) | 0 % (*0.001*) | 0 % (*0.001*) |

Comparison with several genes whose expression is known to alter during prostate cancer progression agrees with this meta-analysis. For example, digital expression profiles indicate an increased expression of prostate specific antigen (PSA) in prostatic intraepithelial neoplasia in agreement with what is widely accepted (Table 3). Furthermore, meta-analysis shows down regulation of prostatic acid phosphatase (Table 3). Prostatic acid phosphatase was used to diagnose prostate cancer prior to the PSA test (Bostwick, 1996), and is now thought to be associated with loss of androgen responsiveness of prostate tumours (Meng *et al.,* 2000).

Sequences for *Pspu1, Pspu2, Pspu8* and *Pspu43* were sampled multiple times from independent libraries thus giving confidence that they represent legitimate transcripts. Five ESTs contribute to UniGene cluster Hs.197095, the sequence contig we refer to as *Pspu1.* These arose from three cDNA libraries which were NCI_CGAP_Pr28 (dbEST Library ID.1410), NCI_CGAP_Pr22 (dbEST Library ID.910) and NCI_CGAP_Sub2 (dbEST Library ID.2359). Libraries 1410 and 910 were constructed from normal prostate tissue. Library 2359 arose from a subtracted cDNA library which was set up to identify breast specific genes (Bonaldo *et al.* 1996).

Four ESTs contributed to UniGene Hs.444680 or *Pspu2.* These ESTs were identified in cDNA libraries NCI_CGAP_Pr28 (dbEST Library ID.1410), NCI_CGAP_Pr22 (dbEST Library ID.910) and NCI_CGAP_Sub4 (dbEST Library ID.2721). Two of these libraries were made from normal prostate (1410 and 910). The third library was another subtraction library (2721) set up to find prostate specific genes (Bonaldo *et al.* 1996). Our data panning algorithm was not implemented to identify subtraction library 2721 as being constructed from prostate and thus the enrichment level given to this cluster was only 75%. In fact this UniGene may reflect a transcript solely restricted to the prostate.

*Pspu8* consisted of four EST sequences taken from the three clones that comprise UniGene Hs.458397. These clones were isolated from two libraries both of which were constructed from prostate carcinoma cell lines. These libraries were dbEST library 14129 and library 8834.

Six ESTs made up UniGene Hs.161160. The contig formed from these EST sequences is referred to as *Pspu43.* These ESTs arise from five clones found in two cDNA libraries. These libraries were NCI_CGAP_Pr28 (dbEST library ID.1410) and NCI_CGAP_Pr2 (dbEST library ID. 574). The data-panning algorithm indicated that this transcript was only 83% enriched in the prostate. Library 574, however, was not incorporated into the list of prostate specific libraries and so ESTs from this library were not tagged as being of prostate origin. Like *Pspu2* this transcript is likely to be solely of prostate origin.

The ESTs making up *Pspu1, Pspu2, Pspu8* and *Pspu43* were aligned to give the best consensus sequence for each candidate. The consensus sequences are given in Figure 1 and BLASTN (Altschul *et al.* 1990) results against the non-redundant GenBank database at the NCBI are given in Table 4 below.

Table 4: Summary of BLASTN analysis for *Pspu1, Pspu2, Pspu8* and *Pspu43* prostate specific candidates

| Contig Sequence | Contig Base Pairs that align to GenBank Candidate | Accession number for best GenBank alignment | Comments |
|---|---|---|---|
| *Pspu1* | 1-353 | AC044849.12 | Genomic DNA |
| *Pspu2* | 1-379 | AC090786.6 | Genomic DNA |
| *Pspu8* | 1-1320 | NM_0540281 | cDNA |
| *Pspu43* | 1-392 | AP001207/AP000426 | Genomic DNA |

*Pspu1* and *Pspu2* consensus sequences are 368bp and 394bp respectively in length, however, they both terminate in polyA stretches that could in reality be of variable length (18bop and 15bp respectively). Both sequences map on to the human genome but do not generate high scoring matches to known expressed genes. The *Pspu43* consensus sequence is 392bp long and also maps to the human genome but not expressed sequences. *Pspu8* is 1320bp long and maps to the expressed sequence for human Acyl-malonyl condensing enzyme.

CONCLUSION

We have identified four nucleic acid sequences, *Pspu1, Pspu2, Pspu8* and *Pspu43,* specific to the prostate using a UniGene data mining algorithm. *Pspu1, Pspu2* and *Pspu8* map to 8p12/21 border, 8p21 and 8p22-p23 respectively. Deletions from these loci are known early events in prostate cancer (MacGrogan *et al.* 1994). Loss of two of these sequences in disease is supported by a meta-analysis of gene expression between normal prostate and prostate cancer. *Pspu43* maps to the long arm of chromosome 8 at 8q23. An adjacent region, 8q24, has recently been genetically linked to prostate cancer susceptibility (Amundadottir *et al.* 2006). We suggest that *Pspu1, Pspu2, Pspu8* and *Pspu43* would be useful markers of chromosome 8 alterations that occur as early events in the development of prostate cancer.

Example 2

***Pspu43:*** **Characterization of a prostate disease marker on the long arm of Chromosome 8.**

INTRODUCTION

**Pspu43** ***was identified as being of significance to the prostate by datamining cDNA tissue libraries using the data panning algorithm described in Example 1 above. In total, four sequences were identified that mapped to chromosome 8 using the data-panning approach. Three were located on the short arm of chromosome 8 while*** **Pspu43** ***was located on the long arm of this chromosome.*** **Pspu43** ***lies close to a region on chromosome 8 that is often altered in prostate tumours and has been linked to a genetic susceptibility to prostate cancer (Amundadottir*** **et al.,** ***2006*****).**

This example summarizes our findings for *Pspu43,* including confirmation of genomic sequence, expression profile in a cell culture system and tissue specificity data.

SYSTEMS AND METHODS

*PCR Primer Design*

PCR primers for *Pspu43* were designed from a contig generated by EST cluster Hs.161160. The contig was BLASTN (Altschul *et al.,* 1990) analyzed to ensure no cloning vector sequence was incorporated in the contig. This edited sequence was loaded into a PCR primer design program (Primer3, Rozen and Skaletsky, 2000). Optimal primer pairs that generated the longest amplicon were selected and compared to the non-redundant gene sequence database at NCBI using BLASTN. Simulated PCR was performed using the AMPLIFY program (William Engles, Genetics Department, University of Wisconsin) with contig and primer sequences to ensure fidelity of match, avoid primer dimer formation and to test for possible primer secondary structures. Primers were synthesized by Invitrogen (USA). Primer sequences are given in Table 5.

TABLE 5: PCR primer sequences

| | | |
|---|---|---|
| *Pspu43* | Forward<br>Reverse | 5'-AACAAATATAAAGTACCAGACACTCCA -3'<br>5'-ATCTCCAGATCTTCCTTCTAGCC -3' |
| Transgelin 2 | Forward<br>Reverse | 5'- CTTCCAGAACTGGCTCAAGG-3'<br>5'- GAGAAGAGCCCATCATCTCG-3' |
| PSA | Forward<br>Reverse | 5'- CACTGCATCAGGAACAAAAGCGT-3'<br>5'-CATCACCTGGCCTGAGGAATC-3' |

*Extraction and amplification of RNA and genomic DNA.*

Normal prostate epithelial and stromal cells (PrEC and PrSC; Clonetics, San Diego CA) were grown and maintained in dedicated media (PrEGM BulletKit; Clonetics, San Diego CA) whilst the prostate cancer cell lines LNCaP (ATCC CRL 1740, Manassas, VA), DU145 (ATCC HTB-81, Manassas, VA) and RWPE-2 (ATCC CRL-11610, Manassas, VA) were grown and maintained in RPMI 1640 medium supplemented with 10% fetal bovine serum.
RWPE-2 is a derivative of a human papilloma virus immortalized prostate epithelial cell line (RWPE-1) transformed by v-Ki-*ras*. It is androgen responsive, invasive and tumorogenic (Bello *et al.* 1997; Webber *et al.* 1997a). LNCaP is a derivative of a metastasized prostatic carcinoma lesion, which is responsive to androgen (Webber *et al.,* 1997b). DU-145 is derived from a metastatic prostatic carcinoma lesion, which is unresponsive to androgen, highly invasive and tumorogenic (Webber *et al.,* 1997b).

PrEC and LNCaP cells were seeded at a density of either 2500 cells.$cm^{-2}$ or 4000 cells.$cm^{-2}$ respectively and cultured to 70% confluence in a humidified atmosphere of 5% $CO_2$ at 37˚C in 25 $cm^2$ vented flasks. Cells were harvested by trypsinisation, washed in trypsin free media and centrifuged at 500 g. Genomic DNA (gDNA) and RNA was extracted from cell pellets using TriZol (Invitrogen, Carlsbad, USA) according to the manufacturer's protocol. RNA was converted to cDNA using Superscript II (Invitrogen, Carlsbad, USA) as per manufacturer's instructions. PCR amplification was performed on 160ng genomic DNA or 37.5ng cDNA using the primers described above. PCR was carried out using Amplitaq Gold™ master mix (Applied Biosystems, NJ, USA). PCR conditions were optimized and established an effective annealling temperature of 65˚C. Samples from all prostate cell lines were compared.

RNA from a range of tissues was purchased from Clontech laboratories Inc. (Mountain View, CA, USA) or were donated from other research programs. These samples originated from Mammary Gland, Ovary, Testis, Kidney and Blood. These were converted to cDNA as above and used at a concentration of 37.5ng RNA equivalent for PCR assay.

*Sequencing of PCR products*

PCR products were gel purified using the QIAgen PCR purification system (QIAGEN GmbH, Hilden, Germany). DNA was removed from 1% agarose gel using the QX1 buffer, according to the manufacturer's instructions. DNA was eluted from the purification column in sterile milliQ water.

The purified PCR amplicon was sequenced with both forward and reverse PCR primers (10μM) using BigDye Terminator v3.1 chemistry.

RESULTS

Table 6 shows PCR results from both genomic DNA and cDNA synthesized from three prostate cell lines. These were LNCaP, PrEC and PrSC cell lines. PrEC and PrSC are derived from normal prostate epithelium and stromal cells, respectively. LNCaP is derived from a lymph node metastatic lesion from a patient with prostate cancer. *Pspu43* sequence was detected in genomic DNA isolated from all three cell lines. This indicates that *Pspu43* is part of the human genome and is not lost completely from the LNCaP cell line.

Table 6: Summarized PCR assay results from genomic and cDNA isolated from PrEC, PrSC and LNCaP cell lines.

| | PrEC | PrSC | LNCaP |
|---|---|---|---|
| Genomic DNA | + | + | + |
| cDNA | + | - | + |
| + = expected PCR product/ - = no PCR product | | | |

Gene expression results were obtained using RT-PCR from cDNA templates synthesized from each of the prostate cell lines. Results for all five cell lines are summarized in Table 7. RT-PCR was repeated a minimum of three times on at least two templates. *Pspu43* was expressed in four of the five cell lines. That is, it is present in PrEC, LNCaP, DU145 and RWPE2 samples but not in the PrSC sample. RT-PCR results for Transgelin 2 and PSA are included for comparison.

TABLE 7: RT-PCR results from Cell Lines

| Cell Line | PSPU 43 | Transgelin 2 | PSA |
|---|---|---|---|
| PrSC | - | + | + |
| PrEC | + | + | - |
| LNCaP | + | + | + |
| DU145 | + | + | + |
| RWPE2 | + | + | + |
| + = positive for PCR test, - = negative for PCR test | | | |

Tissue specificity for *Pspu43* was examined using RT-PCR on a number of different RNA samples isolated from Ovary, Kidney, Mammary Gland, Testis and Blood. *Pspu43* sequence was detected in both the Mammary Gland and Kidney but not the other tissues tested (Table 8).

Table 8: Summarized PCR assay results from RNA isolated from five tissues.

| Ovary | Testes | Mammary Gland | Kidney | Blood |
|---|---|---|---|---|
| - | - | + | + | - |
| + = expected PCR product/ - = no PCR product | | | | |

Since first identifying Hs.161160 as a UniGene cluster of interest to prostate biology it has been grouped with 142 ESTs, 7 mRNA sequences and named TFCP2L3 (Grainyhead-like 2 (Drosophila)). The original 6 EST that made up contig *Pspu43* still reside in this UniGene cluster. However, contig *Pspu43* does not map to any of the mRNA sequences currently associated with TFCP2L3. This was shown using 2-way BLAST between contig *Pspu43* and all 7 mRNA sequences. A BLAST alignment of the *Pspu43* contig to the non-redundant sequence database showed a complete match to two genomic clones only: AP000426 and AP001207. These clones are large non-annotated DNA sequences of 239,116 AND 153,936 nucleic acids respectively.

TFCP2L3 (Grainyhead-like 2 (Drosophila)) ESTs are represented highly in the prostate (expression profiler - NCBI) and by Northern Blotting (Peters *et al.,* 2002). TFCP2L3 does not appear to incorporate the *Pspu43* sequence, however. TFCP2L3 is a transcription factor that has been associated with a mutation leading to hearing loss (Peters *et al.* 2002).

CONCLUSION

*Pspu43* is an expressed RNA sequence identified as exclusively present in cDNA libraries made from both normal and cancerous prostate tissues. It is likely to be normally expressed in the prostate epithelium. *Pspu43* expression is therefore a possible marker of prostate health.

Example 3:

**Urine Analysis**

INTRODUCTION

We wished to test if *Pspu43* was detectable in the urine of men undergoing clincial examination for prostate disease. Urine samples were collected from the Department of Urology, Dunedin Hospital, Dunedin, New Zealand. RNA was extracted from these urine samples and subjected to RT-PCR to detect *Pspu43,* Transgelin 2 and PSA. These assays were scored. Patient diagnosis was made available only after RT-PCR results were obtained.

METHODS

All participants in this study gave written consent and the project received ethical approval from the Lower South Regional Ethics Committee ("Development of non-invasive, diagnostic and prognostic tests of prostate cancer" LRS/05/05/016). Men underwent prostate manipulation as part of the usual examination procedure to determine the physical state of their prostate. Prostate manipulation involved digital palpation of right and left lobes and the apex to base by sweeping the index finger three times, each side. Following this a 20 to 30 ml urine sample was collected. An equal volume of phosphate buffer (pH7.0) was added to the urine sample. This sample was stored overnight at 4˚C. Cells were harvested by centrifugion at 2500g for 15 minutes at 4˚C, the supernatant removed and the cell pellet resuspended in 800μl TriZol (Invitrogen, Carlsbad, USA). Glycogen (Invitrogen, Carlsbad, USA) was added to give a final concentration of 250μg/ml. RNA was extracted according to the manufacturer's protocol. The RNA pellet was resuspended in 16.5μl $H_2O$. Eight microlitres of the sample was treated with Dnase I (Roche, Switzerland) as per manufacture's instructions. Half of the Dnase I treated sample was converted to cDNA using Superscript II (Invitrogen, Carlsbad, USA) as per manufacturer's instructions. PCR amplification was performed on between 1 to 2.5μl cDNA and an equivalent volume of Dnase I treated RNA using the primers described below (Table 9). PCR was carried out using Amplitaq Gold™ master mix (Applied Biosystems, NJ, USA). PCR conditions were optimized and established an effective annealling temperature of 65˚C

TABLE 9: PCR primers for *Pspu43,* Transgelin 2 and PSA

| | | |
|---|---|---|
| *Pspu43* | Forward<br>Reverse | 5'-AACAAATATAAAGTACCAGACACTCCA -3'<br>5'-ATCTCCAGATCTTCCTTCTAGCC -3' |
| Transgelin 2 | Forward<br>Reverse | 5'- CTTCCAGAACTGGCTCAAGG-3'<br>5'- GAGAAGAGCCCATCATCTCG-3' |
| PSA | Forward<br>Reverse | 5'- CACTGCATCAGGAACAAAAGCGT-3'<br>5'-CATCACCTGGCCTGAGGAATC-3' |

RESULTS AND DISCUSSION

We obtained reliable RT-PCR results from urine samples provided by 8 men undergoing prostate examination for suspected disease, and one urine sample from a man who had no symptoms of prostate disease. PCR results were considered reliable if the RNA-only samples did not produce a PCR product. The enzymes used in our PCR system cannot use RNA as a template. Therefore PCR products arising from RNA-only reactions indicate the presence of genomic DNA in the sample. When this is the case it is not possible to distinguish gene expression from genomic contamination. These results are summarized in Table 10.

TABLE 10: Urine samples from 9 men

| Sample ID | PSPU 43 | Transgelin 2 | PSA | Gleason Grade |
|---|---|---|---|---|
| U "S" | - | + | + | NORMAL |
| U1 | - | + | + | No tumour |
| U3 | | | + | 7 |
| U6 | + | + | + | 6 |
| U7 | + | + | + | No tumour |
| U12 | - | + (+RT-) | + | 7 |
| U14 | + | + | + | Benign Prostatic Hyperplasia |
| U20 | - | + | + | Benign Prostatic Hyperplasia |

(continued)

| Sample ID | PSPU 43 | Transgelin 2 | PSA | Gleason Grade |
|---|---|---|---|---|
| U22 | + | + | + | 7 |
| + = positive for PCR test, - = negative for PCR test<br>No reliable results for cell with no entry. | | | | |

These experiments used a non-quantitative RT-PCR assay and no long term follow-up data on these patients was available. Sample U "S" is from a male with no apparent disease. No attempt was made to characterize cell populations in these urine samples. It proved challenging to extract consistent high quality RNA from urine samples and the quantity of RNA obtained was variable. As a result many samples were lost to experimental variables arising from establishing the technology.

It is clear from these results that transcripts of *Pspu43,* Transgelin 2 and PSA can be detected in urine. Pspu43 was detected in two of the known cancer patients (U6 & U22), one no tumour (U7) and one benign (U14). However, we have no long term follow up data on these patients. If *Pspu43* is an early indicator of disease progression it is possible that patients U7 and U14 have been misdiagnosed and are in the very early stages of disease. *Pspu43* was not detected in the normal (U "S"), no tumour (U1) or benign (U20) samples and it was not detected in one cancer patient (U12).

Transgelin 2 was detected in all samples, though the reading for U12 was suspect as a product was also produced from the no RT control. This was also the only known tumour sample that did not give a positive response for *Pspu43.* Three attempts were made to amplify two unrelated products from this sample and each time inconsistent results arose (data not shown). The PSA assay was attempted once only. The most favourable interpretation of results produced from this sample is that the RT-PCR reaction was unreliable, due either to poor quality or low concentration of RNA isolated from this sample.

PSA was detected in all samples regardless of disease state. This would indicate that PSA presence or absence from a urine sample is unlikely to be diagnostic given that it was detected regardless of prostate health or pathology of the individual.

CONCLUSION

*Pspu43* could be detected in the urine of men. It was detected more often in patients that were subsequently diagnosed from prostate biopsy to have prostate tumours than in men without tumours or with benign prostatic hyperplasia (BPH). PSA and Transgelin 2 were detected in all samples. Therefore, for a simple diagnostic test looking for the presence or absence of a marker neither PSA or Trangelin 2 would be suitable. *Pspu43,* on the other hand, may be able to be detected in patients with tumours. This supports the use of *Pspu43* as a marker for prostate cancer. A problem with raised PSA as a predictor of prostate cancer progression is that it cannot distinguish prostate cancer from other pathologies. BPH and prostatitis can both raise blood PSA scores. As *Pspu43* may differentiate between BPH and prostate cancer it potentially has greater sensitivity as a marker of cancer presence.

Example 4:

***Pspu43* expression in prostate tumour tissue**

INTRODUCTION

The purpose of this experiment was to examine changes in Pspu43 expression in the prostate with disease state.

Ten matched pairs of normal and lesion biopsies from single individuals were used in this study. These samples were collected from men undergoing prostatectomy for prostate adenocarcinoma and were all of Gleason Grade 6 and above. RNA was extracted from these samples and subjected to Quantitative PCR (qPCR) to determine both if *Pspu43* was expressed in the prostate and to compare the relative level of expression in tumour versus normal tissue. Matched biopsies ensured that underlying genetic variation between different individuals did not confound the results. We used Transgelin 1, shown to be downregulated in other cancers (Chang *et al., 2001,* Shields *et al., 2002*) as a comparison for *Pspu43* expression.

METHODS

*Tissues*

Tissue biopsies were obtained from the Cancer Society Tissue Bank (Christchurch, New Zealand). Written consent was obtained from all patients donating material to the tissue bank and specific approval for this project was obtained from the Cancer Society Tissue Bank Governing Board and The Lower South Regional Ethics Committee ("Development of non-invasive, diagnostic and prognostic tests of prostate cancer" LRS/05/05/016). Tissues were supplied as frozen tissue blocks that had been snap frozen in liquid nitrogen within 15 to 30 minutes after removal from the patient. Tissue and corresponding patient details are given in Tables 11 and 12. All tumours were of histological type prostatic adenocarcinoma and all displayed perineural invasion.

TABLE 11: Tissue and Patient Details

| ID | Age | N or T | Gleason grade | Max Size Tumour mm | % vol of tumour | Lymph/ vascular invasion | Necrosis | Mets in Nodes* |
|---|---|---|---|---|---|---|---|---|
| 5 F6 | 60 | Normal | | | | No | | |
| 5 F8 | 60 | Tumour | 6 | | 25 | No | | |
| 9 B3 | 70 | Normal | | | | | | |
| 9 B4 | 70 | Tumour | 6 | | 30 | | | |
| 9FS | 70 | Normal | | | | | | |
| 9F6 | 70 | Tumour | 6 | | 25 | Yes | | |
| 14 B9 | 71 | Normal | | | | | | 1/5 |
| 14 Cl | 71 | Tumour | 9 | | 85 | Yes | | 1/5 |
| 14 D1 | 49 | Normal | | | | | | |
| 14 D2 | 49 | Tumour | 7 | | 80 | +/- | | |
| 37 i9 | 67 | Normal | | | | | No | 0/12 |
| 38 A1 | 67 | Tumour | 7 | | 20 | No | No | 0/12 |
| 38 A3 | 63 | Normal | | 20 | | | Yes | 0/5 |
| 38 A4 | 63 | Tumour | 8 | 20 | 10 | Yes | Yes | 0/5 |
| 38 F1 | 66 | Normal | | | | | No | 0/14 |
| 38 F2 | 66 | Tumour | 7 | | 30 | No | No | 0/14 |
| 42 B1 | 63 | Normal | | 20 | | | No | 0/11 |
| 42 B2 | 63 | Tumour | 7 | 20 | | No | No | 0/11 |
| 43 C3 | 62 | Normal | | 25 | | | No | 0/2 |
| 43 C4 | 62 | Tumour | 7 | 25 | 20 | +/- | No | 0/2 |
| *Metastases in Lymph Nodes | | | | | | | | |

TABLE 12: Pathology Details

| ID | Pathology details |
|---|---|
| 5 F6/5 F8 | well-moderately differentiated prostatic adenocarcinoma arising in the left lobe adjacent to where the fresh material was taken for the tissue bank. Gleason grade 2+4 (score =6) |
| 9 B3/9 B4 | Level 1 capsular invasion, Margin negative. The tumour involves approximately 30% of the gland volume and involves both the left and the right lobes with a periurethral distribution on the right side. |

(continued)

| ID | Pathology details |
|---|---|
| 9 F5/9 F6 | Prostatic adenocarcinoma, Gleason score 6, Level 2 capsular invasion, seminal vesicle involvement. Tumour involves 25% gland volume. Lymphovascular invasion present |
| 14 B9/14 C1 | Present at excision margins |
| 14 D1/14 D2 | level 2 capsular spread. Most of both lobes are infiltrated by tumour |
| 37 i9/38 A1 | No description |
| 38 A3/38 A4 | non-confined level 3 focal |
| 38 F1/38 F2 | confined level 2 |
| 42 B1/42 B2 | level 2, confmed |
| 43 C3/43 C4 | Non-confined , level 3 established. Carcinoma is partly papillary. Areas suggestive of but not DIAGNOSTIC of vascular invasion. PSA=21 |

*RNA isolation*

Each of the prostate tissue blocks were mounted in Cryomoulds in OCT (Lab Tek products, Tennessee, USA). Tissue was then sectioned for RNA preparation. The first and last section taken consisted of an 8μm section which was mounted onto a slide. This slide was stored at -80˚C and used as an histology reference, if needed. Between the first and last section ten 60μm sections were cut and placed into a pottle.

Four millilitres of TriZol (Invitrogen, Carlsbad, USA) was added to the pottle and the sections immediately homogenised for 30 seconds. The homogenate was transferred to a 15 ml falcon tube and 1.5ml of chloroform added. The homogenate was vortexed for 15 seconds and place immediately on ice. These homogenates were then centrifuged at 4000rpm for 15 minutes at 4˚C. The top phase was removed to a clean tube and then reextracted with 1 ml of chloroform, repeating centrifugation at 4000rpm for 15 minutes at 4˚C. The top phase was again collected and transferred to a new tube. 0.53 volumes of 100% ethanol was added to the sample while vortexing vigorously. The entire nucleic acid/ethanol mix was then transferred to an Rneasy column (Qiagen, Germany) coupled to a vacuum manifold and the vacuum applied. The column, with bound nucleic acid, was then washed with 700μl RW1 wash buffer, followed again by application of vacuum. 500μl of wash buffer RPE was then drawn through the column under vacuum. The column was disassembled and then dried by centrifuging at 8000rpm for 15 to 30 seconds. The column was placed into a new 1.5ml centrifuge tube, 30μl of water added to the column and then centrifuged at 8000rpm for a further 15 to 30 seconds. The 30μl flow through volume was added back to the top of the column and the unit centrifuged again at 8000rpm for 15 to 30 seconds. This column eluant contained the RNA extracted from each set of ten 60μm sections obtained from each tissue block. The quality of the recovered RNA was tested by determining the optical density and 260/280 ratio using a Nanodrop spectrometer and also by electrophoresis using the Experion Bio-analyzer chip system (Bio-Rad, California, USA). RNA was stored at -80˚C until used.

*cDNA synthesis*

One microlitre of 5μg/μl Random Hexamers (Roche, Switzerland) was added to 600ng of RNA. This mix was heated to 95˚C for five minutes followed by five minutes at 25˚C. Samples were then transferred to ice. A cocktail of 4μl First strand buffer (Invitrogen, Carlsbad, USA), 4μl dNTP at 2.5mM each, 2μl 0.1M DTT, 0.5μl 40U/μl Rnase inhibiter (Invitrogen, Carlsbad, USA) and 1μl 200U/μl Superscript II (Invitrogen, Carlsbad, USA) was added to the RNA and mixed by pipetting. This was incubated at 42˚C for 120 minutes followed by a 10 minute incubation at 70˚C and a 1 minute incubation at 80˚C.

The cDNA was cleaned using a Qiagen PCR cleanup column (Qiagen, Germany). Eighty microlitres of water and 500μl of PB buffer were added to the 20μl cDNA synthesis reaction. This was centrifuged through a Qiagen cleanup column at 12000rpm for 1 minute. The flow through was discarded and the column, containing the bound cDNA was washed by centrifuging 750μl PE buffer at 12000rpm for 1 minute. The column was then dried by centrifugation at 12000rpm for 1 minute. The column was transferred to a new eluant collector and 40μl of water added. cDNA was eluted from the column by centrifuging at 8000rpm for 1 minute. A second 40μl aliquot was added to the column and the centrifugation step repeated. The clean, eluted cDNA sample was stored at -80˚C until used.

*qPCR*

cDNA was diluted to a concentration of 7.5ng/μl and 2 x 5μl aliquots of each sample were pipetted into the wells of a 96 opti-well plate. RNA samples were also diluted to 7.5ng/μl and one 5μl aliquot pipetted into a well on a 96 well plate. No template controls to check for PCR contamination and replicate standard curve cDNA was added to each 96 well plate. To each sample was added appropriate probe/primer mixes or SYBR green (Applied Biosystems, Foster City, CA, USA) and qPCR master mix (Applied Biosystems, Foster City, CA, USA) was added. The plates were sealed, mixed and then briefly centrifuged to ensure contents were collected at the base of each well. qPCR was performed on an ABI7000 machine (Applied Biosystems, Foster City, CA, USA).

Primer and probe sets are given in Table 13. Two systems were used to measure RNA transcript levels. Where single products were detected by dissociation analysis SYBR green was used as the non-specific inter-chelating dye to detect DNA amplification. In the presence of multiple bands in the dissociation analysis, a dual-labeled Taqman probe was used to provide amplicon discrimination. Each system was used in the 96 well format according to the manufacturer's protocol. Results were analysed using the SDS software package (version 1.2.3) from Applied Biosystems, California, USA.

TABLE 13: Primers and Probes used for qPCR

| Target | Forward | Probe | Reverse |
|---|---|---|---|
| *Pspu43* | 5'GGCTCTAGGTC TGGACTCTTGGT3' | (5'FAM)TGCTCTGTCCCCACA CTAAGCCAGG(3'DABCYL) | 5'CCTGACTATGTA CACAAGCCCAGAT3 |
| *Pspu8* | 5'GGCTGGGCCTG CTTCTGT3' | (5'FAM)CTCAACGTCCTCAGC ATTGGATGTGC(3'DABCYL) | 5'GCGGAGCATACG GTGGAA3' |
| *Pspu2* | 5'CCCTGTATGAA ATACTAAGAGGAG TCCTT3' | (5'FAM)CGGACATGAAAGGA CACTAGACAAATCCACA (3'DABCYL) | 5'CTATCGTTTATA TTTGCCTATGTAG TTACTTCAC3' |
| *Pspu1* | 5'TGGCTGTTACC TGCTCTTTCAC3' | (5'FAM)AGCTATCTTGCCACTG CAGACTCAGCAGT(3'DABCYL | 5'CAGGAGGGCTGA GGTACTGTGT3' |
| Transgelin1 (T1) | 5'AAGAATGATG GGCACTACCG 3' | SYBR Green | 5'ACTGATGATCTG CCGAGGTC3' |
| Transgelin2 (T2) | 5'CTTCCAGAACT GGCTCAAGG3' | SYBR Green | 5'GAGAAGAGCCC ATCATCTCG3' |

RESULTS AND DISCUSSION

RNA was extracted from each pair of matched tumour and normal samples from prostates taken from individuals undergoing prostatectomy. The average quantity of RNA obtained from each extract was 586ng/μl with 260/280 ratios of between 1.77 and 2.

qPCR reactions for each primer/probe combination were initially optimised using cDNA from the PC3 prostate cancer cell line (ATCC CRL 1435, Virginia, USA). Assays using SYBR green technology worked well for T1 and T2 but not for any of the *Pspu* transcript assays, as determined from dissociation peak analysis (Figure 2). Therefore, primer/probe sets were designed for the *Pspu* candidates to be used with the TaqMan qPCR system.

The absolute quantitation method was used to determine transcript quantity in each sample. Standard curves, generated from a universal standard of multiple stable cell lines, for each primer/probe set displayed $R^2$ values of between 0.9998 and 0.9932 (Figure 3). Raw total CT values for each sample pair are given in Figure 4. The standard deviation of the CT was calculated from duplicate qPCR reactions and is given as +/-1 standard deviation for each sample in Figure 4. The relative concentration of transcript cDNA was then calculated for the average CT and calculating the corresponding cDNA value from the standard curve. This value was then corrected for genomic DNA contamination by determining the relative cDNA concentration determined from RNA-only qPCR reactions and subtracting this from the values obtained from qPCR using transcribed cDNA. The corrected relative cDNA quantification for each marker from each tissue pair is given in Figure 5. A summary of the data is given in Table 14 along with the Gleason Grade of each patient's tumour.

TABLE 14: Prostate Tumour samples: Matched paired samples of normal and diseased tissue from ten individuals (qPCR)

| Patient | T1 | T2 | Pspu1 | Pspu2 | Pspu8 | Pspu43 | Gleason |
|---|---|---|---|---|---|---|---|
| 5 F6/5 F8 | < | < ns | < | < | < ns | < | 9 |
| 9 B3/9 B4 | < | < | > | < ns | ns | < ns | 7 |
| 9 F5/9 F6 | < | > ns | < ns | > ns | > ns | > | 7 |
| 14 B9/14 C1 | < | > | > | < | > | > | 8 |
| 14 D1/14 D2 | < | < ns | <ns | > | < | > | 7 |
| 37 i9/38 A1 | < | < | < | > | < | > | 7 |
| 38 A3/38 A4 | < | < | > ns | > | > ns | > | 7 |
| 38 F1/38 F2 | > ns | > ns | > ns | > ns | > | > ns | 6 |
| 42 B1/42 B2 | < | < ns | > | > | > ns | > | 6 |
| 43 C3/43 C4 | < | > ns | < ns | < ns | < | < ns | 6 |
| score | 1/9 | 4/6 | 5/5 | 6/4 | 5/5 | 7/3 | |

< = decreased in tumour relative to normal
> = increased in tumour relative to normal
ns = difference between normal and tumour not statistically significant
score = increased in tumour/decreased in tumour.

From these results it was demonstrated that all of the markers are expressed as RNA transcripts in both normal and tumour tissue. In general, fewer T1 transcripts are present in tumour tissue while *Pspu43* transcripts are increased (significant association ($P$ = 0.0055) of raised *Pspu43* in tumour vs normal tissue by 2x3 contingency table, Fisher's extact test). The loss of T1 is consistent with the findings for other cancers (Chang *et al., 2001,* Shields *et al., 2002*) and would correspond to a loss in cell cytoskeletal integrity and metastasis.

*Pspu43* was upregulated in all tumours with Gleason grades between 6 and 8 relative to the normal sample taken from each individual where the difference between each sample was greater than the standard error. In the most severe lesion (14B9/C1, Gleason 9) there was relatively more Pspu43 marker in the normal portion of the prostate. It is questionable that this 'normal' sample reflected a normally functioning prostate given the extent of the disease in this particular organ (85% involvement of the gland) and this may reflect the advanced nature of the disease. It is quiet possible that the transcriptome characteristics of a tumour of Gleason Grade 9 are significantly different from less severe forms of the disease.

No overtly consistent pattern was seen in expression of the markers *Pspu1, Pspu2, Pspu8* or T2. Therefore, though the *Pspu* markers 1, 2 and 8 were identified by our bioinformatics algorithm they did not prove able to differentiate tumour from normal prostate in this test.

CONCLUSION

This experiment showed that Transgelin 1 and 2, and *Pspu 1, 2, 8 and 43* are all expressed in prostate tissue regardless of disease state. No overtly cancer differentiating pattern of expression was demonstrated for markers T2, *Pspu 1, Pspu2* or *Pspu8.* T1 however tended to be down-regulated in tumour tissue, consistent with the findings of others for lung, breast and colon cancers (Chang *et al.,* 2001, Shields *et al.,* 2002). Conversely, *Pspu43* tended to be up-regulated in tumour tissue compared to the normal sample. This is significant. We know that this region of chromosome 8 is altered during the early disease process in many men. These results indicate that elevated *Pspu43* is indicative of prostate cancer.

REFERENCES

Altschul, S.F., Gish, W., Miller, W., Myers, E.W. & Lipman, D.J. (1990) Basic local alignment search tool. J. Mol. Biol. 215:403-410

Amundadottir LT, Sulem P, Gudmundsson J et al. (2006) A common variant associated with prostate cancer in European and African populations. Nat. Genet. (doi:10.1038/ng1808)

Beheshti B, Park PC, Sweet JM, Trachtenberg J, Jewett MA, Squire JA (2001) Evidence of chromosomal instability in prostate cancer determined by spectral karyotyping (SKY) and interphase fish analysis. Neoplasia 3: 62-69

Bello et al. (1997). Androgen responsive adult human prostatic epithelial cell lines immortalised by human papillomavirus 18. Carcinogenesis., 18, 1215-1223.

Bonaldo, Lennon & Soares (1996): Normalization and Subtraction: Two Approaches To Facilitate Gene Discovery. Genome Research 6, 791-806.

Bostwick DG (1996). Prospective origins of prostate carcinoma. Cancer 78: 330-336.

Chang JW, Jeon HB, Lee JH, Yoo JS, Chin JS, Kim JH, Yoo YJ (2001). Augmented expression of peroxidoxin I in lung cancer /Biochem Biophys Res Com/ 289: 507-512

Cher ML, MacGrogan D, Bookstein R, Brown JA, Jenkins RB, Jensen RH (1994). Comparative genomic hybridization, allelic imbalance, and fluorescence in situ hybridization on chromosome 8 in prostate cancer. Genes, Chromosomes & Cancer 11: 153-162.

Fehm et al (2002). Cytogenetic evidence that circulating epithelial cells in Patients with Carcinomas are malignant. Clinical Cancer Research 8 : 2073-2084.

Häggman MJ, Wojno KJ, Pearsall CP, Macoska JA (1997). Allelic loss of 8p sequences in prostatic intraepithelial neoplasia and carcinoma. Urology 50: 643-647.

Jefford CE, Irminger-Finger I (2006). Mechanisms of chromosome instability in cancers. Critical Reviews in Oncology/ Hematology 59: 1-14

MacGrogan D, Levy A, Bostwick D, Wagner M, Wells D, Bookstein R, Loss of chromosome arm 8p loci in prostate cancer: Mapping by quantitative allelic imbalance (1994) Genes, Chromosomes & Cancer 10: 151-159.

Macoska JA, Trybus TM, Sakr WA et al., (1994). Fluoresence in situ hybridisation analysis of 8p allelic loss and chromosome 8 instability in human prostate cancer. Cancer Research 54: 5390-5395.

Macoska JA, Trybus TM, Benson PD et al., (1995). Evidence for three tumor suppressor gene loci on chromosome 8p in human prostate cancer. Cancer Research 55: 5390-5395.

Macoska JA, Trybus TM, Wojno KJ (2000). 8p22 loss concurrent with 8c gain is associated with poor outcome in prostate cancer. Urology 55: 776-782.

Meng TC, Lee MS, Lin MF (2000) Interaction between protein tyrosine phosphatase and protein tyrosine kinase is involved in androgen-promoted growth of human prostate cancer cells. Oncogene 19:2664-77.

Peters LM, Anderson DW, Griffith AJ, Grundfast KM, San Agustin TB, Madeo AC, Friedman TB, Morell RJ (2002) Mutation of a transcription factor, TFCP2L3, causes progressive autosomal dominant hearing loss, DFNA28. Hum. Mol. Genet. 11: 2877-2885.

Rozen S, and Skaletsky HJ (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386

Schuler, G.D., Boguski, M.S., Stewart, E.A., Stein, L.D., Gyapay, G., Rice, K., White, R.E., Rodriguez-Tome, P., Aggarwal, A., Bajorek, E., Bentolila, S., Birren, B.B., Butler, A., Castle, A.B., Chiannilkulchai, N., Chu, A., Clee, C., Cowles, S., Day, P.J., Dibling, T., Drouot, N., Dunham, I., Duprat, S., East, C., Hudson, T.J., et al. (1996) A gene map of the human genome, Science 274, 540-546.

Shields JM, Rogers-Graham K, Der CJ (2002). Loss of transgelin in breast and colon tumours and in RIE-I cells by ras deregulation of gene expression through raf independent pathways. J Biol Chem 277, 9790-9799

Stajich, J.E., Block, D., Boulez, K., Brenner, S.E., Chervitz, S.A., Dagdigian, C., Fuellen, G., Gilbert, J.G., Korf, I., Lapp, H., Lehvaslaiho, H., Matsalla, C., Mungall, C.J., Osborne, B.I., Pocock, M.R., Schattner, P., Senger, M., Stein, L.D., Stupka, E., Wilkinson, M.D., Birney, E. (2002) The Bioperl toolkit: Perl modules for the life sciences, Genome Res. 12, 1611-1618.

Stanton, JL, Green DPL (2001). Meta-analysis of gene expression in mouse preimplantation embryo development. Molecular Human Reproduction, 7, 545-552.

Webber M et al. (1997a). Acinar differentiation by non-malignant immortalized human prostatic epithelial cells and its loss by malignant cells. Carcinogenesis, 18, 1225-1231. Webber M. et al. (1997). Immortalised and tumorogenic adult human prostatic epithelial cell lines: Characteristics and applications part 2. Tumorogenic cell lines. Prostate, 30, 58-64.

## Claims

**1.** An *in vitro* method of (a) diagnosing prostatic intraepithelial neoplasia (PIN) or prostate cancer (PRC) or a predisposition to PIN or PRC in a patient, (b) monitoring a course of treatment for PIN or PRC in a patient, or (c) for prognosing PIN or PRC in a patient, comprising
obtaining a PIN or PRC expression profile from a sample from the patient, the expression profile comprising a pattern of marker expression including a nucleic acid molecule, and
comparing the expression profile to an expression profile of a control sample,
wherein the nucleic acid molecule comprises:

(i) the sequence of SEQ NO:3,
(ii) a sequence which hybridises under stringent conditions to SEQ ID NO:3,
(iii) a sequence which has 70%, 75%, 80%, 90%, 95%, or 99% sequence identity to SEQ ID NO:3, or
(iv) an at least 20 nucleotide fragment of (i) or a complement thereof, which fragment or complement hybridizes under stringent conditions to:

(a) a nucleic acid molecule of (i), (ii) or (iii) or a complement thereof;
(b) a reverse complement of (a).

**2.** An *in vitro* method according to claim 1, wherein the nucleic acid molecule is at least 30, at least 40, at least 50 nucleotides, at least 60 nucleotides, at least 70 nucleotides, at least 80 nucleotides, at least 90 nucleotides, or at least 100 nucleotides in length.

**3.** An *in vitro* method according to claim 1 or claim 2, wherein the PIN or PRC expression profile further comprises one or more markers selected from SEQ ID NO:6 (PCA3), SEQ ID NO:7 (transgelin 1), SEQ ID NO:8 (transgelin 2) and SEQ ID NO:5 (PSA).

**4.** An *in vitro* assay for diagnosing prostatic intraepithelial neoplasia (PIN) or prostate cancer (PRC) in a patient, the assay comprising detecting the presence of a nucleic acid molecule as defined in claim 1 or claim 2 in a sample, said detection comprising:

(a) contacting the sample with a nucleotide probe which hybridises to a nucleic acid sequence as defined in claim 1 or claim 2 under stringent hybridisation conditions; and
(b) detecting the presence of a hybridisation complex in the sample.

**5.** The *in vitro* assay of claim 4, wherein the diagnosing comprises testing for, prognosing or monitoring response for treatment of PIN or PRC in a patient.

**6.** The *in vitro* assay of claim 4 or claim 5, wherein the nucleic acid molecule is SEQ ID NO: 3 (PSPU43).

**7.** The *in vitro* assay of claim 4, 5 or 6, wherein the probe is a labelled probe.

**8.** The *in vitro* assay of claim 7, wherein the probe is a fluorescently labelled probe.

**9.** The *in vitro* assay of any one of claims 4 to 8, wherein the probe is a complement of SEQ ID NO:3.

**10.** An *in vitro* method of diagnosing prostatic intraepithelial neoplasia (PIN), prostate cancer (PRC) or a predisposition to developing PIN or PRC in a patient, the method comprising determining the expression level of a nucleic acid molecule as defined in claim 1 or claim 2, in a patient sample, wherein an alteration in expression level compared to a control level of said nucleic acid molecule indicates that the patient has PIN, PRC, or is at risk of developing PIN or PRC.

**11.** The *in vitro* method of claim 10, wherein the alteration is an increase in expression level.

**12.** The *in vitro* method of claim 11, wherein the increase in expression level is at least 10% above the normal control level.

**13.** The *in vitro* method of any one of claims 10 to 12, wherein the control level is measured in a sample derived from normal prostate.

**14.** An *in vitro* method of monitoring response to treatment of prostatic intraepithelial neoplasia (PIN) or prostate cancer (PRC) in a patient, the method comprising determining the expression level of a nucleic acid molecule as defined in claim 1 or claim 2, in a patient sample, and comparing the level of said nucleic acid molecule to a control level, wherein a statistically significant change in the determined level from the control level is indicative of a response to the treatment.

**15.** The *in vitro* method of any one of claims 10 to 14, which further comprises determining the level of one or more additional markers of PIN or PRC and comparing the levels to marker levels from a control, wherein a significant deviation in the levels from a control level, together with a statistically significant increase in the level of a nucleic acid molecule as defined in claim 1 or claim 2, is indicative of PRC or PIN, or can be used to monitor PIN or PRC status.

**16.** The *in vitro* method of claim 15, wherein the additional markers are selected from the group consisting of SEQ ID NO:7 (transgelin 1), SEQ ID NO:5 (prostate specific antigen), and SEQ ID NO:6 (PCA3).

**17.** The *in vitro* method of any one of claims 10 to 16, wherein the sample is a urine, lymph, blood, plasma, semen, prostate massage fluid, or prostate tissue sample.

**18.** The *in vitro* method of any one of claims 10 to 17, wherein SEQ ID NO:8 (transgelin 2) is used as a reference marker.

**19.** The *in vitro* method of any one of claims 1-3 and 10-18, wherein the nucleic acid molecule is SEQ ID NO:3 (PSPU43).

**20.** The *in vitro* method of any one of claims 10-19, wherein the level of expression of the nucleic acid molecule is determined by a method comprising:

(i) direct or indirect measurement of the nucleic acid molecule; or
(ii) amplifying a DNA or cDNA sequence of the nucleic acid molecule or complement thereof; and measuring the level of one or more of DNA, cDNA or RNA in said sample.

**21.** The *in vitro* method of claim 20, wherein the nucleic acid molecule is employed in an *in situ* hybridisation or RT-PCR analysis.

**22.** The *in vitro* method of claim 20, wherein the DNA or cDNA is amplified using PCR.

**23.** The *in vitro* method of claim 20, 21 or 22, wherein the level of DNA, cDNA, or RNA in the sample is measured using electrophoresis.

**24.** Use of a nucleic acid molecule as defined in claim 1 or claim 2 in an *in vitro* method of diagnosing PIN or PRC in a patient.

**25.** The use of claim 24, wherein the nucleic acid molecule is SEQ ID NO:3 (PSPU43).

**26.** The use of claim 25 or claim 26, wherein the diagnosing comprises testing for, prognosing or monitoring response for treatment of PIN or PRC in a patient.

**Patentansprüche**

**1.** In-vitro-Verfahren zur (a) Diagnose von prostatischer intraepithelialer Neoplasie (PIN) oder Prostatakrebs (PRC) oder einer Prädisposition für PIN oder PRC bei einem Patienten, (b) Überwachen eines Behandlungsverlaufs für PIN oder PRC bei einem Patienten, oder (c) zur Prognose von PIN oder PRC bei einem Patienten, enthaltend
Erhalten eines PIN- oder PRC-Expressionsprofils aus einer Probe von dem Patienten, wobei das Expressionsprofil ein Muster einer Markerexpression enthält, ein Nukleinsäuremolekül beinhaltend, und
Vergleichen des Expressionsprofils mit einem Expressionsprofil einer Kontrollprobe,
wobei das Nukleinsäuremolekül enthält:

(i) die Sequenz von SEQ ID NR:3
(ii) die Sequenz, die unter stringenten Bedingungen an SEQ ID NR:3 hybridisiert,
(iii) eine Sequenz, die 70%, 75%, 80%, 90%, 95% oder 99% Sequenzidentität mit SEQ ID NR:3 aufweist, oder
(iv) ein zumindest 20 Nukleotid-Fragment von (i) oder ein Komplement davon, wobei das Fragment oder Komplement unter stringenten Bedingungen an:

(a) ein Nukleinsäuremolekül von (i), (ii) oder (iii) oder ein Komplement davon;
(b) ein reverses Komplement von (a)

hybridisiert.

**2.** In-vitro-Verfahren nach Anspruch 1, wobei das Nukleinsäuremolekül mindestens 30, mindestens 40, mindestens 50 Nukleotide, mindestens 60 Nukleotide, mindestens 70 Nukleotide, mindestens 80 Nukleotide, mindestens 90 Nukleotide oder mindestens 100 Nukleotide lang ist.

**3.** In-vitro-Verfahren nach Anspruch 1 oder Anspruch 2, wobei das PIN- oder PRC-Expressionsprofil des Weiteren einen oder mehrere Marker enthält, die ausgewählt sind aus SEQ ID NR:6 (PCA3), SEQ ID NR:7 (Transgelin 1), SEQ ID NR:8 (Transgelin 2) und SEQ ID NR:5 (PSA).

**4.** In-vitro-Test zur Diagnose von prostatischer intraepithelialer Neoplasie (PIN) oder Prostatakrebs (PRC) bei einem Patienten, wobei der Test den Nachweis des Vorhandenseins eines Nukleinsäuremoleküls, wie in Anspruch 1 oder Anspruch 2 definiert, in einer Probe enthält, wobei der Nachweis enthält:

(a) Kontaktieren der Probe mit einer Nukleotidsonde, die an eine Nukleinsäuresequenz, wie in Anspruch 1 oder Anspruch 2 definiert, unter stringenten Bedingungen hybridisiert; und
(b) Nachweisen des Vorhandenseins eines Hybridisierungskomplexes in der Probe.

**5.** In-vitro-Test nach Anspruch 4, wobei die Diagnose einen Test auf, eine Prognose oder Überwachung der Reaktion auf eine Behandlung von PIN oder PRC bei einem Patienten enthält.

**6.** In-vitro-Test nach Anspruch 4 oder Anspruch 5, wobei das Nukleinsäuremolekül SEQ ID NR:3 (PSPU43) ist.

**7.** In-vitro-Test nach Anspruch 4, 5 oder 6, wobei die Sonde eine markierte Sonde ist.

**8.** In-vitro-Test nach Anspruch 7, wobei die Sonde eine fluoreszierend markierte Sonde ist.

**9.** In-vitro-Test nach einem der Ansprüche 4 bis 8, wobei die Sonde ein Komplement von SEQ ID NR:3 ist.

**10.** In-vitro-Verfahren zur Diagnose von prostatischer intraepithelialer Neoplasie (PIN) oder Prostatakrebs (PRC) oder einer Prädisposition zur Entwicklung von PIN oder PRC bei einem Patienten, wobei das Verfahren das Bestimmen des Expressionswertes eines Nukleinsäuremoleküls, wie in Anspruch 1 oder Anspruch 2 definiert, in einer Patientenprobe enthält, wobei einer Veränderung im Expressionswert im Vergleich zum Kontrollwert des Nukleinsäuremoleküls anzeigt, dass der Patient PIN, PRC oder ein Risiko für die Entwicklung von PIN oder PRC hat.

**11.** In-vitro-Verfahren nach Anspruch 10, wobei die Veränderung eine Erhöhung im Expressionswert ist.

**12.** In-vitro-Verfahren nach Anspruch 11, wobei die Erhöhung im Expressionswert zumindest 10% über dem normalen Kontrollwert liegt.

**13.** In-vitro-Verfahren nach einem der Ansprüche 10 bis 12, wobei der Kontrollwert in einer Probe gemessen wird, die von einer normalen Prostata erhalten wird.

**14.** In-vitro-Verfahren zur Überwachung der Reaktion auf eine Behandlung einer intraepithelialer Neoplasie (PIN) oder eines Prostatakrebses (PRC) bei einem Patienten, wobei das Verfahren das Bestimmen des Expressionswertes eines Nukleinsäuremoleküls, wie in Anspruch 1 oder Anspruch 2 definiert, in einer Patientenprobe und das Vergleichen des Wertes des Nukleinsäuremoleküls mit einem Kontrollwert enthält, wobei eine statistisch signifikante Veränderung in dem bestimmten Wert gegenüber dem Kontrollwert eine Reaktion auf die Behandlung anzeigt.

**15.** In-vitro-Verfahren nach einem der Ansprüche 10 bis 14, das des Weiteren das Bestimmen des Wertes eines oder mehrerer zusätzlicher Marker von PIN oder PRC und das Vergleichen der Werte mit Markerwerten von einer Kontrolle enthält, wobei eine signifikante Abweichung in den Werten von einem Kontrollwert, gemeinsam mit einem statistisch signifikanten Anstieg in dem Wert eines Nukleinsäuremoleküls, wie in Anspruch 1 oder Anspruch 2 definiert, PRC oder PIN anzeigt oder zum Überwachen des PIN- oder PRC-Status verwendet werden kann.

**16.** In-vitro-Verfahren nach Anspruch 15, wobei die zusätzlichen Marker ausgewählt sind aus der Gruppe bestehend aus SEQ ID NR:5 (prostataspezifisches Antigen) und SEQ ID NR:6 (PCA3).

**17.** In-vitro-Verfahren nach einem der Ansprüche 10 bis 16, wobei die Probe Urin, Lymphe, Blut, Plasma, Samen, Prostatmassageflüssigkeit oder eine Prostatgewebeprobe ist.

**18.** In-vitro-Verfahren nach einem der Ansprüche 10 bis 17, wobei SEQ ID NR:8 (Transgelin 2) als Referenzmarker verwendet wird.

**19.** In-vitro-Verfahren nach einem der Ansprüche 1 bis 3 oder 10 bis 18, wobei das Nukleinsäuremolekül SEQ ID NR: 3 (PSPU43) ist.

**20.** In-vitro-Verfahren nach einem der Ansprüche 10 bis 19, wobei der Expressionswert des Nukleinsäuremoleküls durch ein Verfahren bestimmt wird, enthaltend:

(i) direkte oder indirekte Messung des Nukleinsäuremoleküls; oder
(ii) Amplifizieren einer DNA- oder cDNA-Sequenz des Nukleinsäuremoleküls oder Komplements davon; und Messen des Wertes von einem oder mehr von DNA, cDNA oder RNA in der Probe.

21. In-vitro-Verfahren nach Anspruch 20, wobei das Nukleinsäuremolekül in einer In-situ-Hybridisierung oder RT-PCR-Analyse verwendet wird.

22. In-vitro-Verfahren nach Anspruch 20, wobei die DNA oder cDNA unter Verwendung von PCR amplifiziert wird.

23. In-vitro-Verfahren nach Anspruch 20, 21 oder 22, wobei der Wert von DNA, cDNA oder RNA in der Probe mit Hilfe einer Elektrophorese gemessen wird.

24. Verwendung eines Nukleinsäuremoleküls, wie in Anspruch 1 oder Anspruch 2 definiert, in einem In-vitro-Verfahren zur Diagnose von PIN oder PRC bei einem Patienten.

25. Verwendung nach Anspruch 24, wobei das Nukleinsäuremolekül SEQ ID NR:3 (PSPU43) ist.

26. Verwendung nach Anspruch 25 oder Anspruch 26, wobei die Diagnose einen Test auf, eine Prognose oder Überwachung der Reaktion auf eine Behandlung von PIN oder PRC in einem Patienten enthält.

## Revendications

1. Procédé in vitro de (a) diagnostic d'une néoplasie intra-épithéliale prostatique (PIN) ou d'un cancer de la prostate (PRC) ou d'une prédisposition à une PIN ou à un PRC chez un patient, (b) suivie de l'évolution du traitement d'une PIN ou d'un PRC chez un patient, ou (c) pronostic d'une PIN ou d'un PRC chez un patient, comprenant l'obtention d'un profil d'expression de PIN ou de PRC à partir d'un échantillon du patient, le profil d'expression comprenant un schéma d'expression de marqueur comprenant une molécule d'acide nucléique, et
la comparaison du profil d'expression à un profil d'expression d'un échantillon témoin,
dans lequel la molécule d'acide nucléique comprend :

   (i) la séquence de SEQ ID N°: 3,
   (ii) une séquence qui s'hybride dans des conditions dures à la SEQ ID N°: 3,
   (iii) une séquence qui a une identité de séquence de 70 %, 75 %, 80 %, 90 %, 95 % ou 99 % avec la SEQ ID N°: 3, ou
   (iv) un fragment d'au moins 20 nucléotides de (i) ou un complément de celui-ci, fragment ou complément qui s'hybride dans des conditions dures à :

      (a) une molécule d'acide nucléique de (i), (ii) ou (iii) ou à un complément de celle-ci ;
      (b) un complément inverse de (a).

2. Procédé in vitro selon la revendication 1, dans lequel la molécule d'acide nucléique a une longueur d'au moins 30, d'au moins 40, d'au moins 50 nucléotides, d'au moins 60 nucléotides, d'au moins 70 nucléotides, d'au moins 80 nucléotides, d'au moins 90 nucléotides ou d'au moins 100 nucléotides.

3. Procédé in vitro selon la revendication 1 ou la revendication 2, dans lequel le profil d'expression d'une PIN ou d'un PRC comprend en outre un ou plusieurs marqueurs choisis parmi SEQ ID N° : 6 (PCA3), SEQ ID N° : 7 (transgéline 1), SEQ ID N° : 8 (transgéline 2) et SED ID N° : 5 (PSA).

4. Dosage in vitro pour le diagnostic d'une néoplasie intra-épithéliale prostatique (PIN) ou d'un cancer de la prostate (PRC) chez un patient, le dosage comprenant la détection de la présence d'une molécule d'acide nucléique telle que définie dans la revendication 1 ou la revendication 2 dans un échantillon, ladite détection comprenant :

   (a) la mise en contact de l'échantillon avec une sonde de nucléotide qui s'hybride à une séquence d'acides nucléiques telle que définie dans la revendication 1 ou la revendication 2 dans des conditions d'hybridation dures ; et
   (b) la détection de la présence d'un complexe d'hybridation dans l'échantillon.

5. Dosage in vitro selon la revendication 4, dans lequel le diagnostic comprend la détermination, le pronostic ou le suivie d'une réponse au traitement d'une PIN ou d'un PRC chez un patient.

6. Dosage in vitro selon la revendication 4 ou la revendication 5, dans lequel la molécule d'acide nucléique est SEQ ID N° : 3 (PSPU43).

7. Dosage in vitro selon la revendication 4, 5 ou 6, dans lequel la sonde est une sonde marquée.

8. Dosage in vitro selon la revendication 7, dans lequel la sonde est une sonde marquée par fluorescence.

9. Dosage in vitro selon l'une quelconque des revendications 4 à 8, dans lequel la sonde est un complément de SEQ ID N° : 3.

10. Procédé in vitro de diagnostic d'une néoplasie intra-épithéliale prostatique (PIN), d'un cancer de la prostate (PRC) ou d'une prédisposition au développement d'une PIN ou d'un PRC chez un patient, le procédé comprenant la détermination du niveau d'expression d'une molécule d'acide nucléique telle que définie dans la revendication 1 ou la revendication 2, dans un échantillon d'un patient, dans lequel l'altération du niveau d'expression par rapport à un niveau témoin de ladite molécule d'acide nucléique indique que le patient a une PIN ou un PRC ou présente un risque de développer une PIN ou un PRC.

11. Procédé in vitro selon la revendication 10, dans lequel l'altération est une augmentation du niveau d'expression.

12. Procédé in vitro selon la revendication 11, dans lequel l'augmentation du niveau d'expression est d'au moins 10 % supérieur au niveau témoin normal.

13. Procédé in vitro selon l'une quelconque des revendications 10 à 12, dans lequel le niveau témoin est mesuré dans un échantillon provenant d'une prostate normale.

14. Procédé in vitro de suivie d'une réponse à un traitement d'une néoplasie intra-épithéliale prostatique (PIN) ou d'un cancer de la prostate (PRC) chez un patient, le procédé comprenant la détermination du niveau d'expression d'une molécule d'acide nucléique telle que définie dans la revendication 1 ou la revendication 2, dans un échantillon d'un patient, et la comparaison du niveau de ladite molécule d'acide nucléique à un niveau témoin, dans lequel un changement statistiquement significatif du niveau déterminé par rapport au niveau témoin est une indication d'une réponse au traitement.

15. Procédé in vitro selon l'une quelconque des revendications 10 à 14, qui comprend en outre la détermination du niveau d'un ou de plusieurs marqueurs supplémentaires d'une PIN ou d'un PRC et la comparaison des niveaux aux niveaux de marqueur provenant d'un témoin, dans lequel un écart significatif des niveaux par rapport à un niveau témoin, avec une augmentation statistiquement significative du niveau d'une molécule d'acide nucléique telle que définie dans la revendication 1 ou la revendication 2, est une indication d'un PRC ou d'une PIN, ou peut être utilisé pour suivre l'état d'une PIN ou d'un PRC.

16. Procédé in vitro selon la revendication 15, dans lequel les marqueurs supplémentaires sont choisis dans le groupe constitué par SEQ ID N° : 7 (transgéline 1), SED ID N° : 5 (antigène spécifique de la prostate) et SEQ ID N° : 6 (PCA3).

17. Procédé in vitro selon l'une quelconque des revendications 10 à 16, dans lequel l'échantillon est l'urine, la lymphe, le sang, le plasma, le sperme, le fluide de massage prostatique ou un échantillon de tissu prostatique.

18. Procédé in vitro selon l'une quelconque des revendications 10 à 17, dans lequel SEQ ID N° : 8 (transgéline 2) est utilisée comme marqueur de référence.

19. Procédé in vitro selon l'une quelconque des revendications 1 à 3 et 10 à 18, dans lequel la molécule d'acide nucléique est SEQ ID N° : 3 (PSPU43).

20. Procédé in vitro selon l'une quelconque des revendications 10 à 19, dans lequel le niveau d'expression d'une molécule d'acide nucléique est déterminé par un procédé comprenant :

(i) la mesure directe ou indirecte de la molécule d'acide nucléique ; ou
(ii) l'amplification de la séquence d'ADN ou d'ADN complémentaire de la molécule d'acide nucléique ou d'un complément de celle-ci ; et la mesure du niveau d'un ou de plusieurs éléments parmi l'ADN, l'ADN complémentaire ou l'ARN dans ledit échantillon.

21. Procédé in vitro selon la revendication 20, dans lequel la molécule d'acide nucléique est employée dans une hybridation in situ ou dans une analyse RT-PCR.

22. Procédé in vitro selon la revendication 20, dans lequel l'ADN ou l'ADN complémentaire est amplifié par PCR.

23. Procédé in vitro selon la revendication 20, 21 ou 22, dans lequel l'ADN, l'ADN complémentaire ou l'ARN dans l'échantillon est mesuré par électrophorèse.

24. Utilisation d'une molécule d'acide nucléique telle que définie dans la revendication 1 ou la revendication 2 dans un procédé in vitro de diagnostic d'une PIN ou d'un PRC chez un patient.

25. Utilisation selon la revendication 24, dans laquelle la molécule d'acide nucléique est SEQ ID N° : 3 (PSPU43).

26. Utilisation selon la revendication 25 ou la revendication 26, dans laquelle le diagnostic comprend la détermination, le pronostic ou le suivie d'une réponse au traitement d'une PIN ou d'un PRC chez un patient.

## Pspu43

TTTATTAACAAATATAAAGTACCAGACACTCCAAGTGCTTAGAAAATTAAATTAACTCATTTAATTTTACTAAAAACCTGT
GAGATAAGGGACTATTGTTATGCCCATTTTTCAGATGAGGAAAGTGGGACAGAGTTTACTGAGTGGTAGAGATGAGATTTG
AACCCAGGCAAGCAGGCTCTAGGTCTGGACTCTTGGTCACTCTGCTCTGTCCCCACACTAAGCCAGGTATCTGGGCTTGTG
TACATAGTCAGGTAAGTGGAAATCACAGGAATTGCTAGAGGACTCCAGTAGAGGGATGATTTTCATGAATGTTCTTTCATC
TCAAGAAAGCAAAGGCTGCTTTTTGGGTGCAAAGGAGTTTGGCTAGAAGGAAGATCTGGAGATAGAGC

**FIGURE 1**

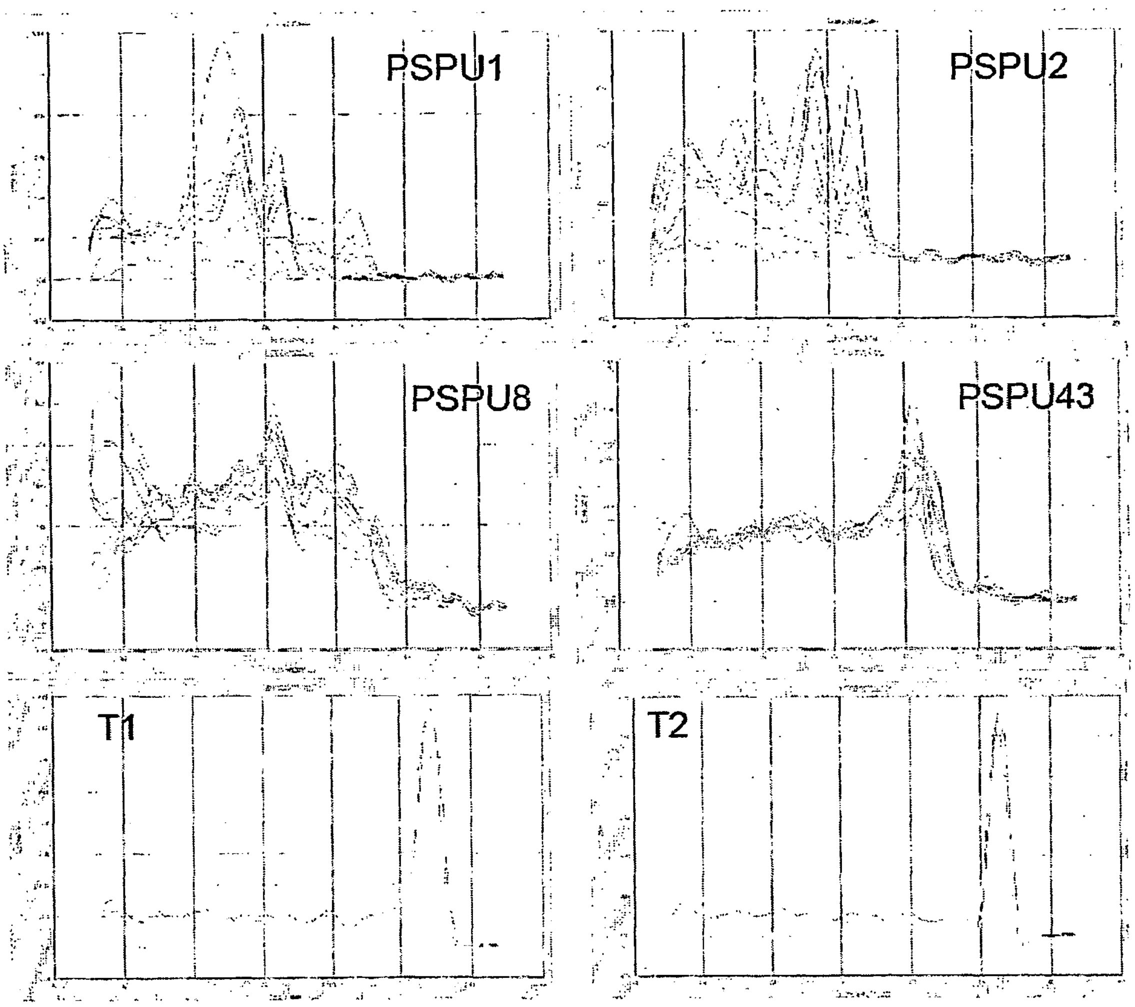


FIGURE 2

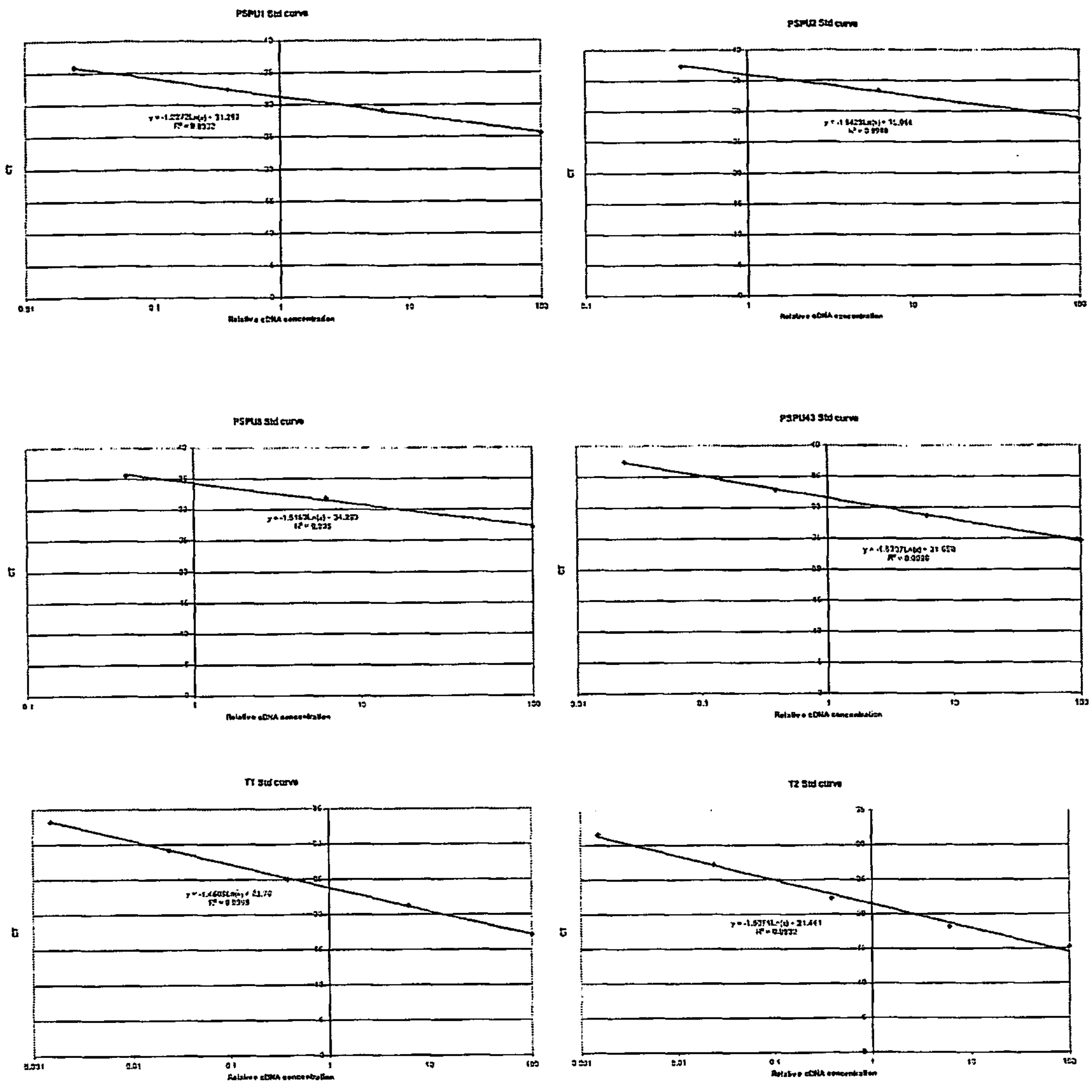
PSPU1 Std curve
PSPU2 Std curve
PSPU3 Std curve
PSPU43 Std curve
T1 Std curve
T2 Std curve
Ct
Relative cDNA concentration

**FIGURE 3**

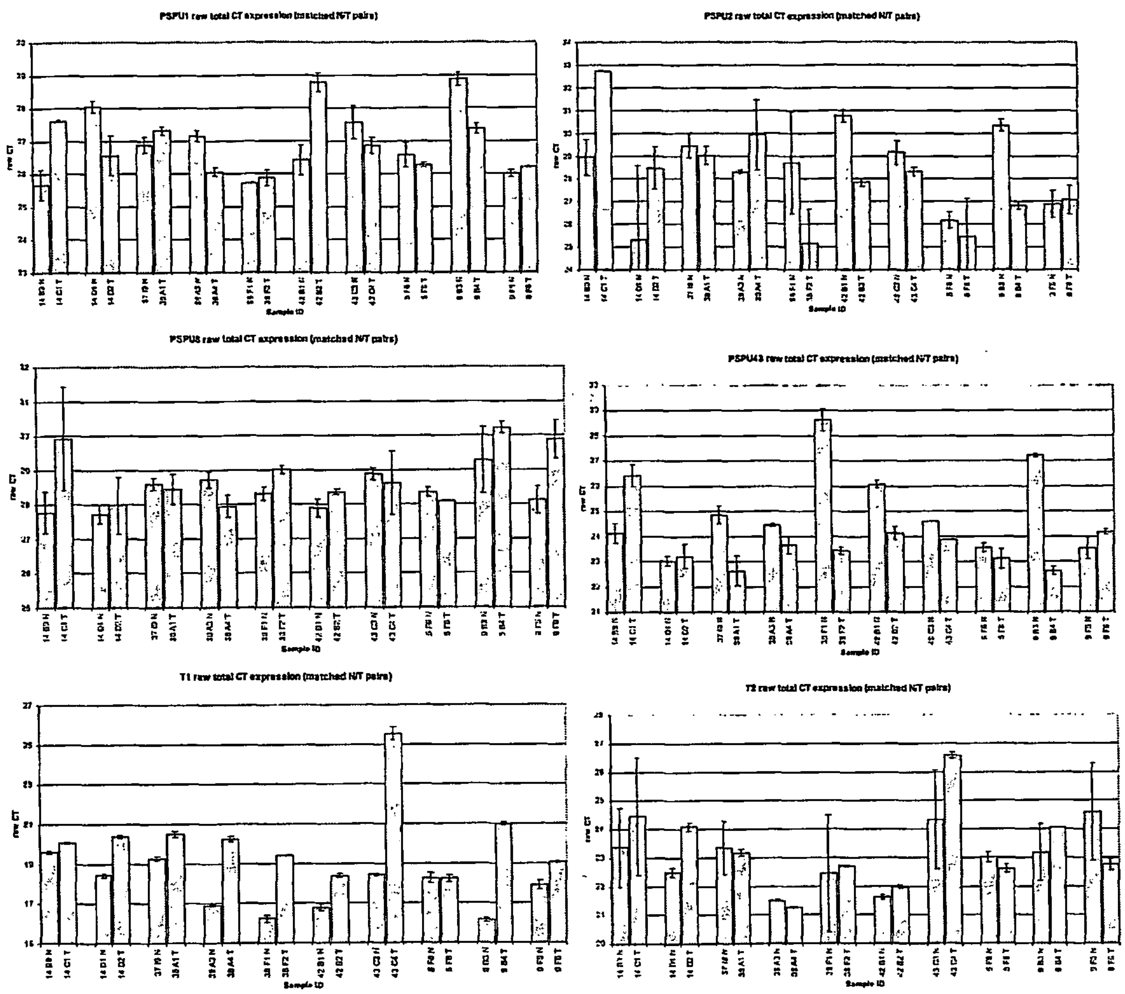

FIGURE 4

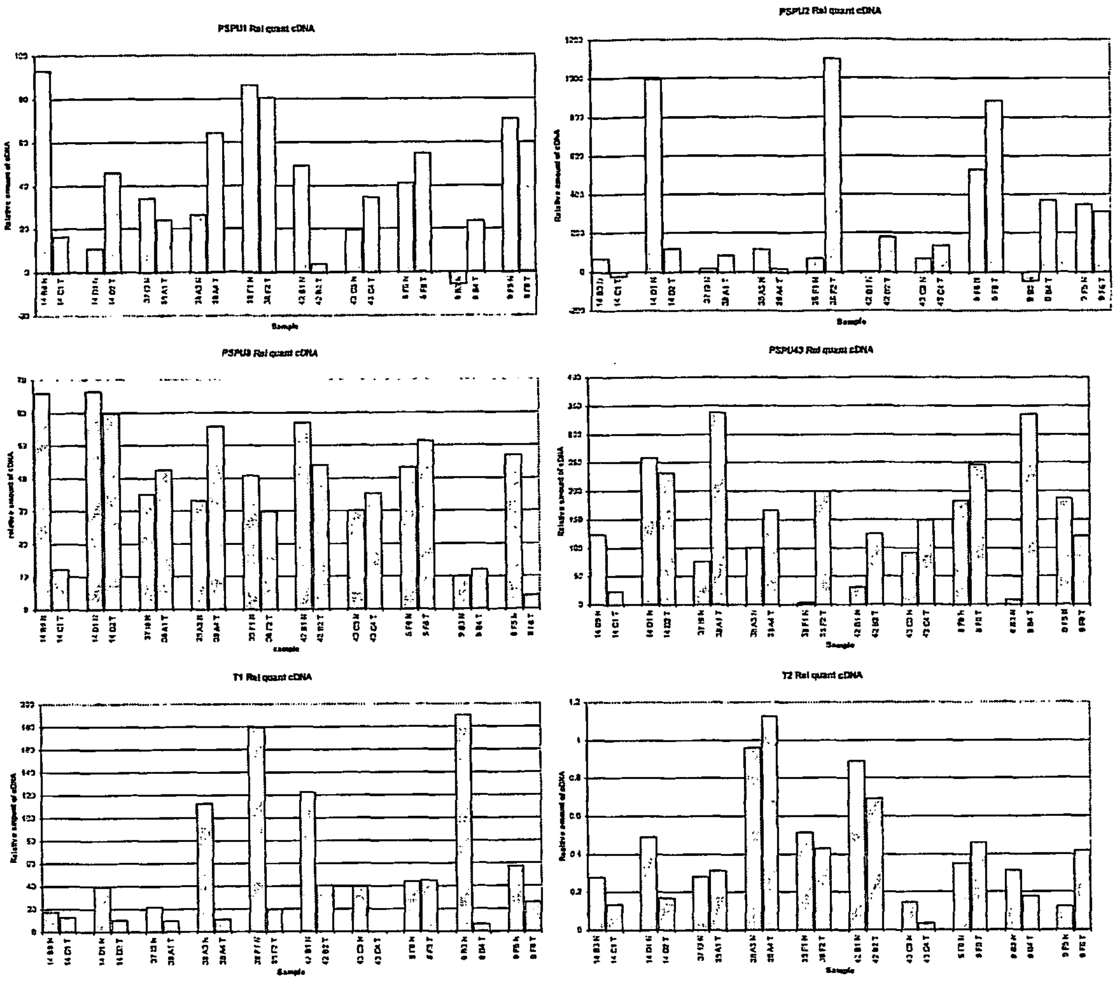

FIGURE 5

## Forward Reading Frame 1:

FINKYKVPDTPSA-KIKLTHLILHKTCEIRDYCYAHFSDEESGTEFTEW-R-
DLNPGKQALGLDSWSLCCVPTLSQVSGLVYIVR-VECTGIARGLQ-RDDFHECSFISRKQRLLFGCKGVWLEGRSGDR

## Forward Reading Frame 2:

LLTNIKYQTLQVLRKLN-LI-FY-KPVR-GTIVMPIFQMRKVGQSLLSGRDEI-TQASRL-VWTLGHSALSPH-
ARYLGLCT-SGKWKSQELLEDSSRGMIFMNVLSSQESKGCFLGAKEFG-KEDLEIE

## Forward Reading Frame 3:

Y-QI-STRHSKCLEN-INSFNFTKNL-DKGLLLCPFFR-GKWDRVY-
VVEMRFEPRQAGSRSGLLVTLLCPHTKPGIWACVHSQVSGNHRNC-RTPVEG-FS-
MFFHLKKAKAAFWVQRSLARRKIWR-S

## Reverse Reading Frame 1:

ALSPDLPSSQTPLHPKSSLCFLEMKEHS-
KSSLYWSPLAIPVISTYLTMYTSPDTWLSVGTEQSDQESRPRACLPGFKSHLYHSVNSVPLSSSEKWA-Q-
SLISQVFSKIK-VNLIF-ALGVSGTLYLLI

## Reverse Reading Frame 2:

LYLQIFLLAKLLCTQKAAFAFLR-KNIHENHPSTGVL-QFL-FPLT-
LCTQAQIPGLVWGQSRVTKSPDLEPACLGSNLISTTQ-TLSHFPHLKNGHNNSPLSHRFLVKLNELI-
FSKHLECLVLYIC--

## Reverse Reading Frame 3:

SISRSSF-PNSFAPKKQPLLS-DERTFMKIIPLLESSSNSCDFHLPDYVHKPRYLA-CGDRAE-PRVQT-
SLLAWVQISSLPLSKLCPTFLI-KMGITIVPYLTGF--N-MN-FNFLSTWSVWYFIFVNK

**FIGURE 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 01070979 A
- US 2004142325 A
- US 5792294 A
- US 20050064511 A
- US 5221685 A
- US 5310687 A
- US 5480792 A
- US 5525524 A
- US 5679526 A
- US 5824799 A
- US 5851776 A
- US 5885527 A
- US 5922615 A
- US 593927 A
- US 5647124 A
- US 5985579 A
- US 6019944 A
- US 6113855 A
- US 6143576 A
- US 5955371 A
- US 5631171 A
- US 5744305 A
- US 4816567 A
- US 4676980 A
- US 5082670 A
- US 60811407 B


### Non-patent literature cited in the description


- **CHAKRABARTI et al.** *Cancer Genetics and Cytogenetics,* 2002, vol. 139, 115-125
- **YU et al.** *J. Clin. Oncology,* 2004, vol. 22, 2790-2799
- **ERNST et al.** *Verh. Dtsch. Ges. Path.,* 2002, vol. 86, 165-175
- **OUDES et al.** *BMC Cancer,* 2005, vol. 5, 86-97
- **NEEDLEMAN, S. B. ; WUNSCH, C. D.** *J. Mol. Biol.,* 1970, vol. 48, 443-453
- **RICE,P. ; LONGDEN,I. ; BLEASBY,A.** EMBOSS: The European Molecular Biology Open Software Suite. *Trends in Genetics,* June 2000, vol. 16 (6), 276-277
- **HUANG, X.** *On Global Sequence Alignment. Computer Applications in the Biosciences,* 1994, vol. 10, 227-235
- *Nucleic Acids Res,* 2001, vol. 29, 1-1011-16
- **TATIANA A. et al.** *FEMS Microbiol Lett.,* 1999, vol. 174, 247-250
- **ALTSCHUL et al.** *Nuc.Acis Res,* 1997, vol. 25, 3389-3402
- Molecular Cloning, A Laboratory Manual. Cold Spring Harbor Press, 1987
- **AUSUBEL et al.** Current Protocols in Molecular Biology. Greene Publishing, 1987
- **BOLTON ; MCCARTHY.** *PNAS,* 1962, vol. 84, 1390
- **NIELSEN et al.** *Science,* 06 December 1991, vol. 254 (5037), 1497-500
- **GIESEN et al.** *Nucleic Acids Res.,* 01 November 1998, vol. 26 (21), 5004-6
- **BOWIE et al.** *Science,* 1990, vol. 247, 1306
- **HUANG, X.** On Global Sequence Alignment. *Computer Applications in the Biosciences,* 1994, vol. 10, 227-235
- The Polymerase Chain Reaction. Birkhauser, 1994
- **SAMBROOK.** *Methods Enzymol.,* 1993, vol. 218, 340-56
- **TRIGLIA et al.** *Nucleic Acids Res,* 1998, vol. 16, 8186
- **THOMPSON et al.** *Nucleic Acids Research,* 1994, vol. 22, 4673-4680, http://www-igbmc.u-strasbg.fr/BioInfo/ClustalW/Top.html
- **CEDRIC NOTREDAME et al.** *J. Mol. Biol.,* 2000, vol. 302, 205-217
- **FENG et al.** *J. Mol. Evol.,* 1987, vol. 25, 351
- **BAIROCH et al.** *Nucleic Acids Res.,* 1994, vol. 22, 3583
- **HOFMANN et al.** *Nucleic Acids Res.,* 1999, vol. 27, 215
- **FALQUET et al.** *Nucleic Acids Res.,* 2002, vol. 30, 235
- **TATUSOV et al.** *Science,* 1997, vol. 278, 631-637
- **SAMBROOK et al.** Molecular Cloning: A Laboratory Manual. Cold Spring Harbor Press, 1987
- **MERRIFIELD.** *J. Am Chem. Soc.,* 1963, vol. 85, 2149
- **STEWART et al.** Solid-Phase Peptide Synthesis. WH Freeman Co, 1969
- **MATTEUCCI et al.** *J. Am. Chem. Soc.,* 1981, vol. 103, 3185-3191

- **ADELMEN et al.** *DNA,* 1983, vol. 2, 183
- Methods in Enzymology. **DEUTSCHER.** Guide to Protein Purification. 1990, vol. 182
- **COHEN, SN.** *PNAS,* 1972, vol. 69, 2110
- Methods in Enzymology. Guide to Protein Purification. 1990, vol. 182
- **BRINSTER et al.** *PNAS (USA,* 1985, vol. 82, 4438-4442
- **JAENUCH, R.** *PNAS (USA,* 1976, vol. 73, 1260-1264
- **JOHNER, D et al.** *Nature,* 1982, vol. 298, 623-628
- **LOVEL-BADGE, R. H. et al.** Tetracarcinomas and Embryonic Stem Cells: A Practical Approach. DRL Press, 1987, 153-182
- **HOUDEBINE.** Transgenic Animals - Generation and Use. Harwood Academic, 1997
- Immunoassay Handbook. Elsevier Ltd, 2005
- **SOLBER H.** Collected reference values. Determination of reference limits. *Journal of Clinical Chemishy and Clinical Biochemistry,* 1987, vol. 25, 645-656
- **THOMAS.** *Pro. NAH, Acad. Sci. USA,* 1980, vol. 77, 5201-5205
- **JAIN KK.** *Med Device Technol.,* May 2004, vol. 15 (4), 14-7
- **LUTZ et al.** *Exp. Cell. Res.,* 1988, vol. 175, 109-124
- **FELUN et al.** Cytogenetic evidence that circulating epithelial cells in patients with carcinoma are malignant. *Clinical Cancer Research,* 2002, vol. 8, 2073-2084
- **ZOLA.** Monoclonal Antibodies: A Manual of Techniques. CRC Press, Inc, 1987, 147-158
- **HARLOW ; LOME.** Antibodies, A Laboratory Manual. Cold Spring Harbour Publications, 1998
- **LAM.** *Anticancer Drug Des.,* 1997, vol. 12, 1-145
- **DE WITT et al.** *PNAS,* 1993, vol. 90, 6909
- **YUAN et al.** *Nucleic Acids Research,* 2004, vol. 32, W130-W134, http://www.ambion.com/tehlib/misc/siRNA finder.html
- **KOIZUMI et al.** *FEBS Lett.,* 1998, vol. 228, 225
- **KIKUCHI et al.** *NAR,* 1992, vol. 19, 6751
- **HUSTON et al.** *Proc. Natl. Acad. Sci. USA,* 1988, vol. 85, 5879-83
- Antibodies: A Laboratory Manual. CSH press, 1988
- **TANAKA et al.** *Nucleic Acids Research,* 2003, vol. 31 (5), 23
- **KOHLER ; MILSTEIN.** *Nature,* 1975, vol. 256, 495
- **VERHOEYEN et al.** *Science,* 1988, vol. 239, 1534-1536
- **JONES et al.** *Nature,* 1986, vol. 321, 522-525
- **RIECHMANN et al.** *Nature,* 1988, vol. 332, 323-329
- **HOOGENBOOM ; WINTER.** *J. Mol. Biol.,* 1991, vol. 227, 381
- *Nature Biotechnology,* 1996, vol. 14, 826
- **VAUGHAN et al.** *Nature Biotechnology,* 1998, vol. 16, 535-539
- **MILSTEIN ; CUELLO.** *Nature,* 1983, vol. 305, 537-539
- **SURESH et al.** *Methods in Enzymology,* 1986, vol. 121, 210
- **BRENNAN et al.** *Science,* 1985, vol. 229, 81
- **WOLFF et al.** *Cancer Research,* 1993, vol. 53, 2560-2565
- **WAGNER et al.** *Hybridoma,* 1997, vol. 16, 33-40
- Handbook of Experimental Immunology. Blackwell Scientific Publications, 1986
- Retroviral Vectors. DNA cloning: A Practical Approach. DRL Press, 1987, vol. 3
- Remington's Pharmaceutical Sciences. 1980
- *PNAS USA,* 1993, vol. 90, 7889-7893
- Chemotherapy Service. Williams & Wilkins, 1992
- **ALTSCHUL, S.F. ; GISH, W. ; MILLER, W. ; MYERS, E.W ; LIPMAN, D.J.** Basic local alignment search tool. *J. Mol. Biol.,* 1990, vol. 215, 403-410
- **AMUNDADOTTIR LT ; SULEM P ; GUDMUNDSSON J et al.** A common variant associated with prostate cancer in European and African populations. *Nat. Genet.,* 2006
- **BEHESHTI B ; PARK PC ; SWEET JM ; TRACHTENBERG J ; JEWETT MA ; SQUIRE JA.** Evidence of chromosomal instability in prostate cancer determined by spectral karyotyping (SKY) and interphase fish analysis. *Neoplasia,* 2001, vol. 3, 62-69
- **BELLO et al.** Androgen responsive adult human prostatic epithelial cell lines immortalised by human papillomavirus 18. *Carcinogenesis,* 1997, vol. 18, 1215-1223
- **BONALDO ; LENNON ; SOARES.** Normalization and Subtraction: Two Approaches To Facilitate Gene Discovery. *Genome Research,* 1996, vol. 6, 791-806
- **BOSTWICK DG.** Prospective origins of prostate carcinoma. *Cancer,* 1996, vol. 78, 330-336
- **CHANG JW ; JEON HB ; LEE JH ; YOO JS ; CHIN JS ; KIM JH ; YOO YJ.** Augmented expression of peroxidoxin I in lung cancer. *Biochem Biophys Res Com,* 2001, vol. 289, 507-512
- **CHER ML ; MACGROGAN D ; BOOKSTEIN R ; BROWN JA ; JENKINS RB ; JENSEN RH.** Comparative genomic hybridization, allelic imbalance, and fluorescence in situ hybridization on chromosome 8 in prostate cancer. *Genes, Chromosomes & Cancer,* 1994, vol. 11, 153-162

- **FEHM et al.** Cytogenetic evidence that circulating epithelial cells in Patients with Carcinomas are malignant. *Clinical Cancer Research,* 2002, vol. 8, 2073-2084
- **HÄGGMAN MJ ; WOJNO KJ ; PEARSALL CP ; MACOSKA JA.** Allelic loss of 8p sequences in prostatic intraepithelial neoplasia and carcinoma. *Urology,* 1997, vol. 50, 643-647
- **JEFFORD CE ; IRMINGER-FINGER I.** Mechanisms of chromosome instability in cancers. *Critical Reviews in Oncology/Hematology,* 2006, vol. 59, 1-14
- **MACGROGAN D ; LEVY A ; BOSTWICK D ; WAGNER M ; WELLS D ; BOOKSTEIN R.** Loss of chromosome arm 8p loci in prostate cancer: Mapping by quantitative allelic imbalance. *Genes, Chromosomes & Cancer,* 1994, vol. 10, 151-159
- **MACOSKA JA ; TRYBUS TM ; SAKR WA et al.** Fluoresence in situ hybridisation analysis of 8p allelic loss and chromosome 8 instability in human prostate cancer. *Cancer Research,* 1994, vol. 54, 5390-5395
- **MACOSKA JA ; TRYBUS TM ; BENSON PD et al.** Evidence for three tumor suppressor gene loci on chromosome 8p in human prostate cancer. *Cancer Research,* 1995, vol. 55, 5390-5395
- **MACOSKA JA ; TRYBUS TM ; WOJNO KJ.** 8p22 loss concurrent with 8c gain is associated with poor outcome in prostate cancer. *Urology,* 2000, vol. 55, 776-782
- **MENG TC ; LEE MS ; LIN MF.** Interaction between protein tyrosine phosphatase and protein tyrosine kinase is involved in androgen-promoted growth of human prostate cancer cells. *Oncogene,* 2000, vol. 19, 2664-77
- **PETERS LM ; ANDERSON DW ; GRIFFITH AJ ; GRUNDFAST KM ; SAN AGUSTIN TB ; MADEO AC ; FRIEDMAN TB ; MORELL RJ.** Mutation of a transcription factor, TFCP2L3, causes progressive autosomal dominant hearing loss, DFNA28. *Hum. Mol. Genet.,* 2002, vol. 11, 2877-2885
- Primer3 on the WWW for general users and for biologist programmers. **ROZEN S ; SKALETSKY HJ.** Bioinformatics Methods and Protocols: Methods in Molecular Biology. Humana Press, 2000, 365-386
- **SCHULER, G.D. ; BOGUSKI, M.S. ; STEWART, E.A. ; STEIN, L.D. ; GYAPAY, G. ; RICE, K. ; WHITE, R.E. ; RODRIGUEZ-TOME, P. ; AGGARWAL, A. ; BAJOREK, E.** A gene map of the human genome. *Science,* 1996, vol. 274, 540-546
- **SHIELDS JM ; ROGERS-GRAHAM K ; DER CJ.** Loss of transgelin in breast and colon tumours and in RIE-I cells by ras deregulation of gene expression through raf independent pathways. *J Biol Chem,* 2002, vol. 277, 9790-9799
- **STAJICH, J.E. ; BLOCK, D. ; BOULEZ, K. ; BRENNER, S.E. ; CHERVITZ, S.A. ; DAGDIGIAN, C. ; FUELLEN, G. ; GILBERT, J.G. ; KORF, I. ; LAPP, H.** The Bioperl toolkit: Perl modules for the life sciences. *Genome Res.,* 2002, vol. 12, 1611-1618
- **STANTON, JL ; GREEN DPL.** Meta-analysis of gene expression in mouse preimplantation embryo development. *Molecular Human Reproduction,* 2001, vol. 7, 545-552
- **WEBBER M et al.** Acinar differentiation by non-malignant immortalized human prostatic epithelial cells and its loss by malignant cells. *Carcinogenesis,* 1997, vol. 18, 1225-1231
- **WEBBER M. et al.** Immortalised and tumorogenic adult human prostatic epithelial cell lines: Characteristics and applications part 2. Tumorogenic cell lines. *Prostate,* 1997, vol. 30, 58-64